# EUROPEAN PATENT APPLICATION

(11) **EP 3 940 384 A1**
(43) Date of publication of application: **19.01.2022**
(21) Application number: 20769239.3
(22) Date of filing: 11.03.2020
(51) Int. Cl.: G01N 33/68, G01N 33/58, G01N 21/64, G01N 21/552, G01N 21/76, G01N 25/48, A61K 31/404, A61K 31/517, A61K 31/352, A61P 25/16, A61P 25/28

(54) **METHOD FOR SCREENING COMPOUND FOR TREATING OR PREVENTING POLYQ-RELATED NEURODEGENERATIVE DISEASES**

(30) Priority: 11.03.2019 CN 201910180717; 21.10.2019 CN 201911000415
(71) Applicant: Fudan University, Shanghai 200433 (CN)
(72) Inventor: LU, Boxun, Shanghai 200438 (CN); FEI, Yiyan, Shanghai 200433 (CN); DING, Yu, Shanghai 200438 (CN); DANG, Yongjun, Shanghai 200032 (CN)
(74) Representative: Potter Clarkson
(86) International application number: PCT/CN2020/078775
(87) International publication number: WO 2020/182143

(57) **Abstract**

The present invention relates to a method for screening or identifying compound(s) for the treatment or prevention of a polyQ-related neurodegenerative disorder, and the compounds obtained by the method and uses thereof.

## Description

### FIELD OF THE INVENTION

The present invention relates to the biopharmaceutical field, and in particular to a method for screening or identifying compounds for the treatment or prevention of polyQ-related neurodegenerative disorder, and the compounds obtained by the method and uses thereof.

### BACKGROUND OF THE INVENTION

Many diseases are caused by the presence of specific proteins or excessive levels of specific proteins in cells or tissues. The activity of some specific proteins that affect diseases remains unknown. Therefore, for such diseases, the common treatment strategy is to control the protein levels. Some current methods for controlling protein levels, such as biological tools like RNAi, CRIPSR, etc., are difficult to deliver *in vivo.* Therefore, a feasible treatment strategy is to use low molecular weight compounds (compounds for short) to control the level of proteins that affect diseases. An emerging approach is to enhance the ubiquitination of disease-causing proteins and target them to the proteasome degradation pathway using proteolysis-targeting chimera (PROTAC) technology, but this method relies on certain E3 ligases, which may not be present in diseased cells. Moreover, the protein degradation ability of the proteasome is limited, and the degradation efficiency of certain large disease proteins or aggregates is low.

Neurodegenerative disorder refers to diseases caused by nervous system dysfunction resulted from abnormal death of central neurons. So far, there is no ultimate therapy that can slow down its development. Taking Huntington's disease (HD), the most common neurodegenerative disorder, as an example, it is a monogenetic disorder wherein the mutation in the CAG repeat region of the exon1 of the *HTT* gene located in the patient's chromosome 4 results in the expansion of the polyglutamine (polyQ) of the synthesized mutant protein (mHTT). mHTT is susceptible to shearing and aggregating, and causes toxicity which eventually leads to dysfunction and death of specific neuron. The current methods for controlling mHTT levels through low molecular weight compounds lack specificity, and may cause side effects. Furthermore, these methods are nonallele-selective and unable to distinguish between mHTT and wild-type HTT protein (wtHTT), which will lead to a decrease in the level of wtHTT which has important biological functions.

Therefore, there is an urgent need for effective screening or identification methods in the art to obtain compounds that can be used to treat or prevent polyQ-related neurodegenerative disorder.

### DISCLOSURE OF THE INVENTION

In one aspect, the present invention provides a method for screening or identifying compound(s) for the treatment or prevention of a polyQ-related neurodegenerative disorder, comprising
(I): bringing the candidate compound(s) into contact with a test system, the test system comprising
   (1) a protein with abnormally expanded polyQ or a fragment thereof containing the polyQ part, and
   (2) a LC3B protein or a homologue thereof or a fragment thereof; and
(II): determining the ability of the candidate compound(s) to modulate the binding between (1) a protein with abnormally expanded polyQ or a fragment thereof containing the polyQ part, and (2) a LC3B protein, a homologue thereof, or a fragment thereof; and selecting the compound(s) that positively modulate the binding.

In another aspect, the present invention provides the use of the compound obtained by the method of the present invention or the pharmaceutical composition thereof in the preparation of a medicament for the treatment or prevention of a polyQ-related neurodegenerative disorder or use in the preparation of a diagnostic reagent or a kit for detecting subjects considered to be suffering from or susceptible to a polyQ-related neurodegenerative disorder.

In another aspect, the present invention provides the use of a compound or a pharmaceutical composition thereof in the preparation of a medicament for the treatment or prevention of a polyQ-related neurodegenerative disorder; wherein the compound positively modulate the binding between (1) a protein with abnormally expanded polyQ or a fragment thereof containing the polyQ part, and (2) a LC3B protein, a homologue thereof, or a fragment thereof.

### BRIEF DESCRIPTION OF THE DRAWINGS

Figure 1: Detection of the affinity of the compounds in a system with both LC3B and full-length HTT.
Figure 2: Effects of compounds on HTT levels in cortical neurons of Hdh^{Q140/Q7} mice.
Figure 3: Effects of compound 1 and compound 2 on HTT levels in cortical neurons of Hdh^{Q7/Q7} mice.
Figure 4: Detection of HTT levels in cortical neurons of Hdh^{Q140/Q7} mice using a variety of antibodies after treatment with the compounds.
Figure 5: Detection of the N-terminal fragments of mHTT in cortical neurons of Hdh^{Q140/Q7} mice by antibodies MW1 and 3B5H10 after treatment with the compounds.
Figure 6: Cell viability test results of Hdh^{Q140/Q7} mouse cortical neurons after treatment with the compounds.
Figure 7: Effects of compounds on mHTT levels in primary fibroblasts of HD patients at the concentration of 100 nM.
Figure 8: Effects of compounds on mHTT levels in immortalized fibroblasts of HD patients.
Figure 9: Compound 4 reduces mHTT levels in immortalized fibroblasts of HD patients.
Figure 10: Effects of compounds on mHTT levels in HD patient iPSC-derived neurons.
Figure 11: Effects of compounds on the apoptosis of HD patient iPSC-derived neurons. The scale bar is 50 µm.
Figure 12: Effects of compounds on the apoptosis of HD patient iPSC-derived neurons. I, II, III, IV represent compound 1, compound 2, compound 3, compound 4, respectively.
Figure 13: Effects of compounds on mHTT levels in Huntington's disease Drosophilae.
Figure 14: Effects of compounds on the survival rate of Huntington's disease Drosophilae. I, II, III, IV represent compound 1, compound 2, compound 3, compound 4, respectively.
Figure 15: Effects of compounds on the climbing performance of Huntington's disease Drosophilae. I, II, III, IV represent compound 1, compound 2, compound 3, compound 4, respectively.
Figure 16: Effects of intracerebroventricular injection of compounds on the levels of mHTT and wtHTT in the cortices of Huntington's disease mice.
Figure 17: Effects of intraperitoneal injection of compounds on the levels of mHTT and wtHTT in the cortices and striata of Huntington's disease mice.
Figure 18: Detection of mHTT aggregates in the cortices of Huntington's disease mice after intraperitoneal injection of the compound.
Figure 19a and Figure 19b: Effects of intraperitoneal injection of compounds on behavioral defects in Huntington's disease mice. I, II, III, IV represent compound 1, compound 2, compound 3, compound 4, respectively.
Figure 20-1, Figure 20-2, Figure 20-3 and Figure 20-4: The compound does not affect the autophagy function and the levels of control proteins of cultured Hdh^{Q140/Q7} mouse cortical neurons.
Figure 21: Intraperitoneal injection of the compound does not affect the level of SQSTM1/p62 in mouse cortices.
Figure 22: The compound selectively enhances the binding between mHTT and LC3B.
Figure 23: Effects of compounds on the co-localization between mHTT and LC3B in Hela cells. White arrows indicate representative co-localization points. Parts of the images are amplified to show the co-localization points. The scale bar is 10 µm.
Figure 24: Effects of compounds on the co-localization between mHTT and LC3B in STHdh^{Q111/Q111} cells.
Figure 25: Effects of compounds on the level of proteins containing polyglutamine in HEK293T cells.
Figure 26: Effects of compounds on the level of ATXN3 protein in fibroblasts of patients with spinocerebellar ataxia type 3.
Figure 27: Effects of compounds on the level of mutant ATXN1 protein in HEK293T cells.

### DETAILED DESCRIPTION

The specific embodiments are provided below to illustrate technical contents of the present disclosure. Those skilled in the art can easily understand other advantages and effects of the present disclosure through the contents disclosed in the specification. The present disclosure can also be implemented or applied through other different specific embodiments. Various modifications and variations can be made by those skilled in the art without departing from the spirit of the present disclosure.

### General terminology and definitions

Unless defined otherwise, all technical and scientific terms used herein have the same meaning as is commonly understood by one of skill in the art. If there is a contradiction, the definition provided in this application shall prevail. When a trade name is present herein, it refers to the corresponding commodity or the active ingredient thereof. All patents, published patent applications and publications cited herein are incorporated herein by reference.

The terms "including", "comprising", "having", "containing", or "relating to" and other variants thereof, as used herein, are inclusive or open-ended, and not exclusive of other elements or steps of methods that are not enumerated. Those skilled in the art should understand that the above terms such as "include" also cover the meaning of "consist of".

The term "one or more " or the similar expression "at least one" can mean, for example, 1, 2, 3, 4, 5, 6, 7, 8, 9, 10 or more.

When the lower limit and the upper limit of a numerical range are disclosed, any numerical value and any included range falling within the range are specifically disclosed. In particular, each value range of the values disclosed herein should be understood to mean each value and range covered in a wider range. For example, the expression "ATXN1 with polyQ length ≥ 40" can cover the case where the polyQ length ≥ 41, and can cover, for example, the case where the polyQ length is 92. For example, "ATXN2 with polyQ length ≥ 33" can cover the case where the polyQ length ≥ 34. For example, "ATXN3 with polyQ length ≥ 41" can cover the case where the polyQ length ≥ 62 and can cover the case where the polyQ length is 74 or 68, for example. For example, "ATXN3 with polyQ length < 41" can cover the case where the polyQ length is 27 or 28. For example, "ATXN7 with polyQ length≥ 19" can cover the case where the polyQ length≥ 38. For example, "TBP with polyQ length ≥ 44" can cover the case where the polyQ length ≥ 45. For example, "ATN1 with polyQ length ≥ 39" can cover the case where the polyQ length ≥ 49. For example, "HTT with a polyQ length ≥ 36" can cover the case where the polyQ length is 47, 49, 55, 68, 72, 73, 111, 128, or 140. For another example, "HTT with polyQ length < 36" can cover the case where the polyQ length is 7, 16, 19, 23, or 25. For example, "AR with polyQ length ≥ 37" can cover the case where the polyQ length ≥ 38.

The term "optional" or "optionally" means that the event or situation described later may or may not occur, and the description includes occurrence of said event or situation and non-occurrence of said event or situation.

The term "neurodegenerative disorder" refers to a disease caused by the loss or pathological change of neurons and/or their myelin sheaths. Characteristic pathological structures, such as insoluble aggregates of protein, can be observed in the brain neurons of patients with neurodegenerative disorders. Insoluble aggregates may produce cytotoxicity, further leading to neuron loss and disease.

The term "polyQ" or "polyglutamine" refers to the polyglutamine tract contained by the protein. Glutamine is encoded by cytosine-adenine-guanine (CAG) in the gene. The length of the polyglutamine is related to the number of CAG repeats in gene exons. Therefore, the increase in the number of CAG repeats in gene exons result in polyglutamine expansions in the synthesized protein. Proteins with abnormally expanded polyQ are known to be associated with some neurodegenerative disorder. As used herein, the gene name can use the form of "Q+number" to indicate the number of CAG repeats in exons, such as Q25 or Q72, which respectively indicate 25 repeats or 72 repeats of CAG in exons. In the protein name, the length of the polyglutamine can be expressed in the form of "Q+number" as above, such as Q27 or Q73, which means that the length of the polyglutamine is 27 Q (glutamine) or 73 Q, respectively. The CAG repetitions or glutamine repetitions indicated in the form of "Q+number" herein are all continuous repetitions. Unless otherwise specified, the length of polyQ as used herein refers to the length of the continuous polyglutamine.

The term "polyQ-related neurodegenerative disorder" refers to neurodegenerative disorder associated with abnormal expansion of polyQ, or neurodegenerative disorder that responds to levels of proteins containing expanded polyQ. Neurodegenerative disorders are a group of disorders with clinical and genetic heterogeneity.

"Normal polyQ" refers to the polyQ of a protein in a normal physiological state that has a length less than a specific number. Correspondingly, "abnormally expanded" means that the polyQ of the protein has a length longer than the normal length. For diseases or pathological conditions, the length of polyQ is longer.

As used herein, "normal polyQ protein" includes polyQ (i.e., its polyQ part) of a normal length.

As used herein, a "protein with abnormally expanded polyQ" includes a polyQ (i.e., its polyQ part) of abnormal length, for example, a polyQ part with the length ≥ 40, ≥ 33, ≥ 41, ≥ 19,≥ 46, ≥ 44, ≥ 39, ≥ 36 or ≥ 37.

As an example, the length of a fragment of a normal or abnormal polyQ protein can be 1% or higher, 2% or higher, 3% or higher, 4% or higher, 5% or higher, 10% or higher, 15% or higher, 20% or higher, 25% or higher, 30% or higher, 35% or higher, 40% or higher, 50% or higher, 60% or higher, 70% or higher, 80% or higher, 90% or higher or 95% or higher of the full length of the corresponding protein, but contains the complete polyQ part of a normal or abnormal length.

In a preferred embodiment, when used in the method of the present invention, especially a high-throughput method, a fragment of a protein with abnormally expanded polyQ can be used. It contains the polyQ part of the protein, and the length can be only 1% or higher, 2% or higher, 3% or higher, 4% or higher of the corresponding protein. In an exemplary embodiment, the "protein with abnormally expanded polyQ" or a fragment thereof used may comprise the amino acid sequence of SEQ ID No: 1, 2 or 3. In another exemplary embodiment, the "normal polyQ protein" or a fragment thereof used may comprise the amino acid sequence of SEQ ID No: 4 or 5. In another exemplary embodiment, the "normal polyQ protein" or a fragment thereof used may comprise the amino acid sequence of SEQ ID No: 7. In another exemplary embodiment, the "normal polyQ protein" or a fragment thereof used may comprise the amino acid sequence of SEQ ID No: 6.

"Normal polyQ protein" or "protein with abnormally expanded polyQ" can be a naturally occurring protein that contains a polyQ part of a normal or abnormal length. For example, it can be from humans (for example, ATXN1, ATXN2, ATXN3, ATXN7, ATXN12, TBP, ATN1, HTT or AR) or from other animals, for example, from mice (for example, mouse HTT protein, whose polyQ can have a length such as 140 or 111), or from insects, fish, rodents, artiodactyls, primates.

"Normal polyQ protein" or "protein with abnormally expanded polyQ" can also be artificially engineered or modified protein, but still has its corresponding polyQ part. Such modification can be carried out for the object of processing, purification, characterization, tracing, etc., for example, adding MBP tag or His tag or fusing with green fluorescent protein, or introducing various amino acid substitutions, additions, or deletions. Such technology is well-known to those skilled in the art. Preferably, such a protein contains the corresponding normal or abnormal polyQ part, and has 15% or higher, 20% or higher, 25% or higher, 30% or higher, 35% or higher, 40% or higher, 50% or higher, 60% or higher, 70% or higher, 80% or higher, 90% or higher, or 95% or higher sequence identity with the corresponding protein with normal or abnormal polyQ. Such a protein can be cut or spliced from the full length or fragment(s) of the corresponding protein with normal or abnormal polyQ, and optionally include modifications, or it can be designed de novo.

As used herein, "a protein containing an expanded polyglutamine" refers to a specific protein wherein the polyglutamine tract is expanded. In some cases, for certain diseases, "expanded polyglutamine" can refer to "abnormal polyQ expansion". In this case, "protein containing expanded polyglutamine" can have the same meaning as "protein with abnormally expanded polyQ" or "abnormally expanded polyQ protein" described herein. However, in some other cases, the polyQ length of the "expanded polyglutamine" can satisfy both: (1) for some specific diseases, such polyQ is longer than the normal length, which is abnormal expansion; (2) for other specific diseases, the length of such polyQ is normal length and does not involve abnormal expansion. Such "protein containing expanded polyglutamine" can also be understood as "protein with abnormally expanded polyQ" or "abnormally expanded polyQ protein", which is covered by the scope of the present invention and can be used by the skilled person according to actual needs.

As an example, polyQ-related neurodegenerative disorders include but are not limited to spinocerebellar ataxia (SCA) type 1 (polyQ length ≥ 41), type 2 (polyQ length ≥ 34), and type 3 (polyQ length ≥ 62), type 7 (polyQ length ≥ 38), type 12 (polyQ length ≥ 46), type 17 (polyQ length ≥ 45); and dentatorubral-pallidoluysian atrophy (DRPLA, polyQ length ≥ 49), Huntington's Disease (HD, polyQ length ≥ 36) and spinal-bulbar muscular atrophy (SBMA, polyQ length ≥ 38). These diseases are caused by the expansion of CAG repeat regions on ATXN1, ATXN2, ATXN3, ATXN7, ATXN12, TBP, ATN1, HTT and AR genes, respectively (Lesley Jones et al., DNA repair in the trinucleotide repeat disorders, Lancet Neurol. 2017; 16: 88-96). Among them, spinocerebellar ataxia type 3 (SCA3, also known as Machado-Joseph disease, MJD) is the most common autosomal dominant spinocerebellar ataxia and is second only to HD as a common polyQ-related disease in the world. SCA3 is caused by the abnormally expanded polyQ at the C-terminus of the coded protein ATXN3 resulting from the increase in the number of CAG repeats of the Ataxin-3 gene (ATXN3; also known as the MJD1 gene). Examples of normal polyQ proteins described herein include, but are not limited to, ATXN1 with polyQ length < 40, ATXN2 with polyQ length < 33, ATXN3 with polyQ length < 41, ATXN7 with polyQ length < 19, ATXN12 with polyQ length < 46, TBP with polyQ length < 44, ATN1 with polyQ length < 39, HTT with polyQ length < 36, and AR with polyQ length < 37. Correspondingly, examples of the proteins with abnormally expanded polyQ described herein include, but are not limited to, ATXN1 with polyQ length ≥ 40, ATXN2 with polyQ length ≥ 33, ATXN3 with polyQ length ≥ 41, ATXN7 with polyQ length ≥ 19, and ATXN12 with polyQ length≥ 46, TBP with polyQ length ≥ 44, ATN1 with polyQ length ≥ 39, HTT with polyQ length ≥ 36, and AR with polyQ length ≥ 37.

Human microtubule-associated protein 1 light chain 3β (MAP1LC3B, LC3B) belongs to the microtubule-associated protein 1 light chain 3 (LC3) family in the Atg8 protein family. LC3B is located on the membranes of pro-autophagosomes and autophagosomes, and can control the formation of autophagosomes. LC3B is a key protein in the process of autophagy.

The form of LC3B protein that can be used includes, for example, LC3B-I and LC3B-II, but it is not limited thereto. For LC3B protein, for example, see Uniprot Accession: Q9GZQ8.

Homologues of a LC3B protein can be used herein, as long as it can be used in the method of the present invention, for example, interacting with an abnormally expanded polyQ protein or a fragment thereof containing the polyQ part.

The homologues of a LC3B protein used herein can be derived from eukaryotes, such as yeast or other non-human animals, such as insects (for example, Drosophila), fish, rodents, artiodactyls, primates, etc. Homologues of a LC3B protein can also be derived from other proteins with similar structure and function, such as LC3A, LC3C, GABARAP and GABARAPL1, but are not limited to thereto. For example, see LC3A (Uniprot Accession: Q9H492-1 and Q9H492-2), LC3C (Uniprot Accession: Q9BXW4), GABARAP (Uniprot Accession: 095166) and GABARAPL1 (Uniprot Accession: Q9H0R8-1 and Q9H0R8-2), but not limited to thereto.

As used herein, a fragment of a LC3B protein or a homologue thereof can also be used, as long as it can be used in the method of the present invention, for example, interacting with an abnormally expanded polyQ protein or the fragment thereof containing the polyQ part. Such a fragment, for example, may have 25% or higher, 30% or higher, 35% or higher, 40% or higher, 50% or higher, 60% or higher, 70% or higher, 80% or higher, 90% or higher, 95% or higher or 100% identity with a LC3B protein or a homologue thereof.

A protein or a fragment thereof can be used to achieve the purpose of the present invention, wherein the protein has identity or homology with a LC3B protein or a homologue thereof, as long as it can be used in the method of the present invention. For example, such protein or fragment interacts with an abnormally expanded polyQ protein or the fragment thereof containing the polyQ part. For example, the said identity may be 25% or higher, 30% or higher, 35% or higher, 40% or higher, 50% or higher, 60% or higher, 70% or higher, 80% or higher, 90% or higher, 95% or higher or 100%.

In an exemplary embodiment, the LC3B protein, the homologue thereof, or the fragment thereof has 25% or higher, 30% or higher, 35% or higher, 40% or higher, 50% or higher, 60% or higher, 70% or higher, 80% or higher, 90% or higher, 95% or higher or 100% sequence identity with the amino acid sequence of SEQ ID NO: 8. Those skilled in the art should understand that this can also mean that the LC3B protein, the homologue thereof, or the fragment thereof may contain such sequences.

Those skilled in the art should also understand that the mature form of the following can be used: a LC3B protein, a homologue thereof, or a fragment thereof, and/or, a protein with abnormally expanded polyQ, or, a fragment thereof containing the polyQ part. For example, the C-terminal amino acids 121-125 in a LC3B protein such as SEQ ID NO: 8 can be clipped to obtain the mature form of the LC3B protein. Such a technical solution is also within the scope of the present invention.

Those skilled in the art should understand that, to be used in the present invention, the LC3B protein, the homologue thereof, or the fragment thereof and/or the protein with abnormally expanded polyQ or the fragment thereof containing the polyQ part can be modified according to actual needs. Such a technical solution is also covered by the scope of the present invention. Such modification may include, but is not limited to, adding tags (such as GST or HIS) or labeling, or substituting, deleting, adding or replacing some of the amino acids. For example, for the convenience of cutting tags, a small amount of modification can be made to the termini of the sequence of SEQ ID NO: 8. For example, the M at the N-terminal of SEQ ID NO: 8 can be replaced with GG, and then the modified or unmodified sequence can be optionally added with a tag such as GST, and used in the present invention. Such a modified sequence, or a technical solution corresponding to a sequence having 25% or higher, 30% or higher, 35% or higher, 40% or higher, 50% or higher, 60% or higher, 70% or higher, 80% or higher, 90% or higher, 95% or higher or 100% sequence identity with the modified sequence, is also within the scope of the present invention.

The term "affinity screening" refers to the process of detecting the binding affinity between a sample and a target. The detection methods used for affinity screening can be, for example, absorbance methods, radiation methods (such as proximity scintillation analysis), fluorescence methods (such as fluorescence resonance energy transfer, fluorescence polarization detection, especially time-dependent fluorescence technology), chemiluminescence methods (such as amplified luminescent proximity homogeneous assay, ALPHAScreen), surface plasmon resonance (SPR, can be implemented, for example, using GE's Biacore series), isothermal titration calorimetry (ITC), micro thermophoresis (MST) or oblique-incidence reflectivity difference.

As used herein, "sequence identity" between two amino acid sequences means the percentage of amino acids that are identical between the sequences. "Sequence homology" means the percentage of amino acids that are identical or represent conservative amino acid substitutions. For sequence comparison, usually a sequence is used as a reference sequence to compare the test sequence with. When using the sequence comparison algorithm, the test and reference sequences are input into the computer. If necessary, the sub-sequence coordinates are specified, and the sequence algorithm program parameters are specified. The percent sequence identity of the test sequence relative to the reference sequence is calculated by the sequence comparison algorithm based on the specified program parameters. Examples of algorithms suitable for determining sequence identity and sequence similarity percentages include but are not limited to BLAST and BLAST 2.0 algorithms. The software to perform BLAST analysis is available from the National Center for Biotechnology Information (NCBI).

The term "target sample" includes various sample types that are obtained from a subject and can be used for diagnostic analysis. The target sample herein can be, for example, any cell sample, biological fluid (including blood, serum, spinal fluid, etc.) or any biopsy sample obtained from a subject's tissue.

### Screening method

The concept of the present invention is based at least in part on the discovery of the following new mechanism: a compound that can bind to both of the key protein LC3B in the autophagy process and the protein with abnormally expanded polyQ can promote the degradation of protein with abnormally expanded polyQ through cell autophagy, so as to achieve the purpose of reducing the level of protein with abnormally expanded polyQ, treating or preventing corresponding diseases.

Therefore, in one aspect, the present invention provides a method for screening or identifying compound(s) for the treatment or prevention of a polyQ-related neurodegenerative disorder, comprising
(I): bringing the candidate compound(s) into contact with a test system, the test system comprising
   (1) a protein with abnormally expanded polyQ or a fragment thereof containing the polyQ part, and
   (2) a LC3B protein, a homologue thereof, or a fragment thereof; and
(II): determining the ability of the candidate compound(s) to modulate the binding between (1) a protein with abnormally expanded polyQ or a fragment thereof containing the polyQ part, and (2) a LC3B protein, a homologue thereof, or a fragment thereof; and selecting the compound(s) that positively modulate the binding.

The system includes, but is not limited to, a solution system, a subcellular system, a cell (cell culture) system, a tissue system, an organ system, or an animal system.

In one embodiment, candidate compounds can be provided as a compound library. Such libraries can be commercially available or designed and synthesized. Candidate compounds can include peptides, peptidomimetics and small organic molecules, etc. For example, a candidate compound may be selected from a designed and synthesized compound, a compound with a determined structure in a database (e.g. Pubmed), or a compound synthesized de novo. In a specific embodiment, the candidate compound(s) are selected from small organic molecules.

The term "small organic molecule" or "low molecular weight compound" refers to a molecule that is comparable in size to organic molecules commonly used in drugs. The term excludes biological macromolecules (such as proteins, nucleic acids, etc.), but covers small molecular weight proteins or their derivatives, such as dipeptides, tripeptides, tetrapeptides, pentapeptides, etc.

In a specific embodiment, the size of the small organic molecule is about 100 to about 2000 Da, preferably about 200 to about 1000 Da, for example, about 200 to about 900 Da, about 200 to about 800 Da, about 200 to about 700 Da, about 200 to about 600 Da, about 200 to about 500 Da.

The protein with abnormally expanded polyQ or a fragment thereof containing the polyQ part used in the method of the present invention is determined by the targeted polyQ-related neurodegenerative disorder.

In one embodiment, according to the polyQ-related neurodegenerative disorder, the protein with abnormally expanded polyQ comprises a polyQ part with the polyQ length ≥ 40, ≥ 33, ≥ 41, ≥ 19, ≥ 46, ≥ 44, ≥ 39, ≥ 36, or ≥ 37. In a preferred embodiment, the protein with abnormally expanded polyQ comprises ATXN1 with polyQ length ≥ 40 or a fragment thereof containing the polyQ part. In a preferred embodiment, the protein with abnormally expanded polyQ comprises ATXN2 with a polyQ length ≥ 33 or a fragment thereof containing the polyQ part. In another preferred embodiment, the protein with abnormally expanded polyQ comprises ATXN3 with polyQ length ≥ 41 or a fragment thereof containing the polyQ part. In another preferred embodiment, the protein with abnormally expanded polyQ comprises ATXN7 with a polyQ length ≥ 19 or a fragment thereof containing the polyQ part. In a preferred embodiment, the protein with abnormally expanded polyQ comprises ATXN12 with a polyQ length ≥ 46 or a fragment thereof containing the polyQ part. In another preferred embodiment, the protein with abnormally expanded polyQ comprises TBP with a polyQ length ≥ 44 or a fragment thereof containing the polyQ part. In another preferred embodiment, the protein with abnormally expanded polyQ comprises ATN1 with a polyQ length ≥ 39 or a fragment thereof containing the polyQ part. In a preferred embodiment, the protein with abnormally expanded polyQ comprises HTT with a polyQ length ≥ 36 or a fragment thereof containing the polyQ part. In another preferred embodiment, the protein with abnormally expanded polyQ comprises AR with a polyQ length ≥ 37 or a fragment thereof containing the polyQ part.

The "positive regulation" of binding refers to that, when determined under the same conditions, comparing with the case when the compound is absent, when the compound is present, the binding strength between the proteins or the fragments thereof involved in the binding becomes greater, or the binding amount becomes greater. In one embodiment, the positive modulation of the binding by a compound can be measured at the molecular level, for example, by measuring the increase in binding affinity between proteins or the fragments thereof in the presence of the compound. Positive regulation can also be verified by separately measuring the binding strength (such as affinity) of the compound to the proteins or the fragments thereof involved in the binding. A positive regulation can be determined provided that, the compound has affinity for each of the proteins or the fragments thereof involved in the binding, and at the same time does not have affinity activity or has a lower affinity for the corresponding reference.

The present invention does not specifically limit the implementation method of step (II). In one embodiment, step (II) is implemented in the following manner: respectively in the presence/absence of the candidate compound(s), determining the parameters that characterize the binding strength between (1) a protein with abnormally expanded polyQ or a fragment thereof containing the polyQ part, and (2) a LC3B protein, a homologue thereof, or a fragment thereof; and comparing the parameters obtained in the same test in the presence/absence of the candidate compound(s); and selecting the compound(s) that positively regulates the binding of (1) a protein with abnormally expanded polyQ or a fragment thereof containing the polyQ part, and (2) a LC3B protein, a homologue thereof, or a fragment thereof. Depending on the binding test performed, the above-mentioned "parameters" can vary, but in particular can be, for example, absorbance value, radioactive signal and/or distribution in the sample, fluorescence signal intensity and/or distribution in the sample, heat change, reflected light intensity, reflected light phase change, etc.

In one embodiment, the binding between (1) a protein with abnormally expanded polyQ or a fragment thereof containing the polyQ part, and (2) a LC3B protein, a homologue thereof, or a fragment thereof is affinity binding, and step (II) comprises: respectively in the presence/absence of the candidate compound(s), determining the binding affinity between (1) and (2), and/or, respectively in the presence/absence of the candidate compound(s), determining the binding amount of (1) and (2); and comparing the measured values obtained in the presence/absence of the candidate compound(s); and selecting the candidate compound(s) if the binding of candidate compound(s) to the above-mentioned proteins or fragments thereof changes positively when the candidate compound(s) are present,.

In one embodiment, the method for determining the binding between the proteins or fragments thereof in step (II) is selected from in vitro pull-down assay, Split-TEV, co-immunoprecipitation (co-IP), affinity chromatography (AP), complex co-purification, enzyme-linked immunosorbent assay (ELISA), fluorescent molecular sieve (Fluorescence-SEC), yeast two-hybrid (yeast two-hybrid, Y2H), HIP-HOP (haploinsufficiency profiling and homozygous deletion profiling), time-resolved fluorescence resonance energy transfer (TR-FRET), chemiluminescence method, surface plasmon resonance (SPR), isothermal titration calorimetry, micro thermophoresis and any combination thereof. In a preferred embodiment, the method for determining the binding is selected from the group consisting of in vitro pull-down test, co-immunoprecipitation, enzyme-linked immunosorbent assay, fluorescent molecular sieve, time-resolved fluorescence resonance energy transfer, and any combination thereof.

In another embodiment, before step (I), a step of pre-screening the candidate compound(s) for affinity is also included; comprising
(A): detecting the binding affinity of the candidate compound(s) to (1) a protein with abnormally expanded polyQ or a fragment thereof containing the polyQ part, and/or (B): detecting the binding affinity of the candidate compound(s) to (2) a LC3B protein, a homologue thereof, or a fragment thereof;
and (C): selecting the compound(s) that have binding affinity to (1) a protein with abnormally expanded polyQ or a fragment thereof containing the polyQ part and/or (2) a LC3B protein, a homologue thereof, or a fragment thereof.

Wherein step (A) and step (B) can be carried out independently in any order. For example, step (A) and step (B) can be carried out separately in different systems at the same time. Step (A) can be carried out first, then step (B), and vice versa. In a preferred embodiment, the following compound is selected: a compound that have binding affinity to (1) a protein with abnormally expanded polyQ or a fragment thereof containing the polyQ part, and (2) a LC3B protein, a homologue thereof, or a fragment thereof.

As needed, step (A) and step (B) can be independently carried out or not carried out, and can also be carried out at least once in a certain manner each independently (for example, each independently once, twice, 3 times or more times). Such manner can each independently be qualitative or quantitative as needed.

In a preferred embodiment, step (A) may comprise: determining the affinity reaction equilibrium dissociation constant (Kd) between the candidate compound(s) and (1) a protein with abnormally expanded polyQ or a fragment thereof containing the polyQ part; and selecting the compound whose affinity reaction equilibrium dissociation constant is 100µM or less, preferably 10 µM or less, particularly preferably 1 µM or less, such as 600 nM or less, 500 nM or less, 400 nM or less, 300 nM or less, 200 nM or less, 100 nM or less, etc.

In a preferred embodiment, step (B) may comprise: determining the affinity reaction equilibrium dissociation constant between the candidate compound(s) and (2) a LC3B protein, a homologue thereof, or a fragment thereof; and selecting the compound whose affinity reaction equilibrium dissociation constant is 100µM or less, preferably 10 µM or less, particularly preferably 1 µM or less, such as 600 nM or less, 500 nM or less, 400 nM or less, 300 nM or less, 200 nM or less, 100 nM or less, etc.

In an alternative, step (A) and step (B) are carried out simultaneously in the same system, that is, the candidate compound(s) are brought into contact with (1) a protein with abnormally expanded polyQ or a fragment thereof containing the polyQ part, and (2) a LC3B protein, a homologue thereof, or a fragment thereof at the same time; and detecting the binding affinity of candidate compound(s) to the protein or the fragment thereof, respectively.

In one embodiment, the method for detecting binding affinity in step (A) or step (B) is selected from the group consisting of proximity scintillation analysis, fluorescence resonance energy transfer, fluorescence polarization detection, fluorescent molecular sieves, micro thermophoresis, chemiluminescence, surface plasmon resonance, isothermal titration calorimetry, oblique-incidence reflectivity difference and any combination thereof. In a preferred embodiment, the method for detecting binding affinity in step (A) or step (B) is selected from the group consisting of fluorescence resonance energy transfer, fluorescence polarization detection, oblique-incidence reflectivity difference and any combination thereof. In a particularly preferred embodiment, the method for detecting the binding affinity in step (A) or step (B) is the oblique-incidence reflectivity difference.

In a preferred embodiment, the step of pre-screening the candidate compound(s) for affinity is performed by high-throughput screening using the oblique-incidence reflectivity difference; comprising:
(a) immobilizing the candidate compound(s) to prepare a compound chip, and scanning the chip to obtain the image before incubation;
(b) incubating the compound chip with the target protein, and scanning the chip to obtain the image after incubation;
   wherein the target protein is (1) a protein with abnormally expanded polyQ or a fragment thereof containing the polyQ part or (2) a LC3B protein, a homologue thereof, or a fragment thereof; and
(c) analyzing the difference image (the image after incubation minus the image before incubation); and selecting the compound(s) that can bind to both (1) and (2).

In another embodiment, the method used in step (A) or step (B) to determine the equilibrium dissociation constant is selected from surface plasmon resonance, isothermal titration calorimetry and micro thermophoresis.

In another embodiment, the method of the present invention may further include step (D): detecting the binding of the candidate compound(s) to a normal polyQ protein or a fragment thereof containing the polyQ part. This step may be carried out, for example, after step (II), or after step (C).

In one embodiment, the binding is binding affinity. In one embodiment, a compound that does not bind to normal polyQ protein or a fragment thereof containing the polyQ part is selected. Step (D) can be carried out or not carried out as needed, or carried out at least once (for example, once, twice, three times or more). When carried out more than once, the normal polyQ polypeptide used each time can be independently normal polyQ protein or a fragment thereof containing the polyQ part.

The normal polyQ protein used in step (D) is the normal polyQ protein corresponding to the protein with abnormally expanded polyQ used in step (I). In one embodiment, the protein with abnormally expanded polyQ used in step (I) comprises HTT with abnormally expanded polyQ or a fragment thereof containing the polyQ part. Correspondingly, if step (D) is present, the normal polyQ protein used therein includes normal HTT or a part thereof containing polyQ. In an exemplary embodiment, the protein with abnormally expanded polyQ used in step (I) is HTT with abnormally expanded polyQ or a fragment thereof containing the polyQ part, for example, HTT with polyQ length ≥ 36 or a fragment thereof containing the polyQ part, such as the amino acid sequence of SEQ ID NO: 1 (HTTexon1-Q72). Correspondingly, if step (D) is present, the normal polyQ protein used is HTT with normal polyQ length or a fragment thereof containing the polyQ part, for example, HTT with polyQ length < 36 or a fragment thereof containing the polyQ part, such as the amino acid sequence of SEQ ID NO: 4 (HTT-Q23) or the amino acid sequence of SEQ ID NO: 5 (HTTexon1-Q25).

In another embodiment, the protein with abnormally expanded polyQ used in step (I) comprises ATXN3 with abnormally expanded polyQ or a fragment thereof containing the polyQ part. Correspondingly, if step (D) is present, the normal polyQ protein used therein contains normal ATXN3 or a part thereof containing polyQ. In an exemplary embodiment, the protein with abnormally expanded polyQ used in step (I) is ATXN3 with abnormally expanded polyQ or a fragment thereof containing the polyQ part, for example, ATXN3 with a polyQ length ≥ 41 or a fragment thereof containing the polyQ part, such as the amino acid sequence of SEQ ID NO: 2 (ATXN3-Q74). Correspondingly, if step (D) is present, the normal polyQ protein used is ATXN3 with normal polyQ length or a fragment thereof containing the polyQ part, for example, ATXN3 with polyQ length < 41 or a fragment thereof containing the polyQ part, such as amino acid sequence of SEQ ID NO: 7 (ATXN3-Q27).

In another embodiment, the protein with abnormally expanded polyQ used in step (I) comprises ATXN1 with abnormally expanded polyQ or a fragment thereof containing the polyQ part. Correspondingly, if step (D) is present, the normal polyQ protein used therein comprises normal ATXN1 or a part thereof containing polyQ. In an exemplary embodiment, the protein with abnormally expanded polyQ used in step (I) is ATXN1 with abnormally expanded polyQ or a fragment thereof containing the polyQ part, for example, ATXN1 with polyQ length ≥ 40 or a fragment thereof containing the polyQ part, such as the amino acid sequence of SEQ ID NO: 3 (ATXN1-Q92). Correspondingly, if step (D) is present, the normal polyQ protein used is ATXN1 with normal polyQ length or a fragment thereof containing the polyQ part, for example, ATXN1 with polyQ length < 40 or a fragment thereof containing the polyQ part.

In another embodiment, the protein with abnormally expanded polyQ used in step (I) comprises polyQ of abnormal length, which is labeled with a detectable molecule. In one embodiment, the protein with abnormally expanded polyQ used in step (I) is a fusion protein of fluorescent protein (such as GFP or YFP) and polyQ with abnormal length. In a preferred embodiment, the protein with abnormally expanded polyQ used in step (I) is a polyQ-GFP protein containing polyQ with abnormal length, preferably selected from Q72-GFP, Q53-GFP, Q46-GFP. According to the specific disease targeted by the screening, in an alternative embodiment, the protein with abnormally expanded polyQ used in step (I) is Q38-GFP.

In another embodiment, the normal polyQ protein used in step (D) contains polyQ of normal length, which is labeled with a detectable molecule. In one embodiment, the normal polyQ protein used in step (D) is a fusion protein of fluorescent protein (such as GFP or YFP) and polyQ with normal length. In a preferred embodiment, the normal polyQ protein used in step (D) is a polyQ-GFP protein containing polyQ of normal length, preferably Q25-GFP. According to the specific disease targeted by the screening, in an alternative embodiment, the normal polyQ protein used in step (D) is Q38-GFP.

In one embodiment, the LC3B protein, the homologue thereof, or the fragment thereof have 25% or higher, 30% or higher, 35% or higher, 40% or higher, 50% or higher, 60% or higher, 70% or higher, 80% or higher, 90% or higher, 95% or higher or 100% sequence identity with the amino acid sequence of SEQ ID NO: 8. In a preferred embodiment, the homologue of the LC3B protein comes from Drosophila or mouse.

In a particular embodiment, the LC3B protein homologue is the Atg8 protein in Drosophila (31% sequence identity with human LC3B).

In yet another aspect, the present invention also provides a method for screening or identifying compound(s) for the treatment or prevention of a polyQ-related neurodegenerative disorder, comprising
(1): determining the effect of the candidate compound(s) on the level of protein with abnormally expanded polyQ in the cell; and selecting the compound(s) that can reduce the level of the protein; or
(2): determining the effect of candidate compound(s) on the subcellular localization of the protein with abnormally expanded polyQ or a fragment thereof containing the polyQ part in the cell; and selecting the compound(s) that can promote the protein with abnormally expanded polyQ or the fragment thereof containing the polyQ part to localize to cellular substructures with autophagy function such as autophagosomes; preferably, a compound that can promote the co-localization between polyQ abnormal expansion protein or a fragment thereof containing the polyQ part and LC3B protein is selected,
wherein the terms such as protein with abnormally expanded polyQ, fragment thereof containing the polyQ part, and LC3B protein are as defined above.

In one embodiment, in step (1) the following compound is selected: a compound that can reduce the level of the protein with abnormally expanded polyQ by more than 10% in the cell, preferably 20% or more, such as about 20%, about 30%, about 40%, about 50%, etc.

The term "polypeptide" refers to a polymer of amino acids of a certain length. Therefore, peptides, oligopeptides and proteins are included in the definition of "polypeptide", and these terms are used interchangeably herein. The term "polypeptide" or "protein" does not exclude post-translational modifications, including but not limited to phosphorylation, acetylation, glycosylation, etc. The protein or protein fragment of the present invention can be produced by any technique known in the art per se, such as but not limited to any chemical, biological, genetic or enzymatic technique used alone or in combination.

Knowing the amino acid sequence of the desired sequence, those skilled in the art can easily prepare the protein or protein fragment by standard techniques for producing the protein or protein fragment. For example, they can be synthesized using the well-known solid-phase method, preferably using a commercially available peptide synthesis equipment (such as those prepared by Applied Biosystems, Foster City, California) and following the manufacturer's instructions.

Alternatively, the protein or protein fragment of the present invention can be synthesized by the well-known recombinant DNA technology in the art. For example, after the DNA sequence encoding the desired (poly)peptide is incorporated into an expression vector and this vector is introduced into a suitable eukaryotic or prokaryotic host for expressing the desired protein or protein fragment, these fragments can be obtained as DNA expression products, and then they can be isolated from the host using well-known techniques.

A variety of host/expression vector combinations can be used to express nucleic acids encoding proteins or protein fragments of the invention. Useful expression vectors include, for example, segments of chromosomal, non-chromosomal and synthetic DNA sequences. Suitable vectors include but are not limited to derivatives of SV40 and pcDNA; and known bacterial plasmids, such as col EI, pCR1, pBR322, pMal-C2, pET, pGEX, pMB9 and derivatives thereof; plasmids, such as RP4; phage DNAs, such as the numerous derivatives of phage I, such as NM989, and other phage DNA, such as M13 and filamentous single-stranded phage DNA; yeast plasmids, such as the 2 microns plasmid or derivatives of the 2 microns plasmid, as well as centromeres and integrative yeast shuttle vectors; vectors useful in eukaryotic cells, such as vectors useful in insect or mammalian cells; vectors derived from combinations of plasmids and phage DNAs, such as plasmids that have been modified to employ phage DNA or the expression control sequences; etc.

Therefore, mammalian and typical human cells, as well as bacterial, yeast, fungal, insect, nematode and plant cells can be used in the present invention and can be transfected by the nucleic acid or recombinant vector defined herein. Examples of suitable cells include, but are not limited to, VERO cells; HELA cells, such as ATCC No. CCL2; CHO cell lines, such as ATCC No. CCL61; COS cells, such as COS-7 cells and ATCC No. CRL 1650 cells; W138, BHK, HepG2, 3T3, such as ATCC No. CRL6361; A549, PC12, K562 cells, 293T cells, Sf9 cells, such as ATCC No. CRL1711 and Cv1 cells, such as ATCC No. CCL70. Other suitable cells that can be used in the present invention include but are not limited to prokaryotic host cell strains, for example, *Escherichia coli* (e.g., strain DH5-[α]), *Bacillus subtilis*, *Salmonella typhimurium* or strains of the genera of *Pseudomonas*, *Streptomyces* and *Staphylococcus.* Other suitable cells that can be used in the present invention include yeast cells, such as Saccharomyces cells, such as Saccharomyces cerevisiae.

Those skilled in the art can understand that the protein or protein fragment of the present invention can be modified for purposes such as facilitating experimental operations, increasing solubility, facilitating crystallization and purification, but does not affect the function of the protein or protein fragment. Such proteins or protein fragments and use thereof also belong to the scope of the present invention.

In one embodiment, (1) a protein with abnormally expanded polyQ or a fragment thereof containing the polyQ part or (2) a LC3B protein, a homologue thereof, or a fragment thereof can be labeled with detectable molecules for screening purposes.

The detectable molecule can be composed of any compound or substance that can be detected by spectroscopic, photochemical, biochemical, immunochemical, or chemical means. For example, useful detectable molecules include radioactive substances (including those containing 32P, 25S, 3H, or 1251), fluorescent dyes (including 5-bromodeoxyuridine, fluorescein, acetamidofluorene or digitoxin), fluorescent proteins (such as GFP and YFP, where GFP can be sfGFP, for example).

In one embodiment, the detectable label is located on or bind to the amino acid residues outside the sequence of (1) a protein with abnormally expanded polyQ or a fragment thereof containing the polyQ part or (2) a LC3B protein, a homologue thereof, or a fragment thereof; and thereby minimizing or preventing any artificial influence on the binding between the proteins or protein fragments or between the candidate compound(s) and any of the proteins or protein fragments.

In a specific embodiment, the protein or protein fragment of the present invention is fused to a fluorescent protein such as a GFP tag (green fluorescent protein). In another specific embodiment, the protein or protein fragment of the present invention is labeled respectively with a suitable fluorescent group in a manner suitable for fluorescence energy transfer analysis.

Regardless of the implementation methods of steps (I), (II), (A), (B), (C), (b), step (1) or step (2) of the screening method of the present invention, a complete protein with abnormally expanded polyQ and a complete LC3B protein or a homologue thereof can be used for the assay. Alternatively, a fragment of a protein with abnormally expanded polyQ or a fragment of LC3B protein or a homologue thereof can be used in the assay; wherein the said fragment includes the binding site.

In a further aspect, the present invention provides a screening system comprising more than two parts, wherein part (A) contains an unlabeled or suitably labeled protein with abnormally expanded polyQ or a fragment thereof containing the polyQ part, and part (B) contains an unlabeled or suitably labeled LC3B protein, a homologue thereof, or a fragment thereof. Each part is independently isolated or not isolated from the other. Each part independently optionally contains suitable buffer(s) and/or detection reagent(s). In a specific embodiment, the screening system of the present invention is in the form of a kit. In another specific embodiment, the prepared compound chip is placed in the kit of the present invention, after incubating with (1) a protein with abnormally expanded polyQ or a fragment thereof containing the polyQ part, and (2) a LC3B protein, a homologue thereof, or a fragment thereof, the signal is analyzed with a suitable plate reader, and compounds that can bind to both (1) and (2) are obtained from screening.

### Compounds of the invention

The present invention also relates to compounds obtained by the method of the present invention. The compounds of the present invention encompass the pharmaceutically acceptable salts, stereoisomers, solvates, polymorphs, tautomers, isotopic compounds, metabolites or prodrugs thereof.

The term "pharmaceutically acceptable" refers to that when contacted with the patient's tissue within the scope of normal medical judgment, no undue toxicity, irritation, allergic reactions, etc. shall arise.

The pharmaceutically acceptable salts of the compounds of the present invention include acid addition salts and base addition salts thereof. The method for preparing the pharmaceutically acceptable salt of the compound of the present invention is known to those skilled in the art.

The compounds of the present invention may exist in specific geometric or stereoisomeric forms, including cis and trans isomers, (-)- and (+)-enantiomers, (*R*)- and (*S*)-enantiomers, diastereomers, (*D*)-isomers, (*L*)-isomers, and their racemic mixtures and other mixtures, for example, mixtures enriched in enantiomers or diastereomers, all of which are within the scope of the present invention.

The compounds of the present invention may exist in the form of solvates (preferably hydrates), wherein the compound of the present invention contains a polar solvent as a structural element of the compound crystal lattice, especially for example water, methanol, or ethanol. The amount of polar solvents, especially water, can be present in stoichiometric or non-stoichiometric ratios.

The present invention also covers all possible crystalline forms or polymorphs of the compounds of the present invention, which can be a single polymorph or a mixture of more than one polymorph in any ratio.

The metabolites of the compounds of the present invention are also included within the scope of the present invention, that is, substances formed in the body when the compounds of the present invention are administered. Such products can be produced, for example, by oxidation, reduction, hydrolysis, amidation, deamidation, esterification, enzymatic hydrolysis, etc. of the administered compound.

The present invention further includes within its scope the prodrugs of the compounds of the present invention, which are certain derivatives of the compounds of the present invention that have less or no pharmacological activity themselves but when administered to or on the body, can be converted into the compound of the present invention with the desired activity by, for example, hydrolytic cleavage.

The term "polymorphism" or "polymorph" refers to a single polymorph or a mixture of more than one polymorph in any ratio.

The term "crystal form" or "crystal" refers to any solid substance exhibiting a three-dimensional order, as opposed to an amorphous solid substance, which produces a characteristic X-ray powder diffraction pattern with sharp and defined peaks.

The term "amorphous" refers to any solid substance which lacks order in three dimensions.

The compound of the present invention can be applied in the form of a pharmaceutical composition. The pharmaceutical composition comprises the compound or a pharmaceutically acceptable salt, stereoisomer, solvate, polymorph, tautomer, isotopic compound, metabolite or prodrug thereof, and at least one pharmaceutical acceptable carrier.

The term "pharmaceutically acceptable carrier" refers to those substances that have no obvious stimulating effect on the organism and will not damage the biological activity and performance of the active compound. "Pharmaceutically acceptable carriers" include but are not limited to glidants, sweeteners, diluents, preservatives, dyes/colorants, flavors, surfactants, wetting agents, dispersants, disintegrants, stabilizer, solvent or emulsifier.

In one aspect, the present invention provides the use of the compound of the present invention or the pharmaceutical composition thereof in the preparation of a medicament for the treatment or prevention of a polyQ-related neurodegenerative disorder.

In another aspect, the present invention provides compounds of the present invention for use in the treatment or prevention of a polyQ-related neurodegenerative disorder.

In another aspect, the present invention provides a method of treating or preventing a polyQ-related neurodegenerative disorder, comprising administering a compound of the present invention to an individual in need thereof.

In another aspect, the present invention provides use of a compound or a pharmaceutical composition thereof that positively modulates the binding between (1) a protein with abnormally expanded polyQ or a fragment thereof containing the polyQ part, and (2) a LC3B protein, a homologue thereof, or a fragment thereof in the preparation of a medicament for the treatment or prevention of a polyQ-related neurodegenerative disorder.

In another aspect, the present invention provides a compound or a pharmaceutical composition thereof that positively regulates the binding between (1) a protein with abnormally expanded polyQ or a fragment thereof containing the polyQ part, and (2) a LC3B protein, a homologue thereof, or a fragment thereof; for treating or preventing a polyQ-related neurodegenerative disorder.

In another aspect, the present invention provides a method of treating or preventing a polyQ-related neurodegenerative disorder, including administering a compound or a pharmaceutical composition thereof that positively regulates the binding between (1) a protein with abnormally expanded polyQ or a fragment thereof containing the polyQ part, and (2) a LC3B protein, a homologue thereof, or a fragment thereof, to individuals in need thereof.

The said compound that positively regulates the binding between (1) a protein with abnormally expanded polyQ or a fragment thereof containing the polyQ part, and (2) a LC3B protein, a homologue thereof, or a fragment thereof can be obtained, for example, by the method of the present invention.

In a preferred embodiment, the polyQ-related neurodegenerative disorder is selected from spinocerebellar ataxia type 1 (SCA1), spinocerebellar ataxia type 2 (SCA2), spinocerebellar ataxia type 3 (SCA3), spinocerebellar ataxia type 7 (SCA7), spinocerebellar ataxia type 12 (SCA12), spinocerebellar ataxia type 17 (SCA17), dentatorubral-pallidoluysian atrophy (DRPLA), Huntington's disease (HD) and spinal-bulbar muscular atrophy (SBMA), especially Huntington's disease and spinocerebellar ataxia type 3. In a more preferred embodiment, the polyQ-related neurodegenerative disorder is selected from Huntington's disease and SCA3; especially Huntington's disease.

In a further aspect, the present invention provides an article of manufacture, for example in the form of a kit. The article of manufacture of the present invention contains the compound or the pharmaceutical composition of the present invention, and optionally includes a packaging material and a package insert (instructions).

In yet another aspect, the present invention provides the use of the compounds of the present invention for screening or identifying target proteins that can be degraded through the autophagy pathway. In one embodiment, for example, the compound obtained from screening can be used to find the target protein by the following method. 1) Screening or identifying proteins that can bind to the compound in affinity; and 2) determining the ability of the compound to modulate the binding between the protein obtained in 1) and a LC3B protein, a homologue thereof, or a fragment thereof; wherein, if the compound can positively regulate the binding, the protein obtained in 1) can be degraded through the autophagy pathway.

In another aspect, the present invention provides the use of the compound of the present invention or a pharmaceutical composition thereof in the preparation of a diagnostic reagent or a kit for detecting subjects considered to be suffering from or susceptible to a polyQ-related neurodegenerative disorder. In one embodiment, the aforementioned test includes the step of analyzing a target sample obtained from the subject, comprising:
i) labeling the compound with a detectable label such as a fluorescent group;
ii) treating the target sample with the compound obtained in i);
iii) detecting the position and/or intensity of the detectable signal in the target sample; and obtaining the position and/or content of the protein with abnormally expanded polyQ or the fragment thereof containing the polyQ part in the target sample.

In a preferred embodiment, the target sample is selected from cell samples, blood, serum, spinal fluid; or any biopsy sample obtained from the subject's tissue.

In a further aspect, the present invention provides a detection kit. The kit contains the compound of the present invention or a pharmaceutical composition thereof, and optionally contains reagent(s) required for detection, such as aqueous solutions, solvents, suitable detection reagent(s) such as chemiluminescence reagent(s), etc.

The invention also relates to compounds selected from:

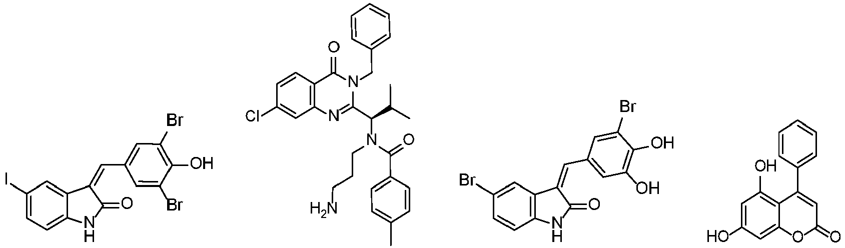

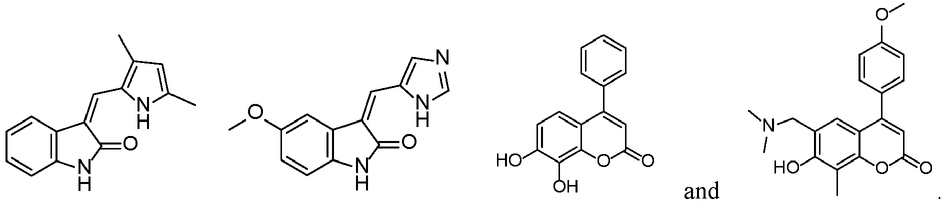

As mentioned above, the embodiments of the present invention can be listed as follows:
[1] A method for screening or identifying compound(s) for the treatment or prevention of a polyQ-related neurodegenerative disorder, comprising
   (I): bringing the candidate compound(s) into contact with a test system, the test system comprising
      (1) a protein with abnormally expanded polyQ or a fragment thereof containing the polyQ part, and
      (2) a LC3B protein, a homologue thereof, or a fragment thereof; and
   (II): determining the ability of the candidate compound(s) to modulate the binding between (1) a protein with abnormally expanded polyQ or a fragment thereof containing the polyQ part, and (2) a LC3B protein, a homologue thereof, or a fragment thereof and selecting the compound(s) that positively modulate the binding.
[2] The method of [1] above, wherein step (II) comprises:
   determining the binding affinity between (1) a protein with abnormally expanded polyQ or a fragment thereof containing the polyQ part, and (2) a LC3B protein, a homologue thereof, or a fragment thereof in the presence/absence of the candidate compound(s); and/or
   determining the binding amount of (1) a protein with abnormally expanded polyQ or a fragment thereof containing the polyQ part, and (2) a LC3B protein, a homologue thereof, or a fragment thereof in the presence/absence of the candidate compound(s).
[3] The method of [1] or [2] above, wherein the protein with abnormally expanded polyQ comprises a polyQ part of polyQ length ≥ 40, ≥ 33, ≥ 41, ≥ 19, ≥ 46, ≥ 44, ≥ 39, ≥ 36, or ≥ 37;
   preferably, the protein with abnormally expanded polyQ comprises
   ATXN1 with polyQ length ≥ 40 or a fragment thereof containing the polyQ part,
   ATXN2 with polyQ length ≥ 33 or a fragment thereof containing the polyQ part,
   ATXN3 with polyQ length ≥ 41 or a fragment thereof containing the polyQ part,
   ATXN7 with polyQ length ≥ 19 or a fragment thereof containing the polyQ part,
   ATXN12 with polyQ length ≥ 46 or a fragment thereof containing the polyQ part,
   TBP with polyQ length ≥ 44 or a fragment thereof containing the polyQ part,
   ATN1 with polyQ length ≥ 39 or a fragment thereof containing the polyQ part,
   HTT with polyQ length ≥ 36 or a fragment thereof containing the polyQ part, or
   AR with polyQ length ≥ 37 or a fragment thereof containing the polyQ part.
[4] The method of any one of the above [1] to [3], wherein (2) a LC3B protein, a homologue thereof, or a fragment thereof has 25% or higher, 30% or higher, 35% or higher, 40% or higher, 50% or higher, 60% or higher, 70% or higher, 80% or higher, 90% or higher, 95% or 100% sequence identity with the amino acid sequence of SEQ ID NO: 8.
[5] The method of any one of [1] to [4] above, wherein the method for determining the binding is selected from the group consisting of in vitro pull-down test, Split-TEV, co-immunoprecipitation, affinity chromatography, complex co-purification, enzyme-linked immunosorbent assay, fluorescent molecular sieve, yeast two-hybrid, HIP-HOP, time-resolved fluorescence resonance energy transfer, chemiluminescence method, surface plasmon resonance, isothermal titration calorimetry, micro thermophoresis and any combination thereof; preferably, the method for determining the binding is selected from the group consisting of in vitro pull-down experiments, co-immunoprecipitation, enzyme-linked immunosorbent assay, fluorescent molecular sieves, time-resolved fluorescence resonance energy transfer, and any combination thereof.
[6] The method of any one of [1] to [5] above, wherein before step (I), it further includes a step of pre-screening the candidate compound(s) for affinity, comprising:
   (A): detecting the binding affinity of the candidate compound(s) to (1) a protein with abnormally expanded polyQ or a fragment thereof containing the polyQ part, and/or (B): detecting the binding affinity of the candidate compound(s) to (2) a LC3B protein, a homologue thereof, or a fragment thereof; and
   (C): selecting the compound(s) that have binding affinity to (1) a protein with abnormally expanded polyQ or a fragment thereof containing the polyQ part and/or (2) a LC3B protein, a homologue thereof, or a fragment thereof;
   wherein step (A) and step (B) can be carried out independently in any order.
[7] The method of [6] above, wherein
   step (A) comprises: determining the affinity reaction equilibrium dissociation constant between the candidate compound(s) and (1) a protein with abnormally expanded polyQ or a fragment thereof containing the polyQ part; and selecting the compound(s) whose affinity reaction equilibrium dissociation constant is 100 µM or less, preferably 10 µM or less, particularly preferably 1 µM or less; and/or
   step (B) comprises: determining the affinity reaction equilibrium dissociation constant between the candidate compound(s) and (2) a LC3B protein, a homologue thereof, or a fragment thereof; and selecting the compound(s) whose affinity reaction equilibrium dissociation constant is 100 µM or less, preferably 10µM or less, particularly preferably 1µM or less.
[8] The method of [6] or [7] above, wherein the method for determining binding affinity is selected from the group consisting of proximity scintillation analysis, fluorescence resonance energy transfer, fluorescence polarization detection, fluorescent molecular sieves, micro thermophoresis, chemiluminescence, surface plasmons resonance, isothermal titration calorimetry, oblique-incidence reflectivity difference and any combination thereof; Preferably selected from the group consisting of fluorescence resonance energy transfer, fluorescence polarization detection, oblique-incidence reflectivity difference and any combination thereof; particularly preferably oblique-incidence reflectivity difference.
[9] The method of any one of [6]-[8] above, wherein
   the step of pre-screening the candidate compound(s) for affinity is performed by high-throughput screening using the oblique-incidence reflectivity difference; comprising:
   (a) immobilizing the candidate compound(s)candidate compound(s) to prepare a compound chip, and scanning the chip to obtain the image before incubation;
   (b) incubating the compound chip with the target protein, and scanning the chip to obtain the image after incubation;
      wherein the target protein is (1) a protein with abnormally expanded polyQ or a fragment thereof containing the polyQ part or (2) a LC3B protein, a homologue thereof, or a fragment thereof; and
   (c) analyzing the difference image (the image after incubation minus the image before incubation); and selecting the compound(s) that can bind to (1) a protein with abnormally expanded polyQ or a fragment thereof containing the polyQ part, and (2) a LC3B protein, a homologue thereof, or a fragment thereof.
[10] The method of any one of [1]-[9] above further comprises the following steps:
   (D): detecting the binding of the candidate compound(s) to a normal polyQ protein or a fragment thereof containing the polyQ part and selecting the compound(s) that do not bind to the normal polyQ protein or the fragment thereof containing the polyQ part.
[11] Use of the compound obtained by the method of any one of the above [1]-[10] or the pharmaceutical composition thereof in the preparation of a medicament for the treatment or prevention of a polyQ-related neurodegenerative disorder, or use in the preparation of diagnostic a reagent or a kit for detecting subjects considered to be suffering from or susceptible to a polyQ-related neurodegenerative disorder;
   Preferably, the polyQ-related neurodegenerative disorder is selected from the group consisting of spinocerebellar ataxia type 1, spinocerebellar ataxia type 2, spinocerebellar ataxia type 3, spinocerebellar ataxia type 7, and spinocerebellar ataxia type 12, spinocerebellar ataxia type 17, dentatorubral-pallidoluysian atrophy, Huntington's disease, and spinal-bulbar muscular atrophy, especially Huntington's disease and spinocerebellar ataxia type 3.
[12] The use of [11] above, wherein the detection includes the step of analyzing a target sample obtained from the subject, comprising
   i) labeling the compound with a detectable label such as a fluorescent group;
   ii) treating the target sample with the compound obtained in i);
   iii) detecting the position and/or intensity of the detectable signal in the target sample and obtain the position and/or content of the protein with abnormally expanded polyQ or the fragment thereof containing the polyQ part in the target sample.
[13] Use of the compound or a pharmaceutical composition thereof in the preparation of a medicament for the treatment or prevention of a polyQ-related neurodegenerative disorder, or use in the preparation of a diagnostic reagent or a kit for detecting subjects considered to be suffering from or susceptible to a polyQ-related neurodegenerative disorder, wherein the compound is selected from: 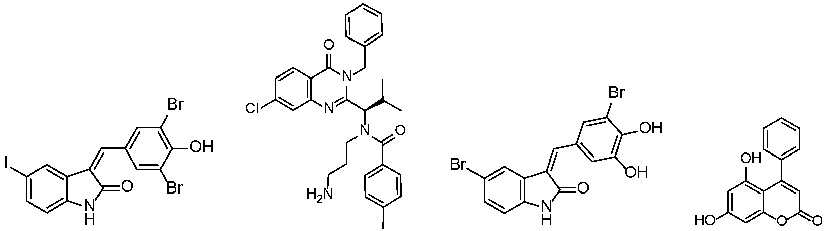 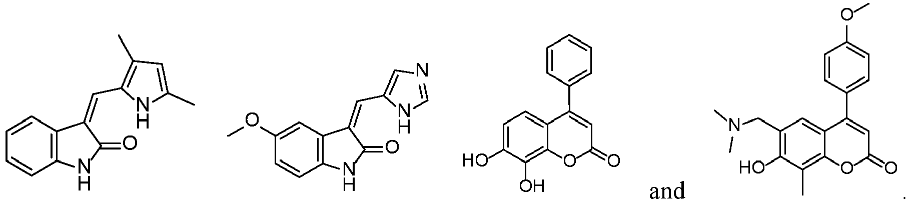
[14] A pharmaceutical composition, detection reagent or kit comprising a compound obtained by the method of any one of [1] to [10] above.
[15] A pharmaceutical composition, detection reagent, kit or screening system, which comprises at least one compound selected from the following: 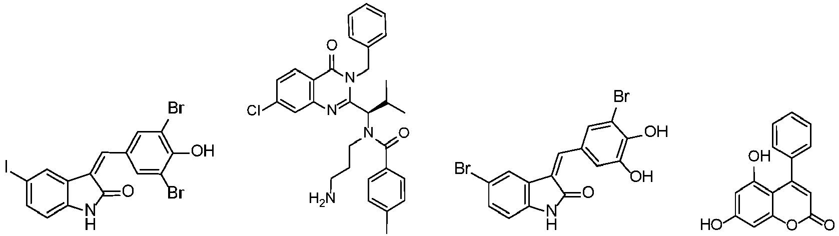 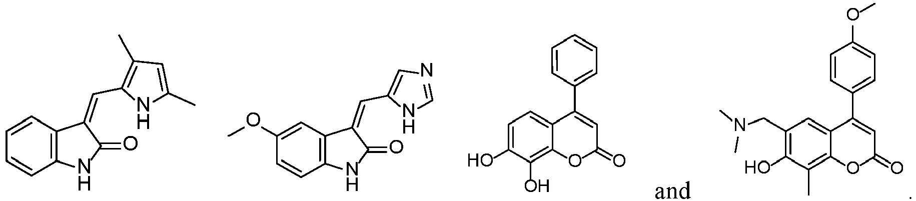
[16] A screening system comprising
   (1) a protein with abnormally expanded polyQ or a fragment thereof containing the polyQ part, and
   (2) a LC3B protein, a homologue thereof, or a fragment thereof;
      preferably, the screening system is in the form of a kit.
[17] Use of a compound or a pharmaceutical composition thereof that positively modulates the binding between (1) a protein with abnormally expanded polyQ or a fragment thereof containing the polyQ part, and (2) a LC3B protein, a homologue thereof, or a fragment thereof in the preparation of a medicament for the treatment or prevention of a polyQ-related neurodegenerative disorder.

### Beneficial Effects

The compound screened by the method of the present invention can selectively reduce the level of the protein with abnormally expanded polyQ in the cell by targeting autophagy, and has no influence on the autophagy function of cells. Therefore, the method of the present invention can be used to effectively screen compounds for treating or preventing polyQ-related neurodegenerative disorders.

Autophagy is a highly genetically conserved protein degradation pathway, which is ubiquitous in eukaryotic cells. It has strong protein degradation ability but shows low selectivity. The existing research mainly focuses on autophagy modulators, which leaves an unsolved problem about the global effect of non-specific regulation of autophagy, and therefore has potential side effects. The inventor unexpectedly discovered that the screening method of the present invention can obtain compounds that specifically and autophagy-dependently reduce the level of proteins with abnormally expanded polyQ, which have low side effects and good safety, and are easy to pass through the BBB, potentially facilitating oral administration. In addition, the screening method of the present invention has the advantages of easy implementation and high screening efficiency.

### EXAMPLES

Unless otherwise specified, the instruments and reagent materials used herein are all commercially available, or can be prepared by conventional methods known in the art.

Unless otherwise specified, in all statistical analyses in this article, ^{∗} represents p< 0.05; ^{∗∗} represents p< 0.01; ^{∗∗∗} represents p< 0.001, ^{∗∗∗∗} represents p< 0.0001. For the comparison between the two groups, the statistical analysis method used is the two-tailed unpaired t test. For comparisons between three or more groups, two-tailed one-way ANOVA was applied when there is only one variable, and two-tailed two-way ANOVA was applied when there are two variables.

**Abbreviation**

| | | | |
|---|---|---|---|
| ATXN | ataxin | HTT | Huntingtin |
| TBP | TATA binding protein | ATN | atrophin |
| *fl* | full length | | |
| BBB | blood-brain barrier | | |
| AR | androgen receptor | | |
| BDNF | brain derived neurotrophic factor | | |
| OI-RD | oblique-incidence reflectivity difference | | |
| MALDI-TOF -MS | matrix-assisted laser desorption/ ionization time of flight mass spectrometry | | |

### Experimental materials, reagents and method steps

### Compounds

The compound library used in the examples is provided by Selleck, containing 3375 biologically active compounds. These include 1,527 drugs approved by the U.S. Food and Drug Administration (FDA), 1,053 natural products from traditional Chinese medicine, and 795 known inhibitors. Among them:
Compound 1: GW5074, available from DC Chemicals, catalog number DC8810;
Compound 2: ispinesib, PubChem CID: 6851740, available from Selleck, catalog number S1452;
Compound 3: PubChemID 1759437, available from Specs, catalog number AN-655/15003575;
Compound 4: PubChem CID 5398649, available from ChemDiv, catalog number D715-2435;
Compound 5: Semaxanib, available from Selleck, CAS No. 194413-58-6;
Compound 6: Su9516, available from Selleck, CAS No. 377090-84-1;
Compound 7: available from TargetMol, CAS No. 842-01-3;
Compound 8: available from ChemDiv.

### Antibody

HTT antibody 2B7 (Weiss et al. Anal Biochem 2009, 395, 8-15), 4C9, ab1 (Sapp et al. J Biol Chem 2012, 287, 13487-13499) and MW1 (Ko et al. Brain research bulletin 2001, 56, 319-329) were prepared by the method of the prior art; the antibody S830 used for immunostaining to detect HTT aggregates was gifted from Dr. Gillian Bates; other antibodies were purchased from companies such as Millipore and Sigma.

### Preparation of recombinant human LC3B

(1) His8 tag and TEV protease cleavage site are added to pGEX-6P1 (from GE Healthcare) to prepare prokaryotic expression vector pGHT. LC3B gene (GenBank: NM_022818.4) is edited to remove Met and add two Glys at the N-terminal of LC3B. The gene is amplified and cloned into the prepared pGHT.
(2) The expression plasmid pGHT-LC3B was introduced into *E. coli* BL21(DE3)pLsyS for expression. Purification is conducted using HisTrap HP column (GE Healthcare, 17524701). Then the protein is mixed with TEV protease (Sigma, T4455) and dialyze overnight. Purification is conducted using HisTrap HP column, Superose 6 Increase 10/300 GL size exclusion column (GE Healthcare) in sequence.
(3) LC3B verification: The prepared human LC3B is verified by MALDI-TOF-MS and X-ray diffraction. The method of X-ray diffraction verification of LC3B is: the mutant LC3BΔG120 protein lacking the G120 lipidation site is obtained using site-directed mutagenesis, and this protein with improved stability is used for high-resolution X-ray diffraction. The known LC3 protein crystal structure (PDB ID: 1UGM) is used as a search model for molecular replacement. The published structure of LC3BΔG120 (PDB ID: 6J04, 1.90Å) was solved by molecular replacement and structural alignment was performed.

### Preparation of recombinant human HTTexon1-MBP protein

(1) 25Q or 72Q HTTexon1 cDNA is synthesized de novo, cloned into mammalian expression vector pTT5SH8Q2. A C-terminal MBP tag is added after the HTTexon1 sequence to generate the pTT-HTTexon1-Q72-MBP and pTT-HTTexon1-Q25-MBP plasmids.
(2) The plasmid is transfected into HEK293T cells for expression with linear polyethyleneimine (Polysciences, 24765). The protein is purified using HisTrap HP column and Superose 6 Increase 10/300 GL size exclusion column.
(3) The purified HTTexon1-MBP protein was dialyzed into 5mM NH₄Ac through the Superose 6 Increase size exclusion column, verified by Bruker FLEX MALDI-TOF.

### Preparation of recombinant human full-length HTT protein

(1) The human HTT gene (GenBank: NM_002111.8) with (CAG)₂₃ or (CAG)₇₃ was synthesized de novo by Genewiz Inc. The human HTT gene was cloned into the modified pCAG vector (from Addgene) with N-terminal protein A tag.
(2) The plasmid was transfected into human embryonic kidney E293 cells for expression using polyethyleneimine (PEI, from Polysciences, 23966). The protein is purified with IgG monoclonal antibody-agarose (Smart-lifesciences, SA030010), digested with TEV protease to remove the protein A tag, and further purified with Mono Q and Superose 6 (5/150 GL) columns from GE healthcare. Validation is conducted using Coomassie blue staining and Western-blots.

### Preparation of recombinant human MBP-ATXN3

(1) His8 tag and TEV protease cleavage site is added in pMal-C2x plasmid (from New England Biolabs) to prepare prokaryotic expression vector pMBP. The ATXN3 gene (GenBank: NM_001127696.2) is edited to control the length of the expanded polyglutamine, the gene is amplified and cloned into the prepared pMBP to obtain the pMBP-ATXN3-Q28 and pMBP-ATXN3-Q68 plasmids with polyQ repeated 28 and 68 times, respectively.
(2) The expression plasmids pMBP-ATXN3-Q28 and pMBP-ATXN3-Q68 were introduced into *Escherichia coli* Rosetta(DE3)pLsyS for expression. Preliminary purification was conducted with HisTrap HP column (GE Healthcare, 17524701). The product was concentrated by ultrafiltration and then further purified with Superose 6 Increase 10/300 GL size exclusion column (GE Healthcare).
(3) As verified by SDS-PAGE, the purity of the prepared human MBP-ATXN3-Q28 and MBP-ATXN3-Q68 exceeds 98%.

### Cells used for tests

Primary cultured cortical neurons: The brains of Hdh^{Q140/Q7} and Hdh^{Q7/Q7} newborn mice (P0) were dissected, digested, dissociated, and cultured.

Some primary patient fibroblasts and wild-type cells are from HD patients (Q47, Q55) of the Mongolian Huntington's disease family and healthy controls (WT, Q19). SCA3 cell line was from patient (Q74). The HD Q68 fibroblast cell line was from Coriell Cell Repositories (Camden, NJ, USA). Immortalized fibroblasts and induced stem cells (iPSCs) are prepared from primary fibroblasts. Mouse striatum cells (STHdh) were from Coriell Cell Repositories (Camden, NJ, USA). HEK293T cells and HeLa cells were from ATCC.

### Animals used for tests

### Huntington's disease Drosophila

Nervous system driver line *elav-GAL4 (c155)*, HTT expressing lines *UAS-fl-HTT-Q16* and *UAS-fl-HTT-Q128* were from Bloomington Drosophila Stock Center (http://flystocks.bio.indiana.edu/) and kept in a 25°C incubator.

Crosses were set up between virgin female Drosophilae carrying *elav-GAL4* driver and the *UAS-fl-HTT-Q16* or *UAS-fl-HTT-Q128* male Drosophilae to generate a transgenic Drosophilae driven by *elav-GAL4* to express human HTT full-length protein (Q16) or (Q128) in the nervous system.

### Huntington's disease mice

Mice expressing wild-type HTT gene (Hdh^{Q7/Q7}) were from the Marian Difiglia laboratory of Massachusetts General Hospital, Harvard University. The Q140 gene knock-in heterozygous mouse (Hdh^{Q140/Q7}) was prepared according to the method of the prior art (Menalled et al., J Comp Neurol, 2003, 465: 11-26).

### Protein analysis

Homogeneous time-resolved fluorescence (HTRF) analysis: the cell or tissue lysate was diluted with the original lysis buffer PBS + 1%(v/v) Triton X-100 + 1×cOmplete^{™} protease inhibitor to lyse the sample, then HTRF assay buffer (50 mM NaH₂PO₄, 400 mM NaF, 0.1% BSA, 0.05% (v/v) Tween-20, 1% (v/v) Triton X-100, pH 7.4) was used to dilute the specified antibody pair for detection. In HTRF buffer, the donor antibody concentration was 0.023 ng/µL and the acceptor antibody concentration was 1.4 ng/µL.

Determination of the amount of protein: the amount of protein was determined by the above method. Background correction was performed using blank sample. The protein concentration for all samples were determined to correct the loadings. Different protein concentrations or cell numbers per well were measured to ensure that the signals are within the linear range.

### Cell analysis

Immunofluorescence: cells were washed, fixed, permeabilized and blocked, then incubated with primary antibody overnight at 4°C, then washed three times with blocking buffer, and incubated with secondary antibody for 1 hour at room temperature, stained with DAPI, and mounted. Images were taken by Zeiss Axio Vert A1 confocal microscope. TUBB3 or co-localization was analyzed using ImageJ.

### Example 1 High-throughput screening of compounds for protein affinity

### 1.1 Affinity screening

(1) Compound chips were prepared using a contact microarray printer (SmartArrayer 136, CapitalBio Corporation) according to the prior art (Zhu et al., Sensors (Basel) 2016, 16(3), 378; Fei et al., J Biomed Opt 2010, 15(1), 016018). Each compound was printed in duplicate on the microarray. The compound not fixed on the surface of the chip was washed away. The chip was sealed and cleaned. OI-RD microscope was applied to scan the images. HTTexon1-Q72-NMP (SEQ ID NO: 1 with MBP tag added, wherein MBP tag was used to increase solubility) was used as the target protein. The compound chip was incubated with the target protein (at a concentration of 238nM) for 2 hours. The chips are cleaned and scanned.
(2) HTTexon1-Q25-NMP (SEQ ID NO: 5 with MBP tag added) was used as the target protein (concentration 454 nM). OI-RD images before and after incubation with the target protein was obtained following the method in (1).
(3) LC3B (SEQ ID NO: 8) was used as the target protein (concentration 680 nM). OI-RD images before and after incubation with the target protein was obtained following the method in (1).

The difference images of the above steps (1), (2), (3) (the image after incubation minus the image before incubation) was analyzed respectively. Compounds that bind to HTTexon1-Q72-MBP and LC3B but not to HTTexon1-Q25-MBP (the detection limit of OI-RD microscope was about 0.001mV) were selected. The following compounds which can bind to LC3B and HTTexon1 with abnormally expanded polyQ, but do not bind to HTTexon1 with normal polyQ were obtained: Compound 1 (GW5074) and compound 2 (ispinesib).

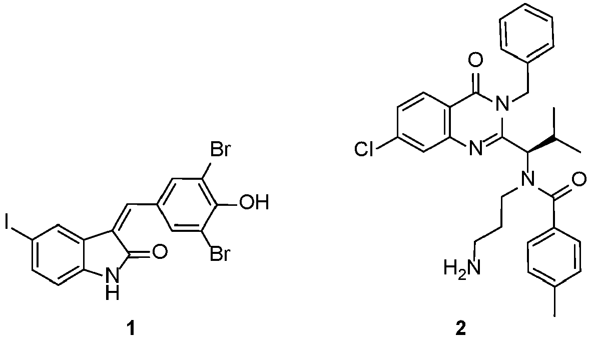

Chips with compounds 1 and 2 are prepared according to the above method. Each compound was printed in triplicates on the microarray. OI-RD was used to measure the affinity reaction parameters of compounds with HTTexon1-Q72 and HTTexon1-Q25.

The compounds showed no binding affinity with HTTexon1-Q25. The affinity reaction parameters with HTTexon1-Q72 are shown in Table 1.

**Table 1. The association rate constants, dissociation rate constants, and equilibrium dissociation constants of the affinity reactions between the compounds with HTTexon1-Q72**

| | **Kₒₙ** (min•nM)⁻¹ | **K_{off}**(min)⁻¹ | **K_{d}** (nM) |
|---|---|---|---|
| compound 1 | 9.07 × 10⁻⁵ | 1.43 × 10⁻² | 157.6 |
| compound 2 | 2.13 × 10⁻⁴ | 6.23×10⁻³ | 29.2 |

Not more than 20 compounds were selected from commercially available known compounds which had the following: bicyclic structures and side chains containing benzene rings like in compounds 1 and 2, and reactive functional groups (such as OH, SH, NH, etc.) that are required for preparing small molecule chips. Screening was conducted according to the above-described method, and two compounds that can bind to HTTexon1-Q72 and LC3B but not to HTTexon1-Q25 were obtained: compound 3 and compound 4.

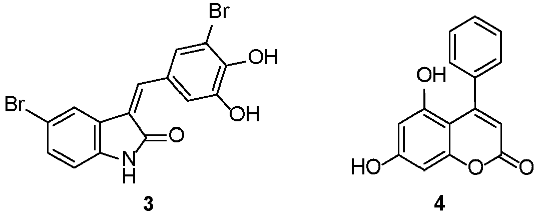

Chips with compounds 1, 2, 4 were prepared according to the above method. Each compound was printed in triplicates on the microarray. OI-RD was used to measure the affinity reaction parameters of the compound with LC3B, full-length mHTT (flHTT-Q73) and full-length HTT-Q23 (flHTT-Q23).

The compound showed no binding affinity with flHTT-Q23 (SEQ ID NO: 4). The affinity reaction parameters with flHTT-Q73 and LC3B are shown in Table 2 and Table 3, respectively.

**Table 2. The association rate constants, dissociation rate constants, and equilibrium dissociation constants of the affinity reactions between the compounds and flHTT-Q73**

| | **Kₒₙ** (min•nM)⁻¹ | **K_{off}** (min)⁻¹ | **K_{d}** (nM) |
|---|---|---|---|
| compound 1 | 5.43 × 10⁻⁵ | 7.97×10⁻³ | 14.7 |
| compound 2 | 6.43 × 10⁻⁵ | 2.07×10⁻² | 321.9 |
| compound 4 | 4.81 × 10⁻⁴ | 1.19×10⁻² | 24.7 |

**Table 3. The association rate constants, dissociation rate constants, and equilibrium dissociation constants of the affinity reactions between the compounds and LC3B**

| | **Kₒₙ** (min•nM)⁻¹ | **K_{off}**(min)⁻¹ | **K_{d}** (nM) |
|---|---|---|---|
| compound 1 | 3.72×10⁻⁵ | 1.74×10⁻² | 467.7 |
| compound 2 | 2.46×10⁻⁵ | 5.86×10⁻³ | 238.2 |
| compound 4 | 4.61×10⁻⁵ | 2.49×10⁻² | 540.1 |

### 1.2 MST detection of the affinity of the compound with full-length HTT and LC3B

The affinity of the compound with full-length HTT and LC3B was verified with micro thermophoresis (MST) using Monolith NT. 115 instrument from NanoTemper Technologies. The reaction buffer was 20 mM HEPES, pH 7.4, 150 mM NaCl. Protein concentration was 500 nM. The compound showed no binding affinity with flHTT-Q23 (SEQ ID NO: 4), and the K_{d} of flHTT-Q73 and LC3B are shown in Table 4, respectively.

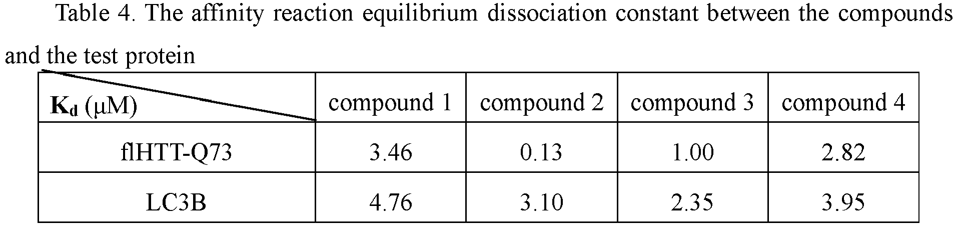

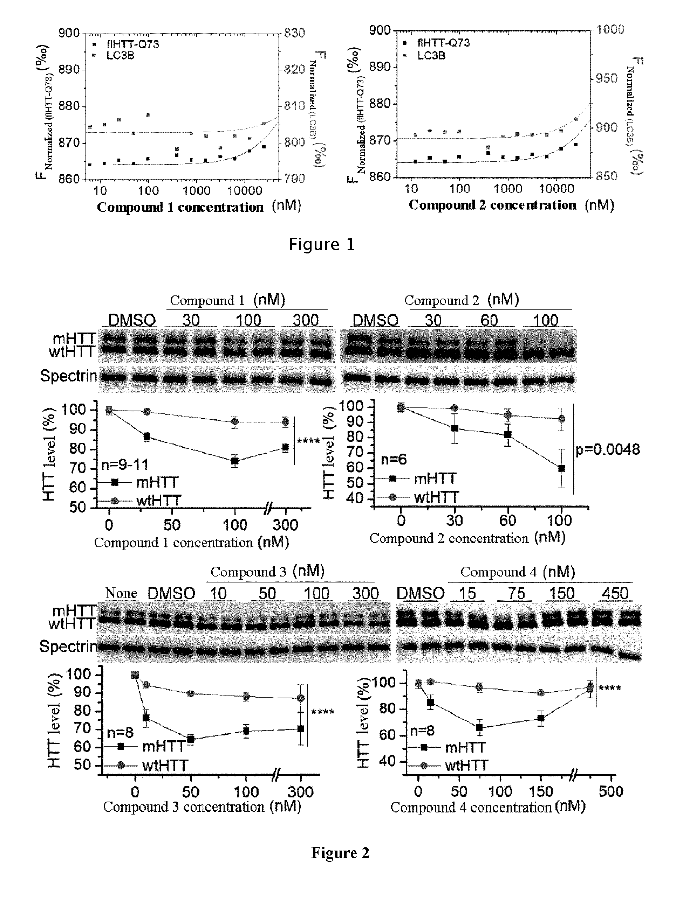

The inventors also verified through MST that the compound had respective affinity with both full-length HTT and LC3B in a system where both full-length HTT and LC3B existed (Figure 1).

In summary, the results of OI-RD measurement and MST assay both showed that the compound had binding affinity to LC3B; for HTT, the compound selectively binded mHTT (HTTexon1-Q72 or flHTT-Q73). This indicated that the compound can positively regulate the binding effect between LC3B and mHTT.

### Example 2 Effects of compounds on cortical neurons in HD model mice

### 2.1 Effects on mHTT and wtHTT levels

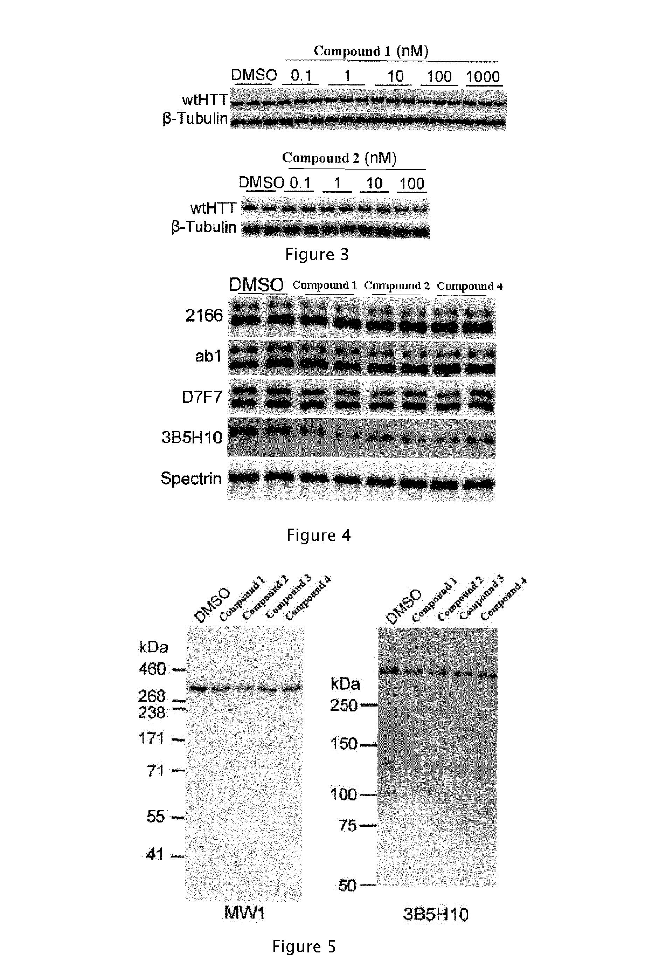

(1) The primary cultured Hdh^{Q140/Q7} and Hdh^{Q7/Q7} mouse cortical neuron cells were treated with the compound for 2 days. Then the levels of mHTT and wtHTT were detected by Western-blots (antibody 2166) (Figure 2 and Figure 3). The compound reduced the level of mHTT in cortical neurons of Q140 knock-in heterozygous mice (Hdh^{Q140/Q7}), but hardly affected the level of wtHTT. Compounds 1 and 2 did not reduce wtHTT levels in cortical neurons of Hdh^{Q7/Q7} mice. When compound 1 was in the range of 30-300 nM (100 nM was preferred), compound 2 was in the range of 30-100 nM (100 nM was preferred), compound 3 was in the range of 10-300 nM (50 nM was preferred), compound 4 was in the range of 15-150 nM (75 nM was preferred), the effect of selectively reducing mHTT levels was observed.
(2) Using a test method similar to (1), the levels of mHTT in Hdh^{Q140/Q7} mouse cortical neurons after compound treatment were detected using a variety of antibodies (2166, ab 1, D7F7, 3B5H10) (Figure 4). The variety of antibody tests gave relatively consistent results, so the detected decrease in mHTT level was not due to changes in the affinity of mHTT for specific antibodies.

mHTT was detected using anti-polyQ antibodies MW1 and 3B5H10, and observed for bands of proteins with smaller molecular weights. As a result, no increase in the N-terminal fragments of mHTT was observed (Figure 5). The detected decrease in mHTT level was not due to an increase in site-specific cleavage.

### 2.2 Cytotoxicity test

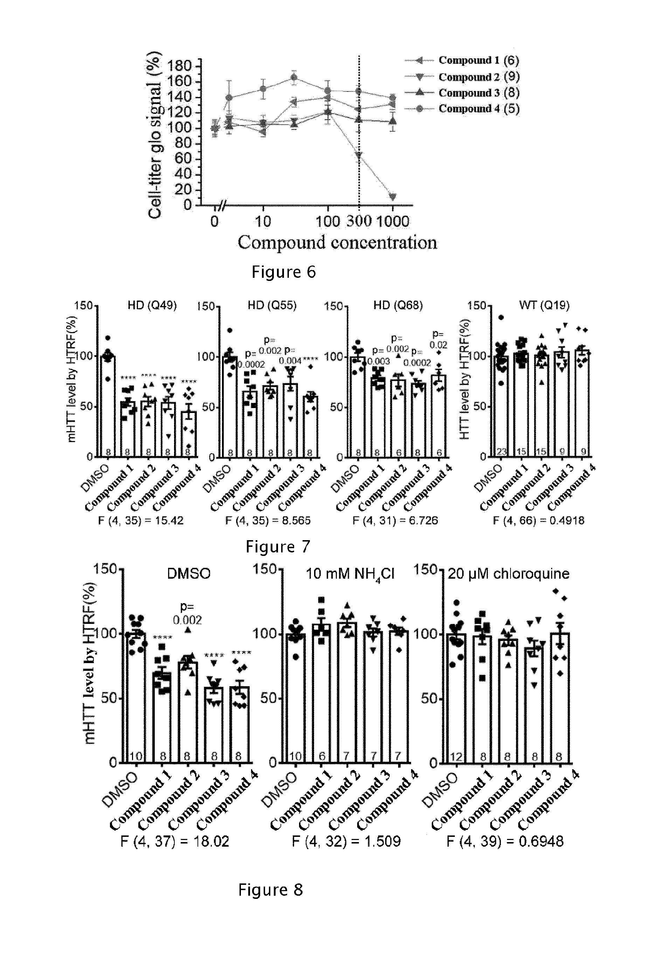

CellTiter-glo (Promega, G7570) was used according to the protocol provided in the kit to determine the viability of Hdh^{Q140/Q7} mouse cortical neurons after compound treatment (Figure 6). The compound showed no cytotoxicity to Hdh^{Q140/Q7} mouse cortical neurons at the test concentration described in 2.1. The detected decrease in mHTT level was not due to neuronal cell loss.

In summary, the compounds obtained by the screening method of the present invention all exhibited excellent allele-selective effects in reducing mHTT levels in cells. Among them, two compounds that was verified to be active at the cellular level were obtained from a structurally diverse compound library containing 3375 biologically active compounds, demonstrating a positive rate of 0.6‰.

### Example 3 Effects of compounds on HTT level in fibroblasts of Huntington's disease patients

### 3.1 Effect on the levels of mHTT and wtHTT in primary fibroblasts of HD patients

Preliminary experiments were conducted. As a result, the compound exhibited the best mHTT reduction effect at a concentration of 100 nM.

Experiment method: The fibroblasts (Q49, Q55, Q68) of HD patients were treated with 100 nM compound for 2 days. Then mHTT (antibody pair: 2B7/MW1) and total HTT (antibody pair: 2B7/2166) were detected by HTRF. The results are shown in Figure 7. Reduced mHTT levels were observed in primary fibroblasts (Q49, Q55, Q68) of HD patients. No reduction in HTT levels was observed in wild-type primary fibroblasts.

### 3.2 Effects on the level of mHTT in HD patient immortalized fibroblasts

A test method similar to that described in 3.1, HTRF (antibody pair: 2B7/MW1) was used to detect the effects of the compound on the level of mHTT in immortalized fibroblasts of HD patients in the presence or absence of autophagy inhibitors (Figure 8). In the absence of autophagy inhibitors, the compound significantly reduced the mHTT level of HD patient immortalized fibroblasts which is consistent with the results observed on HD patient primary fibroblasts On the contrary, in the presence of autophagy inhibitors (NH₄Cl or chloroquine), no decrease in mHTT levels was observed.

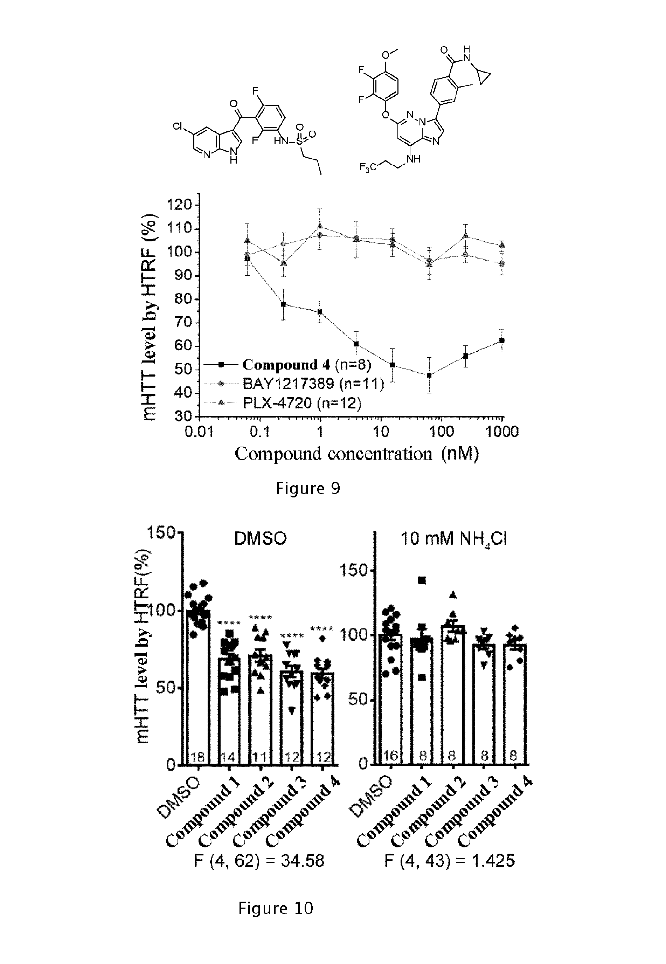

It was reported that compound 1 also had c-Raf inhibitor activity, and compound 2 also had kinesin spindle kinase (KSP) inhibitory activity. For this reason, the inventors tested some c-Raf inhibitors and KSP inhibitors (including GSK923295, BAY1217389, PLX-4720, Dabrafenib, Sorafenib Tosylate). The immortalized fibroblasts of HD patients were treated using the above-mentioned c-Raf inhibitors and KSP inhibitors respectively at a concentration of 100 nM. No decrease in mHTT level was observed. Using PLX-4720 (c-Raf inhibitor), BAY1217389 (KSP inhibitor) and compound 4 for comparison, PLX-4720 and BAY1217389 did not reduce mHTT levels at concentrations below the micromolar level (Figure 9). Therefore, the effect of the compound on mHTT was not caused by the inhibitory activity on c-Raf or KSP.

In summary, the reduction of mHTT levels by the compounds obtained from screening were autophagy-dependent.

### Example 4 Effects of the compounds on mHTT level and neuronal apoptosis of HD patient iPSC-derived neurons

### 4.1 Effect on mHTT and wtHTT levels

A test method similar to that described in 3.1, HTRF (antibody pair: 2B7/MW1) was used to detect the effects of compound 1, compound 2, compound 3, and compound 4 on the mHTT level of HD patient iPSC derived neurons (Q47) (Figure 10). Decreased mHTT levels were observed in HD patient iPSC-derived neurons, and this effect was not observed in the presence of the autophagy inhibitor NH₄Cl.

Using the same method as above, the following compound 5, compound 6, compound 7, and compound 8 were obtained from screening. These compounds were observed to reduce the mHTT level in the HD patient iPSC-derived neurons; wherein compound 5 reduced mHTT levels by approximately 12.2%, compound 6 reduced mHTT levels by approximately 24.9%, compound 7 reduced mHTT levels by approximately 24.2%, and compound 8 reduced mHTT levels by approximately 19.7%. The compound rescued disease-related phenotypes in HD patient iPSC-derived neurons through autophagy.

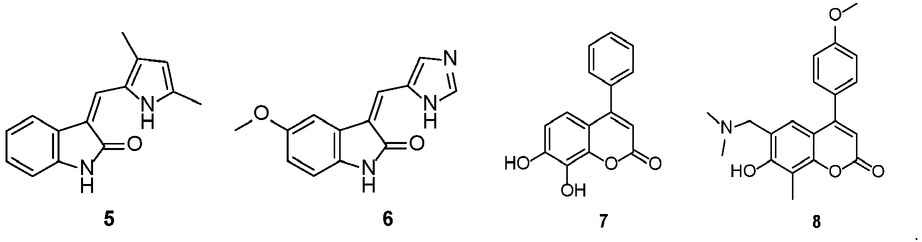

### 4.2 Effects on neuronal apoptosis

### (1) Immunostaining

HD patient iPSC-derived neurons (Q47) were treated using 100nM compound 1, or 100nM compound 2, or 100nM compound 3 or 50nM compound 4 for 1 day. Then the cells were stressed (by BDNF removal).

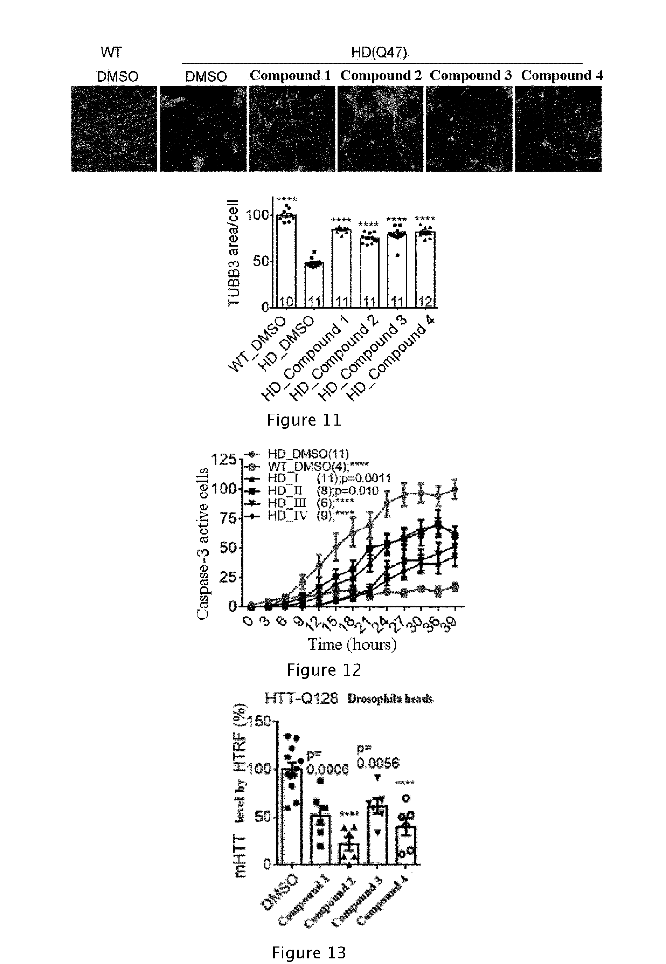

Neuronal specific tubulin marker TUBB3 was stained with DAPI. The TUBB3 signal covered area was normalized to the cell nuclei counts, and the data was normalized to the wild type controls to analyze neuronal apoptosis (Figure 11).

### (2) Caspase-3 activity detection

After removing BDNF, loss of processes and shrinkage of neurons were observed in HD neurons.

NucView 488 (Biotium, 30029) was used to detect active caspase-3. After removing BDNF, Incucyte (Essen Bioscience, IncuCyte FLR) was used to capture images every 3 hours in the incubator, which were analyze with Incucyte 2011A software. Three batches were tested and the results were consistent (Figure 12). The compound significantly improved the loss of processes and shrinkage of HD neurons after removal of BDNF.

### Example 5 Effects of compounds on Huntington's disease Drosophilae

### 5.1 Effects on mHTT level

Experimental method: Q128 Drosophilae and Q16 Drosophilae were randomized into a negative control group and a positive drug group (compound 1, compound 2, compound 3, compound 4), with 75 drosophilae in each group. The negative control group was given the corresponding solvent DMSO, and the positive drug groups was given the corresponding positive drug.

Drosophilae were kept in standard food at 25°C. The newly hatched Drosophilae were transferred to vials with food containing the positive drug (10 µM in 400 DMSO) or DMSO for control. The food was changed every other day.

After fed for 6 consecutive days, the Drosophila head protein was extracted on the 7th day. The mHTT level was measured by HTRF (antibody pair: 2B7/MW1), where each sample included head proteins from five Drosophila (Figure 13). The compound reduced mHTT levels in transgenic Drosophilae expressing human HTT full-length protein (Q128).

### 5.2 Effects of compound on survival rate

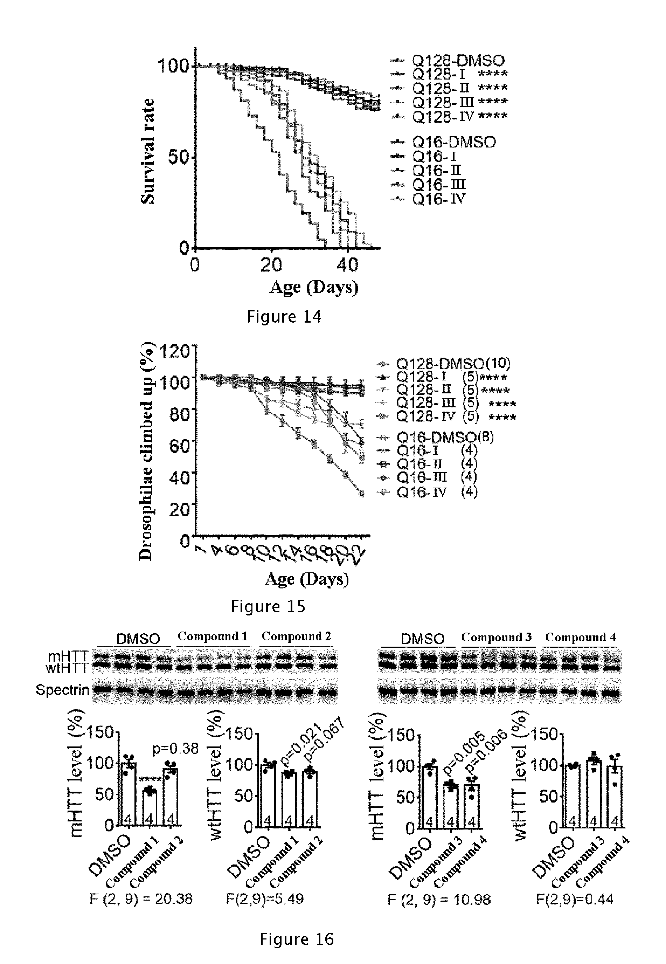

75 age-matched virgin Drosophilae were put into empty plastic vials containing standard food, and the survival rate of each vial was recorded daily to measure the lifespan (Figure 14). The survival rate of the Q128 Drosophila positive drug group was improved compared to the control group.

### 5.3 Effects on climbing performance

15 age-matched virgin Drosophilae was put into empty vials and tapped down so that they were at the bottom of the vials. The percentage of Drosophilae that had climbed past a 7-cm-high line after 15 seconds was record. The mean of five observations for each vial was plotted every day, and the data from multiple vials containing different batches of Drosophilae was plotted and analyzed (Figure 15). The number in brackets indicates the number of tested vials. The Q128 Drosophila positive drug group showed improved the climbing performance compared to the control group.

In the above experiments in 5.2 and 5.3, no compound was observed to have an effect on Q16 Drosophilae.

### Example 6 Effects of compounds on HD model mice

The mice were grouped and housed in individually vented cages with a 12-hour light/dark cycle, with a maximum of 5 adult mice per cage.

### 6.1 Effects of intracerebroventricular injection of compounds on the levels of mHTT and wtHTT in the cortices of HD mice

Experimental animals: Hdh^{Q140/Q7} mice (3 months old), 4 in each group.

Experimental method: one icv-injection was conducted per day using 2 µL artificial cerebrospinal fluid (ACSF: 1 mM glucose, 119 mM NaCl, 2.5 mM KCl, 1.3 mM MgSO₄, 2.5 mM CaCl₂, 26.2 mM, NaHCO₃, 1 mM NaH₂PO₄) containing a compound at a concentration of 25 µM. 2 µL ACSF containing the same amount of DMSO was used as a control.

After 10 days of injection, the brain cortical neuron proteins of the mice were extracted, and the levels of mHTT and wtHTT were detected by Western-blots (antibody 2166). Each test includes three repetitions for each sample, and the mean values was calculated (Figure 16). Compounds 1, 3 and 4 significantly reduced mHTT level in Hdh^{Q140/Q7} mouse cortices and showed mHTT selectivity relative to wtHTT.

### 6.2 Compound 1 or 4 administered by intraperitoneal injection

Experimental method: The compound or control DMSO was diluted with 0.9% NaCl intravenous infusion solution to 0.05 µg/µL. One ip-injection (0.5 mg/kg) was conducted per day. After 14 days of injection, tissue extraction or behavioral experiment were conducted.

In vivo compound detection in brain tissues of ip-injected mice: 2 hours after ip-injection of DMSO or compound (compound 1 or compound 4) in mice, the brain was dissected, weighed, and the compound in the brain tissue was extracted, analyzed with UPLC-MS (Acquity Ultra Performance Liquid Chromatography System, Acquity UPLC BEH C18 (1.7 µm, 2.1×50 mm) column and Xevo TQ-S mass spectrometer, Waters Corporation, Milford, MA, USA) for LC-MS/MS analysis. The results showed that the level of compound 1 reached the brain tissue was 81.0 ng/g. The level of compound 4 reached the brain tissue was 9.48 ng/g. No mass spectrum signal of compound was observed for the control (DMSO) group. The compound can be delivered to the brain of mice through the BBB smoothly, which potentially facilitates oral administration of the compound.

### 6.3 Effects of intraperitoneal injection of compounds on the levels of mHTT and wtHTT in the cortices and striata of HD mice

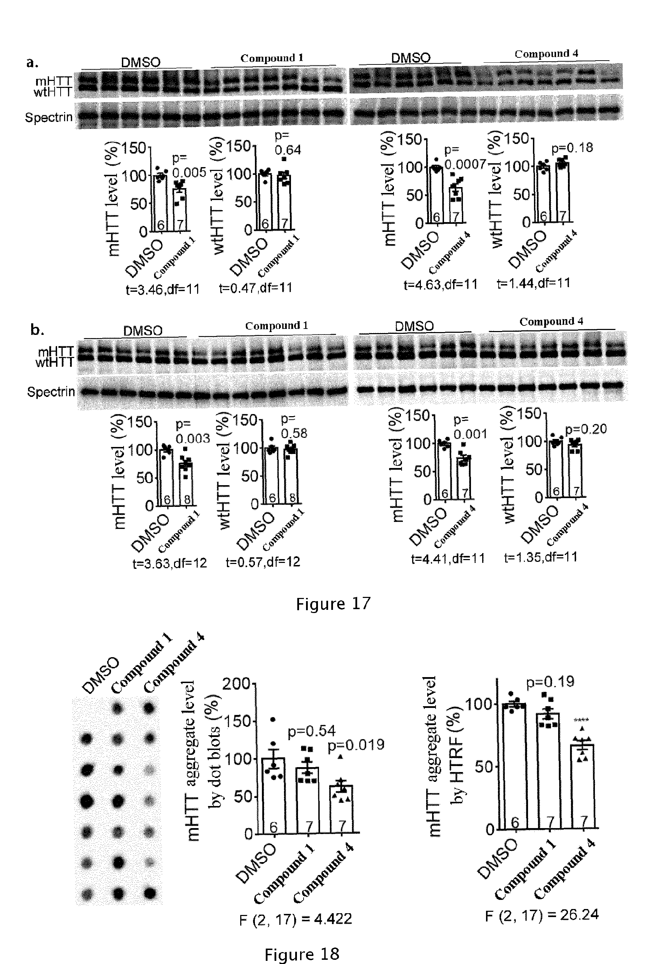

(1) 20 Hdh^{Q140/Q7} mice (5 months old) were randomized into 3 groups.
   Administer compound 1 or 4 as described in 6.2. One ip-injection was conducted per day. After 14 days of injection, the protein was extracted and the levels of mHTT and wtHTT in the cortices of HD mice were detected by Western-blots (Figure 17a).
(2) A total of 21 mice Hdh^{Q140/Q7} mice (10 months old) were used, 6-8 mice in each group.
   According to the above method, the mouse brain striatum neuronal protein was obtained and the levels of mHTT and wtHTT were detected by Western-blots (Figure 17b).
(3) mHTT aggregates in the cortices of Hdh^{Q140/Q7} mice were detected by dot-blot experiment (antibody: 4C9, bar plot showed the results of two repetitions) and HTRF (antibody pair: 4C9/4C9). Sampling was repeated for each mouse two to three times on average (Figure 18). No increase in mHTT aggregates was observed. The decrease in mHTT levels in the compound-treated group was not due to changes in mHTT solubility.

In summary, intraperitoneal injection of the compound reduced the levels of mHTT in the cortices and striata of Hdh^{Q140/Q7} mice, and showed mHTT selectivity relative to wtHTT. Therefore, the compound had the prospect of being developed to an oral drug.

### 6.4 Effects of intraperitoneal injection of compounds on behavioral defects in HD mice

Experimental animals: 42 Hdh^{Q140/Q7} mice were randomized into 3 groups; 48 Hdh^{Q7/Q7} mice were randomized into 3 groups.

Experimental method: administer compound 1 or 4 according to the procedure described in 6.2. One ip-injection was conducted per day, and behavioral experiments were conducted after 14 days of injection.

All behavioral experiments were carried out during the light phase. Before starting the experiments, all mice were kept in the behavioral test room under dim red light for one hour.

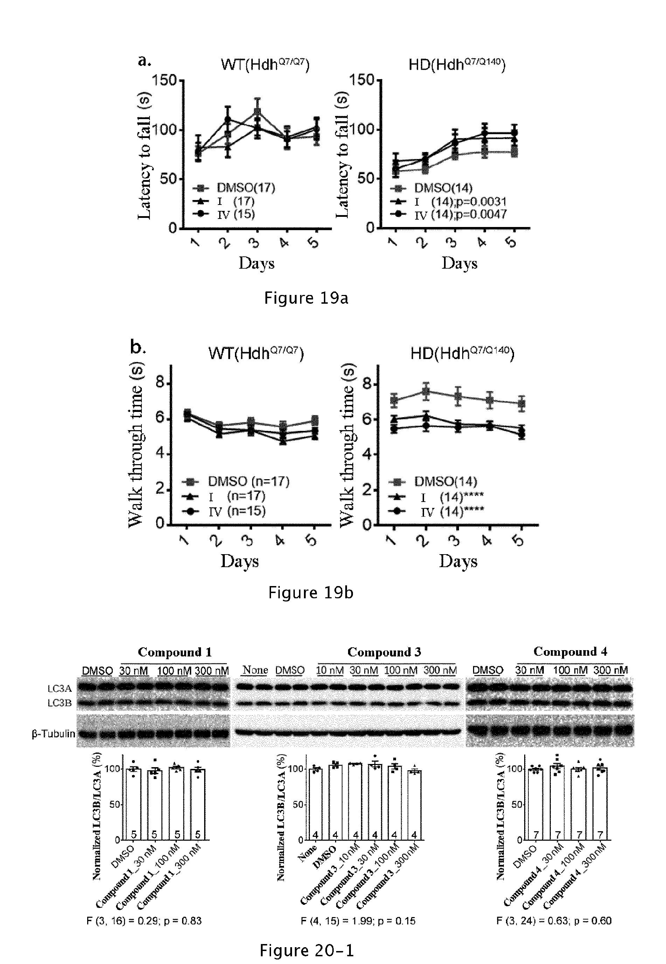

Rotarod test: The mice were pre-trained for 3 consecutive days (on the rotarod rotating at 4 rpm for 2 minutes). The result of each experiment was recorded as time on the rod (time on the rotating rod), until falling from the rod or until the end of the task. Each test include three repetitions with an inter-trial interval of 60 min in order to reduce stress and fatigue. The means of three trials were analyzed for each mouse (Figure 19a).

Balance beam test: a 2 cm thick meter stick with a total length of 100 cm was suspended on the platform on both sides. There was a bright light at the starting point and a dark box with food at the endpoint. The total time for each mouse to walk through the balance beam was recorded (Figure 19b).

In the rotating rod test, F (2, 195) = 4.963; in the balanced beam test, F (2, 195) = 37.31. The compound of the present invention improved the Huntington's disease related behavioral defects in HD model mice, and had no effect on wild-type mice.

### Example 7 Compounds do not affect autophagy function and the level of control proteins 7.1 Effects of compounds on cultured Hdh^{Q140/Q7} mouse cortical neurons

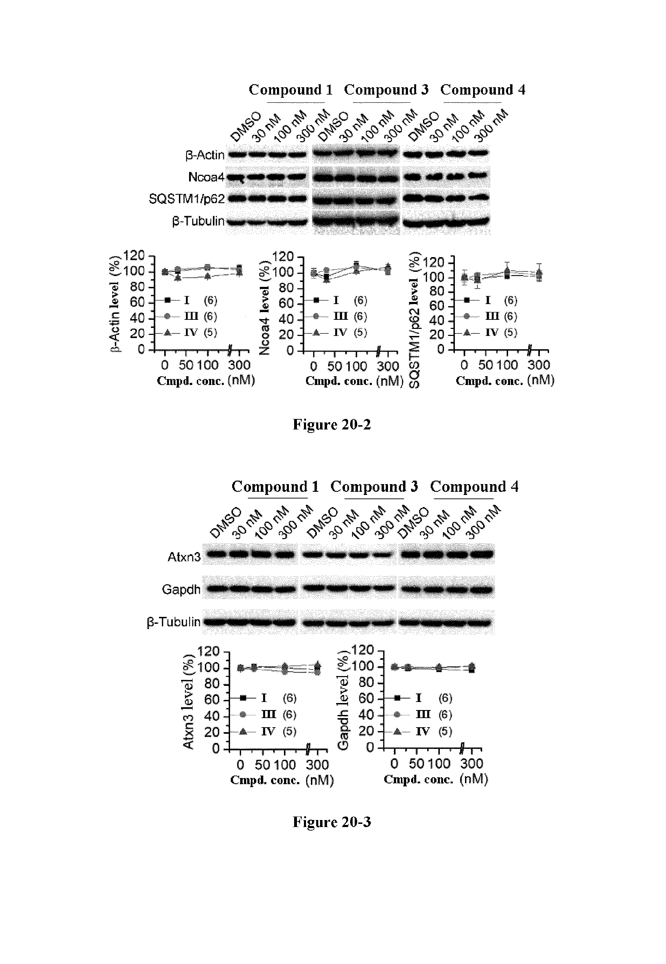

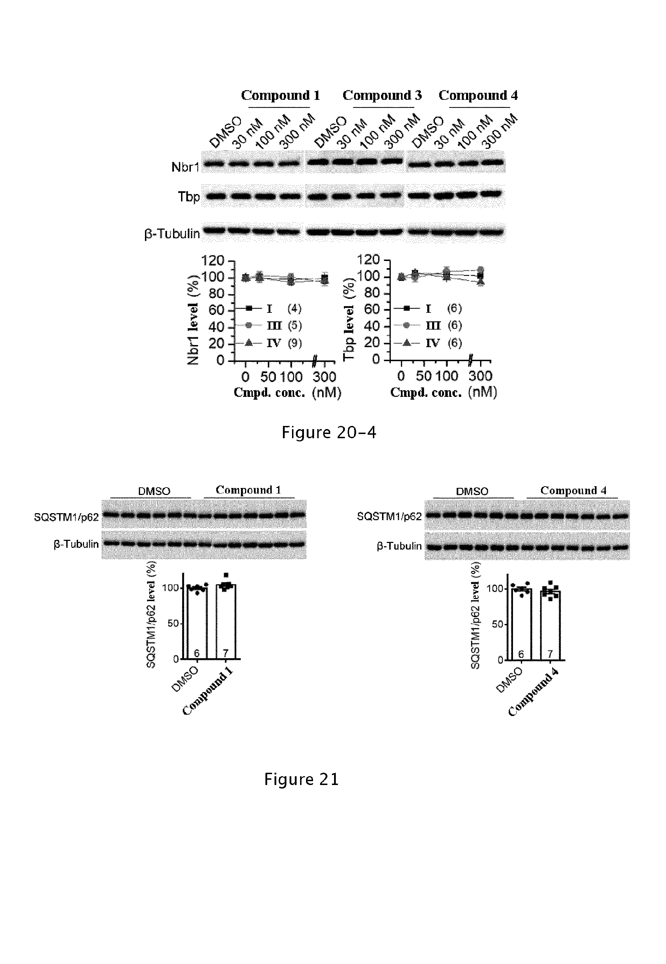

Following the method similar to 2.1, the level of LC3B, other known autophagy selective substrate proteins (including SQSTM1/p62, NBR1 and Ncoa4), other polyQ proteins with wild-type length polyQ (Atxn3 and Tbp) and several control housekeeping proteins in the cortical neurons of Hdh^{Q140/Q7} mice after compound treatment were detected. The results of Western blotting are shown in Figure 20-1, Figure 20-2, Figure 20-3, Figure 20-4. The compound did not affect the expression level of LC3B in the primary cultured cortical neurons, and the levels of other proteins tested were not affected by the compound (protein level change < 10%).

### 7.2 Test the effects of intraperitoneal injection of compounds

Using a method similar to 6.2, Hdh^{Q140/Q7} mice were administered with injections. A method similar to 7.1 was used to detect the effects of ip-injection of compounds on other known autophagy selective substrate proteins (SQSTM1/p62) in the cortical tissue of Hdh^{Q140/Q7} mice. The result of Western blotting is shown in Figure 21. After ip-injection of compound 1 or compound 4, the level of SQSTM1/p62 in the cortices of HD mice did not change.

In summary, the detected reduction in mHTT was not due to changed autophagy functions.

### Example 8 Effects of compounds on the binding between mHTT and LC3B

### 8.1 Effects of compounds on the binding between HTTexon1 and LC3B Protein binding experiment in vitro (in vitro pull-down assay)

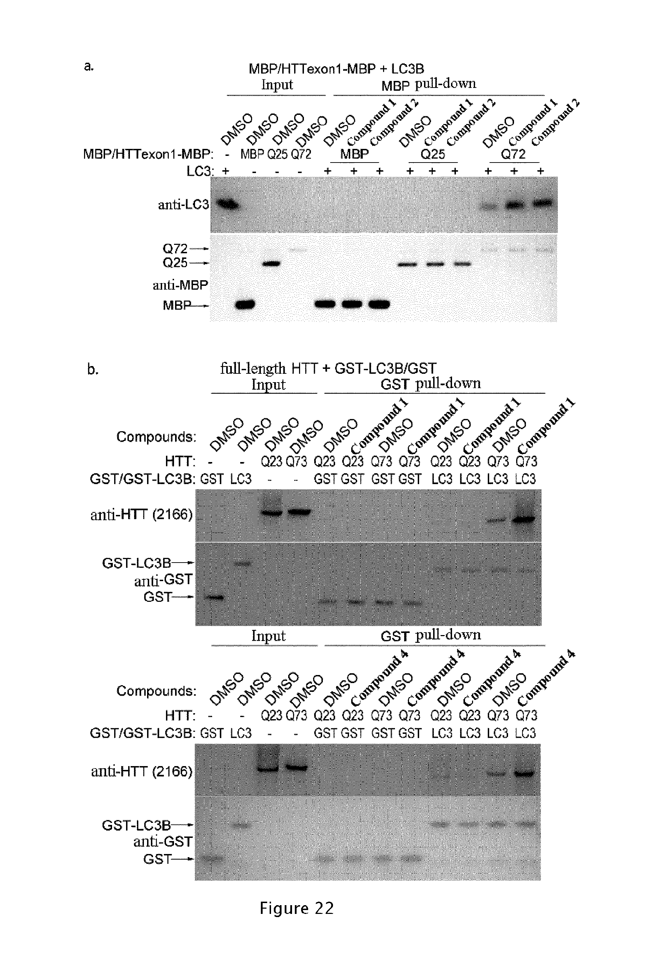

(1) The purified recombinant human HTTexon1-MBP (SEQ ID NO: 1 or SEQ ID NO: 5 with MBP tag added) and MBP used for control were respectively immobilized on amylose resin (New England BioLabs, E8021L) through affinity.
(2) The resin obtained in (1) containing about 10µg MBP-fusion protein or MBP was incubated with the compound (1 µM compound 1 or 100 nM compound 4) or the corresponding solvent DMSO, respectively. Then 40 µg of purified LC3B (corresponding to SEQ ID NO: 8) was added. Incubation was conducted. Then the unbound proteins were removed.
(3) The resin-bound proteins were eluted and analyzed by SDS-PAGE and Western-blots with anti-LC3 antibody (Figure 22a). Compound 1 and Compound 4 could enhance the binding between HTTexon1-Q72-NMP and LC3B, but not between HTTexon1-Q25-NMP and LC3B.

### 8.2 Effects of compounds on the binding between flHTT-Q73 and LC3B

GST-LC3B and GST for control were immobilized on mouse anti-GST antibody coupled magnetic beads (Cell Signaling Technology, 11847S). Pulled-down experiment was conducted using a method similar to 8.1. The GST-LC3B or GST were pulled down using full-length HTT (flHTT-Q73 or flHTT-Q23), respectively (Figure 22b). The pull-down loading ratio was: 100% for GST-LC3B or GST, 10% for full-length HTT. Elution was conducted with SDS-PAGE protein loading buffer. The compound enhanced the binding between full-length mHTT and LC3B.

### Example 9 Effects of compounds on the localization of mHTT and LC3B in cells

### 9.1 Co-localization between HTTexon1 and LC3B in Hela cells under the influence of compounds

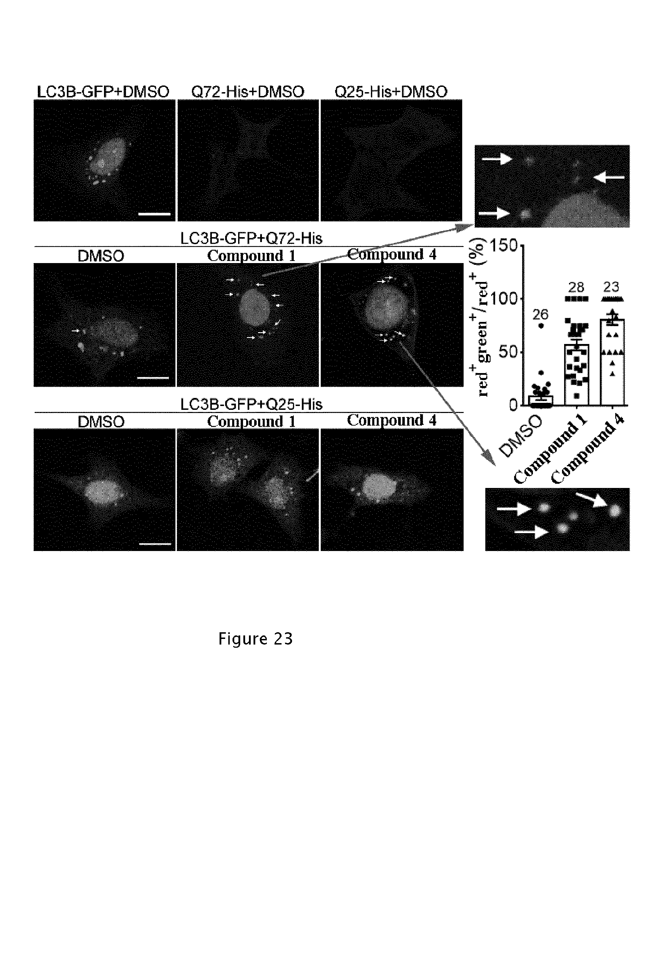

(1) The HTTexon1 cDNA was subcloned into the mammalian expression vector pTT-MBP-His to prepare pTT-HTTexon1-Q72-MBP-His and pTT-HTTexon1-Q25-MBP-His.
(2) The pTT-HTTexon1-Q72-MBP-His and pTT-HTTexon1-Q25-MBP-His prepared in (1) and pEX-GFP-hLC3WT (Addgene, 24987) were transiently transfected into HeLa cells to express HTTexon1-MBP-His (SEQ ID NO: 1 with MBP-His tag added and SEQ ID NO: 5 with MBP-His tag added) and LC3B-GFP (based on SEQ ID NO: 8).
(3) The cells prepared in (1) or (2) were treated with 100 nM compound 1 or 50 nM compound 4, and the cells were mounted 4 hours later. Zeiss Axio Vert A1 confocal microscope was used to detect HTTexon1-MBP-His by anti-His immunofluorescence and LC3B-GFP by GFP fluorescence. The fluorescence signal of the former was red, and the fluorescence signal of the latter was green. Cells transfected with LC3B-GFP alone were detected in the His channel, and cells transfected with HTTexon1-MBP-His alone were detected in the GFP channel to verify the specificity of the detection method. The puncta imaged at both channels were counted and the co-localization was analyzed (Figure 23). The y-axis of the histogram in Figure 23 showed the proportion of the red⁺ (HTTexon1-MBP-His) puncta having the same localization as the green⁺ (LC3B-GFP) puncta, based on the total red⁺ (HTTexon1-MBP-His) puncta.

### 9.2 Co-localization between mHTT and LC3B in the striatal cells of HD mice under the influence of compounds

The source of HD mouse striatal cells STHdh^{Q111/Q111} was as described above.

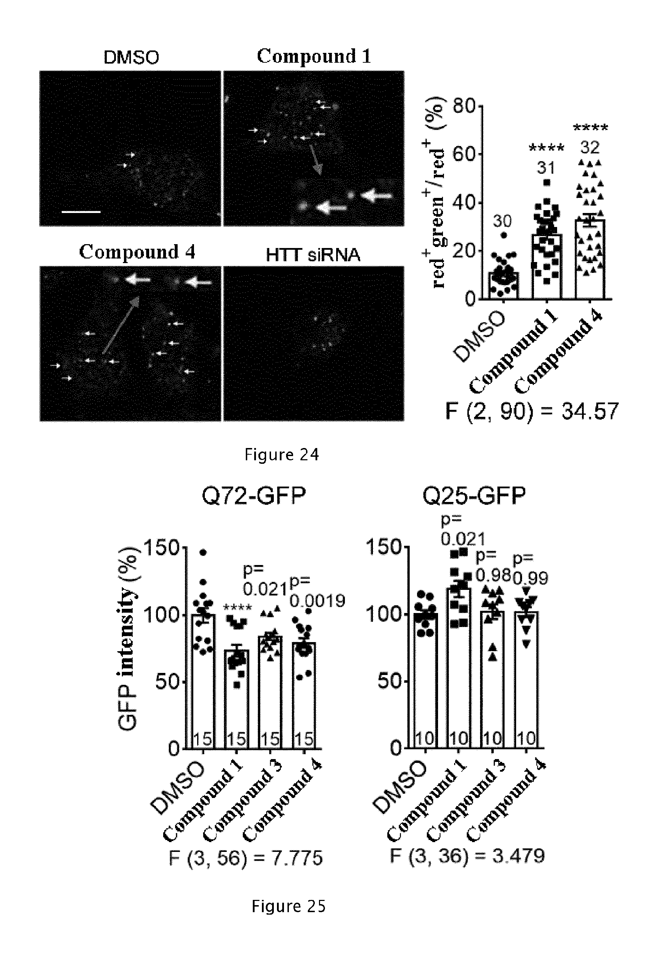

Using a method similar to 9.1, mHTT was detected by antibody 2166, and endogenous LC3B was detected by anti-LC3 antibody that specifically detects LC3B. Various fluorescent pixels were measured respectively and the co-localization between endogenous mHTT and LC3B in STHdh^{Q111/Q111} cells was analyzed (Figure 24). The y-axis of the histogram in Figure 24 showed the proportion of the red⁺ (HTTexon1-MBP-His) puncta having the same localization as the green⁺ (LC3B-GFP), based on the total red⁺ (HTTexon1-MBP-His) puncta. The compound obtained by the screening method of the present invention can promote the co-localization between the protein with abnormally expanded polyQ and LC3B in cells, which confirmed that the compound can simultaneously bind to the protein with abnormally expanded polyQ and LC3B to promote the enrichment of the protein with abnormally expanded polyQ to the cell substructures with autophagy function, thereby reducing the level of the protein with abnormally expanded polyQ in the cell through autophagy.

The inventor also unexpectedly discovered that common detectable markers such as GFP can be effectively used in the screening method of the present invention. Therefore, although the non-labeled detection methods, such as the unlabeled oblique-incidence reflectivity difference, have the advantage of not interfering with the detected object, the screening method of the present invention can also use other known detection methods, such as fluorescence.

### Example 10 Use of proteins containing expanded polyglutamine for affinity screening

### 10.1 Preparation of polyQ-GFP protein

(1) PolyQ-GFP cDNA sequence (expressing Met-polyQ-GFP, wherein polyQ was Q72 or Q25) was synthesized de novo and subcloned into pcDNA vector. It was transfected into HEK293T cells (ATCC, CRL-3216) through forward transfection to obtain Q72-GFP expressing cells and Q25-GFP expressing cells, respectively. And the same method was used to obtain cells expressing Q53-GFP, cells expressing Q46-GFP, and cells expressing Q38-GFP.
(2) The polyQ-GFP proteins expressed by (1) were purified separately to obtain Q72-GFP, Q53-GFP, Q46-GFP, Q38-GFP and Q25-GFP (SEQ ID NO: 6). Among them, Q72-GFP, Q53-GFP, Q46-GFP, Q38-GFP differed from the amino acid sequence of SEQ ID NO: 6 in the length of the expanded polyglutamine.

### 10.2 Affinity screening

To identify compound(s) that can selectively bind to proteins containing expanded polyglutamine, screening was conducted using a method similar to 1.1, except for that the target protein HTTexon1-Q72-NMP in step (1) of 1.1 was replaced with the polyQ-GFP protein prepared in 10.1, and the target protein HTTexon1-Q25-NMP in step (2) of 1.1 was replaced with GFP.

Among them, by analyzing the difference images of compound 1, compound 3, compound 4 after incubation and before incubation with polyQ-GFP protein (the image after incubation minus the image before incubation), it was found that these compounds did not bind to Q25-GFP or GFP, but binded to proteins with expanded polyglutamine. For example, compound 1, compound 3, and compound 4 showed certain binding affinity to proteins with 38 or more glutamine repeats. Using a method similar to 1.1, OI-RD was used to measure the affinity reaction parameters of compound 1, compound 3 and compound 4 with Q72-GFP, Q53-GFP, Q46-GFP and Q38-GFP. The results are shown in Tables 5-1 to 5-3.

**Table 5-1. The association rate constants, dissociation rate constants, and equilibrium dissociation constants of the affinity reactions of compound 1 with Q72-GFP, Q53-GFP, Q46-GFP, and Q38-GFP**

| | **Kₒₙ** (min•nM)⁻¹ | **K_{off}** (min)⁻¹ | **K_{d}** (nM) |
|---|---|---|---|
| Q72-GFP | 8.37×10⁻⁵ | 1.51×10⁻² | 180.4 |
| Q53-GFP | 1.51×10⁻⁴ | 3.78×10⁻³ | 25.0 |
| Q46-GFP | 3.04×10⁻⁴ | 1.83×10⁻² | 60.2 |
| Q38-GFP | 1.06×10⁻⁵ | 2.03×10⁻² | 1915.1 |

**Table 5-2. The association rate constants, dissociation rate constants and equilibrium dissociation constants of the affinity reactions of compound 3 with Q72-GFP, Q53-GFP, Q46-GFP, and Q38-GFP**

| | **Kₒₙ** (min•nM)⁻¹ | **K_{off}** (min)⁻¹ | **K_{d}** (nM) |
|---|---|---|---|
| Q72-GFP | 2.23×10⁻⁵ | 7.94×10⁻³ | 356.1 |
| Q53-GFP | 1.25×10⁻⁴ | 1.35×10⁻² | 108.0 |
| Q46-GFP | 1.29×10⁻⁴ | 7.67×10⁻³ | 59.5 |
| Q38-GFP | 8.72×10⁻⁶ | 2.62×10⁻² | 3004.6 |

**Table 5-3. The association rate constants, dissociation rate constants, and equilibrium dissociation constants of the affinity reactions of compound 4 with Q72-GFP, Q53-GFP, Q46-GFP, and Q38-GFP**

| | **Kₒₙ** (min•nM)⁻¹ | **K_{off}** (min)⁻¹ | **K_{d}** (nM) |
|---|---|---|---|
| Q72-GFP | 6.20×10⁻⁵ | 1.92×10⁻² | 309.7 |
| Q53-GFP | 1.08×10⁻⁴ | 4.65×10⁻³ | 43.1 |
| Q46-GFP | 7.65×10⁻⁶ | 2.57×10⁻² | 3359.5 |
| Q38-GFP | 2.99×10⁻⁶ | 1.5×10⁻² | 5016.7 |

### 10.3 MST detection of the affinity of compounds to proteins containing expanded polyglutamine

Following the method in 2.2, the affinity of compound 1, compound 3, compound 4 to the polyQ-GFP protein (Q25-GFP and Q72-GFP) was detected with a micro thermophoresis instrument for verification. The compound of the present invention showed no binding affinity to Q25-GFP, and the K_{d} of the compounds with Q72-GFP is shown in Table 6.

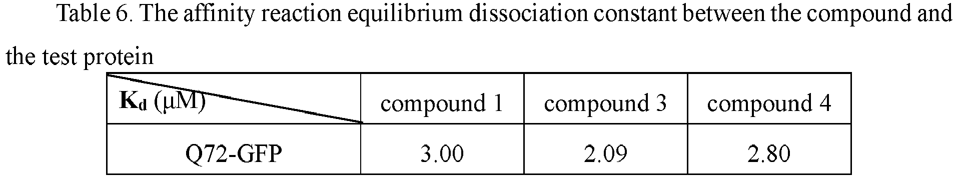

It can be seen from the above experiments that the compound of the present invention selectively binded to proteins containing expanded polyglutamine. A protein with abnormally expanded polyQ or a fragment thereof containing the polyQ part can be applied to the screening method of the present invention. The screening method of the present invention can be effectively applied to screening or identifying compound(s) for the treatment or prevention of diseases caused by proteins containing expanded polyglutamine, such as polyQ-related neurodegenerative disorders.

### Example 11 Effects of the compounds on the level of the protein containing expanded polyglutamine in the cell

The Q72-GFP expressing cells and Q25-GFP expressing cells obtained in step (1) of 10.1 were used. The cells were treated with 100 nM compound 1, or 100 nM compound 3 or 50 nM compound 4 for 2 days. Then the level of polyQ-GFP was measured by Incucyte fluorescence counting (Figure 25). The compounds obtained according to the screening method of the present invention can selectively reduce the level of proteins containing expanded polyglutamine in cells. It showed that the compounds obtained from screening had extended pharmacological effects and can be used to treat or prevent diseases caused by proteins containing expanded polyglutamine.

The following Examples 12 to 14 further test the effects of the compounds obtained by the screening method of the present invention on the levels of ATXN3 with abnormally expanded polyQ and ATXN1 with abnormally expanded polyQ, and it is further confirmed that the screening method of the present invention can use a protein containing an expanded polyglutamine as a target protein representing a disease state.

### Example 12 Using ATXN3 with abnormally expanded polyQ for affinity screening

### 12.1 Affinity screening

Screening was conducted using a method similar to 1.1, except for that the target protein HTTexon1-Q72-NMP in step (1) of 1.1 was replaced with MBP-ATXN3-Q68 and the target protein HTTexon1-Q25-NMP in step (2) of 1.1 was replaced with MBP-ATXN3-Q28, to obtain compounds that can selectively bind to ATXN3 with abnormally expanded polyQ (MBP-ATXN3-Q68).

By analyzing the difference images of compound 1, compound 3, compound 4 after incubation and before incubation with MBP-ATXN3-Q68 and MBP-ATXN3-Q28 respectively (the image after incubation minus the image before incubation), it was found that these compounds did not bind to MBP-ATXN3-Q28, but binded to MBP-ATXN3-Q68.

### 12.2 MST detection of the affinity of the compound to the ATXN3 with abnormally expanded polyQ

Following the method in 2.2, the affinity of the compound with MBP-ATXN3-Q28 and MBP-ATXN3-Q68 protein was verified by microscale thermophoresis.

The results showed that compound 1, compound 3 and compound 4 showed no binding affinity with normal ATXN3 protein (MBP-ATXN3-Q28), and the K_{d} of the compounds with abnormal ATXN3 protein (MBP-ATXN3-Q68) is shown in Table 7.

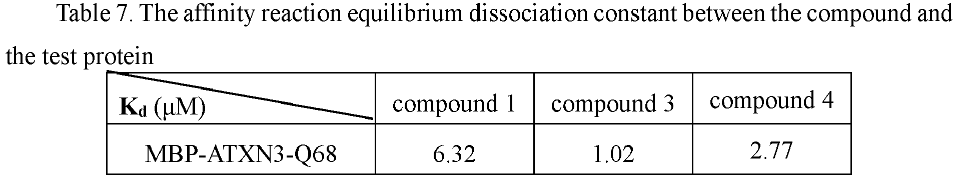

### Example 13 Effects of compounds on ATXN3 level in fibroblasts of patients with spinocerebellar ataxia type 3

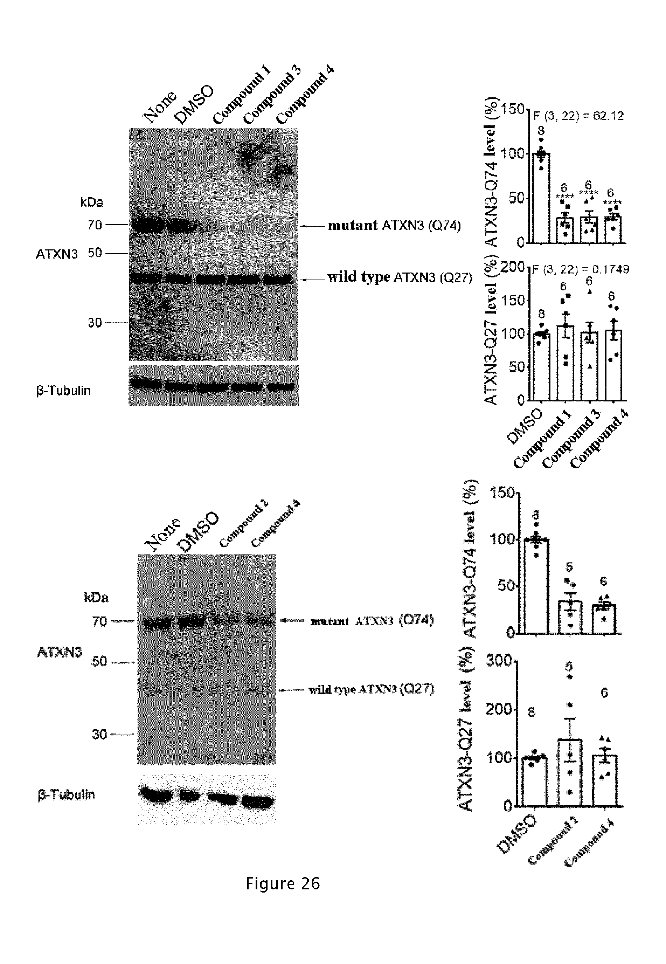

SCA3 patient fibroblasts (Q74), wild-type cells (Q27) were treated with 100 nM compound 1, or 100 nM compound 2, or 100 nM compound 3 or 50 nM compound 4 for 2 days. Then the levels of mutant ATXN3 protein (ATXN3-Q74, SEQ ID NO: 2) and wild-type ATXN3 protein (ATXN3-Q27, SEQ ID NO: 7) were determined by Western-blots (Figure 26). A decrease in mutant ATXN3 protein level was observed on SCA3 patient fibroblasts (Q74), but no decrease in wild-type ATXN3 protein level was observed. Therefore, the compound of the present invention can be used to treat SCA3.

### Example 14 Using ATXN1 with abnormally expanded polyQ for affinity screening

### 14.1 Preparation of ATXN1 with abnormally expanded polyQ

(1) PolyQ-ATXN1 cDNA (where polyQ is Q92) was synthesized de novo and subcloned into pcDNA vector. It transfected into HEK293T cells (ATCC, CRL-3216) to obtain cells expressing His-ATXN1-Q92 (SEQ ID NO: 3 with a His tag at the N-terminal).
(2) The His-ATXN1-Q92 expressed in (1) was purified to obtain ATXN1 with abnormally expanded polyQ.

### 14.2 Affinity screening

To identify compound(s) that can bind to ATXN1 with abnormally expanded polyQ, screening was conducted using a method similar to step (1) of 1.1, except for that the target protein HTTexon1-Q72-NMP in step (1) of 1.1 was replaced with His-ATXN1-Q92.

By analyzing the difference images of compound 1, compound 2, compound 3, compound 4 after incubation and before incubation with His-ATXN1-Q92 (the image after incubation minus the image before incubation), it was found that these compounds can bind to His-ATXN1-Q92.

### Example 15 Effects of Compounds on the Level of ATXN1 with Abnormally Expanded PolyQ in Cells

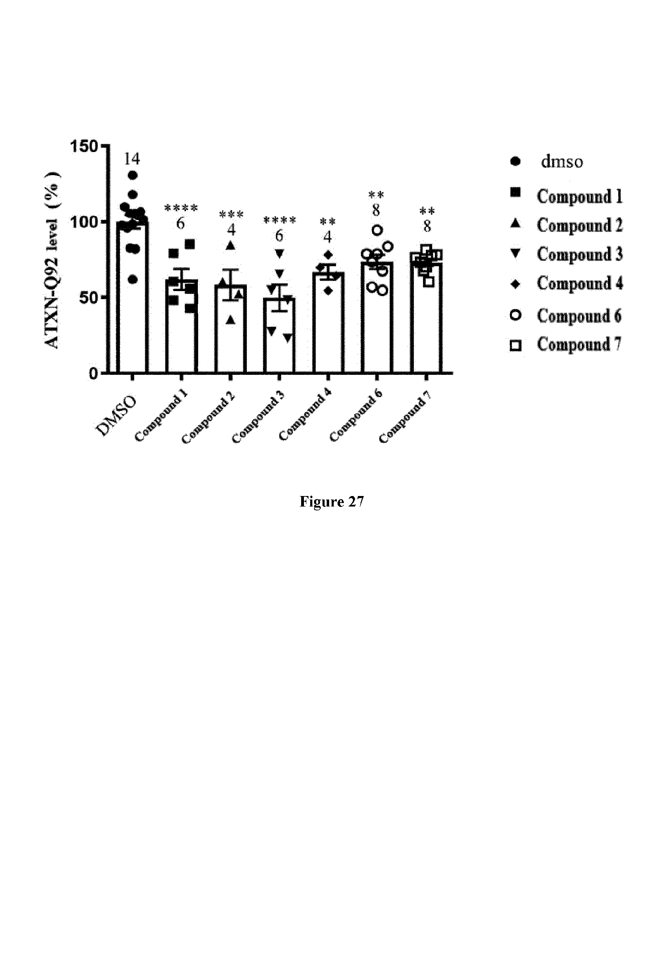

The His-ATXN1-Q92-expressing cells obtained in 14.1 were treated with 100 nM compound (compound 1, or compound 2, or compound 3, or compound 4, or compound 6, or compound 7) for 2 days. Then the level of mutant ATXN1 protein (His-ATXN1-Q92) was detected by HTRF (antibody pair: anti-His-Tb/MW1-D2, Figure 27). Decreased levels of mutant ATXN1 were observed on fibroblasts (Q92) of SCA1 patients. Therefore, the compounds of the present invention can be used to treat or prevent spinocerebellar ataxia type 1.

In summary, it has been experimentally proved that the compounds obtained by the screening method of the present invention can reduce the levels of a variety of proteins containing expanded polyglutamine, such as the levels of mutant mHTT, mutant ATXN3, and mutant ATXN1. Therefore, the screening method of the present invention is effectively used for screening or identifying compound(s). Such compounds can be used to treat or prevent diseases caused by proteins containing expanded polyglutamine, such as polyQ-related neurodegenerative disorders.

### Sequence Listing

[SEQ ID NO: 1] HTTexon1-Q72: 
MATLEKLMKAFESLKSFQQQQQQQQQQQQQQQQQQQQQQQQQQQQQQQQQQQQQ
QQQQQQQQQQQQQQQQQQQQQQQQQQQQQQQQQQQQQPPPPPPPPPPPQLPQPPPQ
AQPLLPQPQPPPPPPPPPPPGPAVAEEPLHR
[SEQ ID NO: 2] ATXN3-Q74: 
MESIFHEKQEGSLCAQHCLNNLLQGEYFSPVELSSIAHQLDEEERMRMAEGGVTSED
YRTFLQQPSGNMDDSGFFSIQVISNALKVWGLELILFNSPEYQRLRIDPINERSFICNYK
EHWFTVRKLGKQWFNLNSLLTGPELISDTYLALFLAQLQQEGYSIFVVKGDLPDCEA
 
DQLLQMIRVQQMHRPKLIGEELAQLKEQRVHKTDLERMLEANDGSGMLDEDEEDLQ
RALALSRQEIDMEDEEADLRRAIQLSMQGSSRNISQDMTQTSGTNLTSEELRKRREAY
FEKQQQKQQQQQQQQQQQQQQQQQQQQQQQQQQQQQQQQQQQQQQQQQQQQQQQQQ
QQQQQQQQQQQQQQQQQQQQQQQQQQQQQQQRDLSGQSSHPCERPATSSGALGSDLG
KACSPFIMFATFTLYLT
[SEQ ID NO: 3] ATXN1-Q92: 
MKSNQERSNECLPPKKREIPATSRSSEEKAPTLPSDNHRVEGTAWLPGNPGGRGH
GGGRHGPAGTSVELGLQQGIGLHKALSTGLDYSPPSAPRSVPVATTLPAAYATPQPGTP
VSPVQYAHLPHTFQFIGSSQYSGTYASFIPSQLIPPTANPVTSAVASAAGATTPSQRSQLE
AYSTLLANMGSLSQTPGHKAEQQQQQQQQQQQQQQQQQQQQQQQQQQQQQQQQQQ
QQQQQQQQQQQQQQQQQQQQQQQQQQQQQQQQQQQQQQQQQQQQQQQQQQQQQ
QQQQQQHQHQQQQQQQQQQQQQQQQHLSRAPGLITPGSPPPAQQNQYVHISSSPQNT
GRTASPPAIPVHLHPHQTMIPHTLTLGPPSQVVMQYADSGSHFVPREATKKAESSRLQQ
AIQAKEVLNGEMEKSRRYGAPSSADLGLGKAGGKSVPHPYESRHVVVHPSPSDYSSR
DPSGVRASVMVLPNSNTPAADLEVQQATHREASPSTLNDKSGLHLGKPGHRSYALSP
HTVIQTTHSASEPLPVGLPATAFYAGTQPPVIGYLSGQQQAITYAGSLPQHLVIPGTQPL
LIPVGSTDMEASGAAPAIVTSSPQFAAVPHTFVTTALPKSENFNPEALVTQAAYPAMVQ
AQIHLPVVQSVASPAAAPPTLPPYFMKGSIIQLANGELKKVEDLKTEDFIQSAEISNDL
KIDSSTVERIEDSHSPGVAVIQFAVGEHRAQVSVEVLVEYPFFVFGQGWSSCCPERTSQ
LFDLPCSKLSVGDVCISLTLKNLKNGSVKKGQPVDPASVLLKHSKADGLAGSRHRYA
EQENGINQGSAQMLSENGELKFPEKMGLPAAPFLTKIEPSKPAATRKRRWSAPESRKL
EKSEDEPPLTLPKPSLIPQEVKICIEGRSNVGK
[SEQ ID NO: 4] HTT-Q23: 
MATLEKLMKAFESLKSFQQQQQQQQQQQQQQQQQQQQQPPPPPPPPPPPQLPQPP
PQAQPLLPQPQPPPPPPPPPPGPAVAEEPLHRPKKELSATKKDRVNHCLTICENIVAQSV
RNSPEFQKLLGIAMELFLLCSDDAESDVRMVADECLNKVIKALMDSNLPRLQLELYK
EIKKNGAPRSLRAALWRFAELAHLVRPQKCRPYLVNLLPCLTRTSKRPEESVQETLAA
AVPKIMASFGNFANDNEIKVLLKAFIANLKSSSPTIRRTAAGSAVSICQHSRRTQYFYS
WLLNVLLGLLVPVEDEHSTLLILGVLLTLRYLVPLLQQQVKDTSLKGSFGVTRKEMEV
SPSAEQLVQVYELTLHHTQHQDHNVVTGALELLQQLFRTPPPELLQTLTAVGGIGQLT
AAKEESGGRSRSGSIVELIAGGGSSCSPVLSRKQKGKVLLGEEEALEDDSESRSDVSSS
ALTASVKDEISGELAASSGVSTPGSAGHDIITEQPRSQHTLQADSVDLASCDLTSSATD
GDEEDILSHSSSQVSAVPSDPAMDLNDGTQASSPISDSSQTTTEGPDSAVTPSDSSEIVL
DGTDNQYLGLQIGQPQDEDEEATGILPDEASEAFRNSSMALQQAHLLKNMSHCRQPS
DSSVDKFVLRDEATEPGDQENKPCRIKGDIGQSTDDDSAPLVHCVRLLSASFLLTGGK
 
NVLVPDRDVRVSVKALALSCVGAAVALHPESFFSKLYKVPLDTTEYPEEQYVSDILNY
IDHGDPQVRGATAILCGTLICSILSRSRFHVGDWMGTIRTLTGNTFSLADCIPLLRKTLK
DESSVTCKLACTAVRNCVMSLCSSSYSELGLQLIIDVLTLRNSSYWLVRTELLETLAEI
DFRLVSFLEAKAENLHRGAHHYTGLLKLQERVLNNVVIHLLGDEDPRVRHVAAASLI
RLVPKLFYKCDQGQADPVVAVARDQSSVYLKLLMHETQPPSHFSVSTITRIYRGYNLL
PSITDVTMENNLSRVIAAVSHELITSTTRALTFGCCEALCLLSTAFPVCIWSLGWHCGV
PPLSASDESRKSCTVGMATMILTLLSSAWFPLDLSAHQDALILAGNLLAASAPKSLRSS
WASEEEANPAATKQEEVWPALGDRALVPMVEQLFSHLLKVINICAHVLDDVAPGPAIK
AALPSLTNPPSLSPIRRKGKEKEPGEQASVPLSPKKGSEASAASRQSDTSGPVTTSKSSS
LGSFYHLPSYLKLHDVLKATHANYKVTLDLQNSTEKFGGFLRSALDVLSQILELATLQ
DIGKCVEEILGYLKSCFSREPMMATVCVQQLLKTLFGTNLASQFDGLSSNPSKSQGRA
QRLGSSSVRPGLYHYCFMAPYTHFTQALADASLRNMVQAEQENDTSGWFDVLQKVS
TQLKTNLTSVTKNRADKNAIHNHIRLFEPLVIKALKQYTTTTCVQLQKQVLDLLAQLV
QLRVNYCLLDSDQVFIGFVLKQFEYIEVGQFRESEAIIPNIFFFLVLLSYERYHSKQIIGIP
KIIQLCDGIMASGRKAVTHAIPALQPIVHDLFVLRGTNKADAGKELETQKEVVVSMLL
RLIQYHQVLEMFILVLQQCHKENEDKWKRLSRQIADIILPMLAKQQMHIDSHEALGV
LNTLFEILAPSSLRPVDMLLRSMFVTPNTMASVSTVQLWISGILAILRVLISQSTEDIVLS
RIQELSFSPYLISCTVINRLRDGDSTSTLEEHSEGKQIKNLPEETFSRFLLQLVGILLEDIV
TKQLKVEMSEQQHTFYCQELGTLLMCLIHIFKSGMFRRITAAATRLFRSDGCGGSFYT
LDSLNLRARSMITTHPALVLLWCQILLLVNHTDYRWWAEVQQTPKRHSLSSTKLLSPQ
MSGEEEDSDLAAKLGMCNREIVRRGALILFCDYVCQNLHDSEHLTWLIVNHIQDLISL
SHEPPVQDFISAVHRNSAASGLFIQAIQSRCENLSTPTMLKKTLQCLEGIHLSQSGAVLT
LYVDRLLCTPFRVLARMVDILACRRVEMLLAANLQSSMAQLPMEELNRIQEYLQSSG
LAQRHQRLYSLLDRFRLSTMQDSLSPSPPVSSHPLDGDGHVSLETVSPDKDWYVHLV
KSQCWTRSDSALLEGAELVNRIPAEDMNAFMMNSEFNLSLLAPCLSLGMSEISGGQK
SALFEAAREVTLARVSGTVQQLPAVHHVFQPELPAEPAAYWSKLNDLFGDAALYQSLP
TLARALAQYLVVVSKLPSHLHLPPEKEKDIVKFVVATLEALSWHLIHEQIPLSLDLQAG
LDCCCLALQLPGLWSVVSSTEFVTHACSLIYCVHFILEAVAVQPGEQLLSPERRTNTPK
AISEEEEEVDPNTQNPKYITAACEMVAEMVESLQSVLALGHKRNSGVPAFLTPLLRNIII
SLARLPLVNSYTRVPPLVWKLGWSPKPGGDFGTAFPEIPVEFLQEKEVFKEFIYRINTL
GWTSRTQFEETWATLLGVLVTQPLVMEQEESPPEEDTERTQINVLAVQAITSLVLSAMT
VPVAGNPAVSCLEQQPRNKPLKALDTRFGRKLSIIRGIVEQEIQAMVSKRENIATHHLY
QAWDPVPSLSPATTGALISHEKLLLQINPERELGSMSYKLGQVSIHSVWLGNSITPLRE
EEWDEEEEEEADAPAPSSPPTSPVNSRKHRAGVDIHSCSQFLLELYSRWILPSSSARRTP
AILISEVVRSLLVVSDLFTERNQFELMYVTLTELRRVHPSEDEILAQYLVPATCKAAAV
 
LGMDKAVAEPVSRLLESTLRSSHLPSRVGALHGVLYVLECDLLDDTAKQLIPVISDYLL
SNLKGIAHCVNIHSQQHVLVMCATAFYLIENYPLDVGPEFSASIIQMCGVMLSGSEEST
PSIIYHCALRGLERLLLSEQLSRLDAESLVKLSVDRVNVHSPHRAMAALGLMLTCMYT
GKEKVSPGRTSDPNPAAPDSESVIVAMERVSVLFDRIRKGFPCEARVVARILPQFLDDF
FPPQDIMNKVIGEFLSNQQPYPQFMATVVYKVFQTLHSTGQSSMVRDWVMLSLSNFT
QRAPVAMATWSLSCFFVSASTSPWVAAILPHVISRMGKLEQVDVNLFCLVATDFYRHQ
IEEELDRRAFQSVLEVVAAPGSPYHRLLTCLRNVHKVTTC
[SEQ ID NO: 5] HTTexon1-Q25: 
MATLEKLMKAFESLKSFQQQQQQQQQQQQQQQQQQQQQQQQPPPPPPPPPPPQLP
QPPPQAQPLLPQPQPPPPPPPPPPGPAVAEEPLHR
[SEQ ID NO: 6] Q25-GFP: 
MQQQQQQQQQQQQQQQQQQQQQQQMSKGEELFTGVVPILVELDGDVNGH
KFSVRGEGEGDATNGKLTLKFICTTGKLPVPWPTLVTTLTYGVQCFSRYPDHMKRHD
FFKSAMPEGYVQERTISFKDDGTYKTRAEVKFEGDTLVNRIELKGIDFKEDGNILGHK
LEYNFNSHNVYITADKQKNGIKANFKIRHNVEDGSVQLADHYQQNTPIGDGPVLLPD
NHYLSTQSVLSKDPNEKRDHMVLLEFVTAAGITHGGSG
[SEQ ID NO: 7] ATXN3-Q27: 
MESIFHEKQEGSLCAQHCLNNLLQGEYFSPVELSSIAHQLDEEERMRMAEGGVTSED
YRTFLQQPSGNMDDSGFFSIQVISNALKVWGLELILFNSPEYQRLRIDPINERSFICNYK
EHWFTVRKLGKQWFNLNSLLTGPELISDTYLALFLAQLQQEGYSIFVVKGDLPDCEA
DQLLQMIRVQQMHRPKLIGEELAQLKEQRVHKTDLERMLEANDGSGMLDEDEEDLQ
RALALSRQEIDMEDEEADLRRAIQLSMQGSSRNISQDMTQTSGTNLTSEELRKRREAY
FEKQQQKQQQQQQQQQQQQQQQQQQQQQQQQQRDLSGQSSHPCERPATSSGAL
GSDLGKACSPFIMFATFTLYLT
[SEQ ID NO: 8] LC3B:

MPSEKTFKQRRTFEQRVEDVRLIREQHPTKIPVIIERYKGEKQLPVLDKTKFLVPDHVN
MSELIKIIRRRLQLNANQAFFLLVNGHSMVSVSTPISEVYESEKDEDGFLYMVYASQET
FGMKLSV

## Claims

1. A method for screening or identifying compound(s) for the treatment or prevention of a polyQ-related neurodegenerative disorder, comprising
(I): bringing the candidate compound(s) into contact with a test system, the test system comprising
(1) a protein with abnormally expanded polyQ or a fragment thereof containing the polyQ part, and
(2) a LC3B protein, a homologue thereof, or a fragment thereof; and
(II): determining the ability of the candidate compound(s) to modulate the binding between (1) a protein with abnormally expanded polyQ or a fragment thereof containing the polyQ part, and (2) a LC3B protein, a homologue thereof, or a fragment thereof; and selecting the compound(s) that positively modulate the binding.

2. The method of claim 1, wherein step (II) comprises:
respectively in the presence/absence of the candidate compound(s), determining the binding affinity between (1) a protein with abnormally expanded polyQ or a fragment thereof containing the polyQ part, and (2) a LC3B protein, a homologue thereof, or a fragment thereof, and/or
respectively in the presence/absence of the candidate compound(s), determining the binding amount of (1) a protein with abnormally expanded polyQ or a fragment thereof containing the polyQ part, and (2) a LC3B protein, a homologue thereof, or a fragment thereof.

3. The method of claim 1 or 2, wherein the protein with abnormally expanded polyQ comprises a polyQ part with polyQ length ≥ 40, ≥ 33, ≥ 41, ≥ 19, ≥ 46, ≥ 44, ≥ 39, ≥ 36, or ≥ 37;
preferably, the protein with abnormally expanded polyQ comprises,
ATXN1 with polyQ length ≥ 40 or a fragment thereof containing the polyQ part,
ATXN2 with polyQ length ≥ 33 or a fragment thereof containing the polyQ part,
ATXN3 with polyQ length ≥ 41 or a fragment thereof containing the polyQ part,
ATXN7 with polyQ length ≥ 19 or a fragment thereof containing the polyQ part,
ATXN12 with polyQ length ≥ 46 or a fragment thereof containing the polyQ part,
TBP with polyQ length ≥ 44 or a fragment thereof containing the polyQ part,
ATN1 with polyQ length ≥ 39 or a fragment thereof containing the polyQ part,
HTT with polyQ length ≥ 36 or a fragment thereof containing the polyQ part, or
AR with polyQ length ≥ 37 or a fragment thereof containing the polyQ part;
particularly preferably, the protein with abnormally expanded polyQ comprises,
ATXN1 with polyQ length ≥ 40 or a fragment thereof containing the polyQ part,
ATXN2 with polyQ length ≥ 33 or a fragment thereof containing the polyQ part,
ATXN3 with polyQ length ≥ 41 or a fragment thereof containing the polyQ part,
ATXN7 with polyQ length ≥ 19 or a fragment thereof containing the polyQ part, or
ATXN12 with polyQ length ≥ 46 or a fragment thereof containing the polyQ part,;
especially ATXN1 with polyQ length ≥ 40 or a fragment thereof containing the polyQ part,
or ATXN3 with polyQ length ≥ 41 or a fragment thereof containing the polyQ part.

4. The method of any one of claims 1-3, wherein (2) a LC3B protein, a homologue thereof, or a fragment thereof comprises a sequence having 25% or higher, 30% or higher, 35% or higher, 40% or higher, 50% or higher, 60% or higher, 70% or higher, 80% or higher, 90% or higher, 95% or higher or 100% sequence identity with the amino acid sequence of SEQ ID NO: 8.

5. The method of any one of claims 1-4, wherein the method for determining the binding is selected from the group consisting of in vitro pull-down test, Split-TEV, co-immunoprecipitation, affinity chromatography, complex co-purification, enzyme-linked immunosorbent assay, fluorescent molecular sieve, yeast two-hybrid, HIP-HOP, time-resolved fluorescence resonance energy transfer, chemiluminescence method, surface plasmon resonance, isothermal titration calorimetry, micro thermophoresis and any combination thereof; preferably, the method for determining the binding is selected from the group consisting of in vitro pull-down experiments, co-immunoprecipitation, enzyme-linked immunosorbent assay, fluorescent molecular sieves, time-resolved fluorescence resonance energy transfer, and any combination thereof.

6. The method of any one of claims 1-5, further comprising a step of pre-screening the candidate compound(s) for affinity before step (I), comprising
(A): detecting the binding affinity of the candidate compound(s) to (1) a protein with abnormally expanded polyQ or a fragment thereof containing the polyQ part, and/or (B): detecting the binding affinity of the candidate compound(s) to (2) a LC3B protein, a homologue thereof, or a fragment thereof; and
(C): selecting the compound(s) that have binding affinity to (1) a protein with abnormally expanded polyQ or a fragment thereof containing the polyQ part and/or (2) a LC3B protein, a homologue thereof, or a fragment thereof; wherein step (A) and step (B) can be carried out independently in any order.

7. The method of claim 6, wherein
step (A) comprises: determining the affinity reaction equilibrium dissociation constant of the candidate compound(s) and (1) a protein with abnormally expanded polyQ or a fragment thereof containing the polyQ part; and selecting a compound whose affinity reaction equilibrium dissociation constant is 100 µM or less, preferably 10 µM or less, particularly preferably 1 µM or less; and/or
step (B) comprises: determining the affinity reaction equilibrium dissociation constant of the candidate compound(s) and (2) a LC3B protein, a homologue thereof, or a fragment thereof; and selecting the compound(s) whose affinity reaction equilibrium dissociation constant is 100 µM or less, preferably 10 µM or less, particularly preferably 1 µM or less.

8. The method of claim 6 or 7, wherein the method for determining binding affinity is selected from the group consisting of proximity scintillation analysis, fluorescence resonance energy transfer, fluorescence polarization detection, fluorescent molecular sieves, micro thermophoresis, chemiluminescence, surface plasmon resonance, isothermal titration calorimetry, oblique-incidence reflectivity difference and any combination thereof; preferably selected from the group consisting of fluorescence resonance energy transfer, fluorescence polarization detection, oblique-incidence reflectivity difference and any combination thereof; particularly preferably oblique-incidence reflectivity difference.

9. The method of any one of claims 6-8, wherein
the step of pre-screening the candidate compound(s) for affinity is performed by high-throughput screening using the oblique-incidence reflectivity difference; comprising:
(a) immobilizing the candidate compound(s) to prepare a compound chip, and scanning the chip to obtain the image before incubation;
(b) incubating the compound chip with the target protein, and scanning the chip to obtain the image after incubation;
wherein the target protein is (1) a protein with abnormally expanded polyQ or a fragment thereof containing the polyQ part or (2) a LC3B protein, a homologue thereof, or a fragment thereof; and
(c) analyzing the difference image (the image after incubation minus the image before incubation); and selecting the compound(s) that can bind to both (1) a protein with abnormally expanded polyQ or a fragment thereof containing the polyQ part, and (2) a LC3B protein, a homologue thereof, or a fragment thereof.

10. The method of any one of claims 1-9, further comprising the following steps:
(D): detecting the binding of the candidate compound(s) to a normal polyQ protein or a fragment thereof containing the polyQ part; and selecting the compound(s) that do not bind to the normal polyQ protein or the fragment thereof containing the polyQ part.

11. Use of a compound obtained by the method of any one of claims 1-10 or a pharmaceutical composition thereof in the preparation of a medicament for the treatment or prevention of a polyQ-related neurodegenerative disorder, or use in the preparation of a diagnostic reagent or a kit for detecting subjects considered to be suffering from or susceptible to a polyQ-related neurodegenerative disorder.

12. The use of claim 11, wherein the polyQ-related neurodegenerative disorder is selected from the group consisting of spinocerebellar ataxia type 1, spinocerebellar ataxia type 2, spinocerebellar ataxia type 3, spinocerebellar ataxia type 7, spinocerebellar ataxia type 12 and spinocerebellar ataxia type 17, especially spinocerebellar ataxia type 1 and spinocerebellar ataxia type 3.

13. The use of claim 11, wherein the polyQ-related neurodegenerative disorder is selected from the group consisting of dentatorubral-pallidoluysian atrophy, Huntington's disease and spinal-bulbar muscular atrophy, especially Huntington's disease.

14. The use of claim 11, wherein the detection includes the step of analyzing a target sample obtained from said subject, comprising:
i) labeling the compound with a detectable label such as a fluorescent group;
ii) treating the target sample with the compound obtained in i);
iii) detecting the position and/or intensity of the detectable signal in the target sample; and obtaining the position and/or content of the protein with abnormally expanded polyQ or the fragment thereof containing the polyQ part in the target sample.

15. Use of the compound or a pharmaceutical composition thereof in the preparation of a medicament for the treatment or prevention of a polyQ-related neurodegenerative disorder, or use in the preparation of a diagnostic reagent or a kit for detecting subjects considered to be suffering from or susceptible to a polyQ-related neurodegenerative disorder, wherein the compound is selected from:

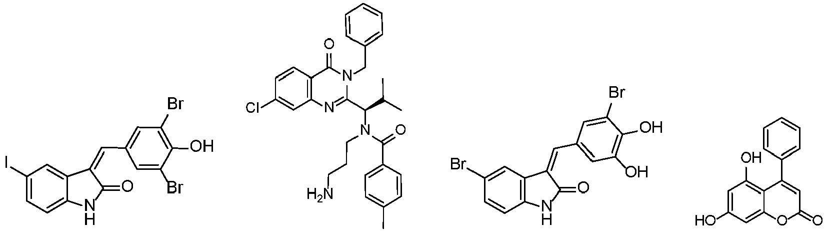

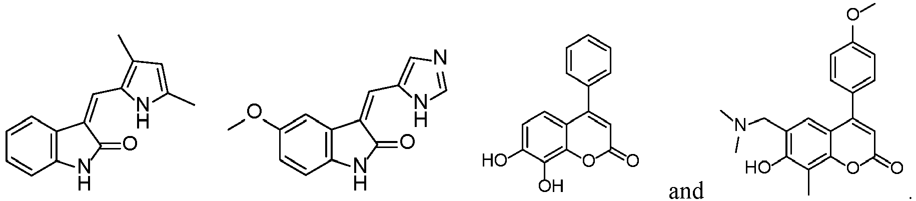

16. A pharmaceutical composition, detection reagent or kit comprising a compound obtained by the method of any one of claims 1-10.

17. A pharmaceutical composition, detection reagent, kit or screening system, which comprises at least one compound selected from the following:

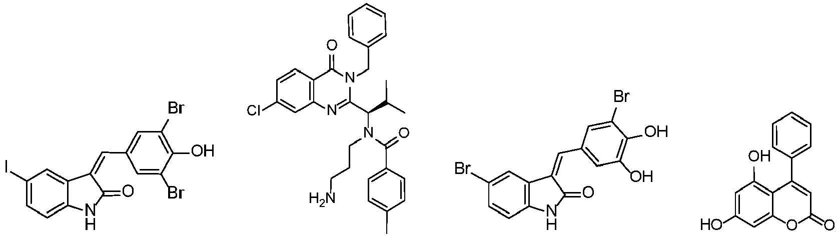

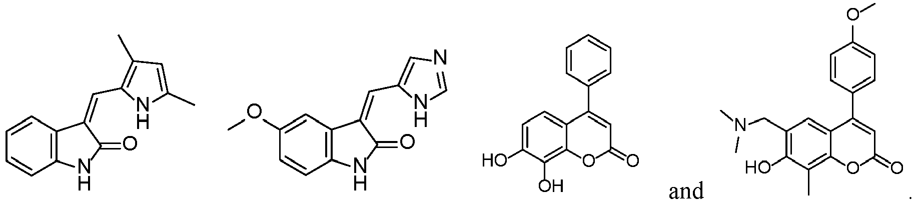

18. A screening system, comprising
(1) a protein with abnormally expanded polyQ or a fragment thereof containing the polyQ part, and
(2) a LC3B protein, a homologue thereof, or a fragment thereof; preferably, the screening system is in the form of a kit.

19. Use of a compound or a pharmaceutical composition thereof that positively modulates the binding between (1) a protein with abnormally expanded polyQ or a fragment thereof containing the polyQ part, and (2) a LC3B protein, a homologue thereof, or a fragment thereof in the preparation of a medicament for the treatment or prevention of a polyQ-related neurodegenerative disorder.


SEQUENCE LISTING

<110>  Fudan University

<120>  A METHOD FOR SCREENING COMPOUNDS FOR TREATING OR PREVENTING
POLYQ-RELATED NEURODEGENERATIVE DISORDER

<130>  P2019TC976

<160>  8

<170>  PatentIn version 3.5

<210>  1
<211>  138
<212>  PRT
<213>  Homo sapiens

<220>
<223>  HTTexon1-Q72

<400>  1

Met Ala Thr Leu Glu Lys Leu Met Lys Ala Phe Glu Ser Leu Lys Ser
1               5                   10                  15

Phe Gln Gln Gln Gln Gln Gln Gln Gln Gln Gln Gln Gln Gln Gln Gln
            20                  25                  30

Gln Gln Gln Gln Gln Gln Gln Gln Gln Gln Gln Gln Gln Gln Gln Gln
        35                  40                  45

Gln Gln Gln Gln Gln Gln Gln Gln Gln Gln Gln Gln Gln Gln Gln Gln
    50                  55                  60

Gln Gln Gln Gln Gln Gln Gln Gln Gln Gln Gln Gln Gln Gln Gln Gln
65                  70                  75                  80

Gln Gln Gln Gln Gln Gln Gln Gln Gln Pro Pro Pro Pro Pro Pro Pro
                85                  90                  95

Pro Pro Pro Pro Gln Leu Pro Gln Pro Pro Pro Gln Ala Gln Pro Leu
            100                 105                 110

Leu Pro Gln Pro Gln Pro Pro Pro Pro Pro Pro Pro Pro Pro Pro Gly
        115                 120                 125

Pro Ala Val Ala Glu Glu Pro Leu His Arg
    130                 135

<210>  2
<211>  412
<212>  PRT
<213>  Homo sapiens

<220>
<223>  ATXN3-Q74

<400>  2

Met Glu Ser Ile Phe His Glu Lys Gln Glu Gly Ser Leu Cys Ala Gln
1               5                   10                  15

His Cys Leu Asn Asn Leu Leu Gln Gly Glu Tyr Phe Ser Pro Val Glu
            20                  25                  30

Leu Ser Ser Ile Ala His Gln Leu Asp Glu Glu Glu Arg Met Arg Met
        35                  40                  45

Ala Glu Gly Gly Val Thr Ser Glu Asp Tyr Arg Thr Phe Leu Gln Gln
    50                  55                  60

Pro Ser Gly Asn Met Asp Asp Ser Gly Phe Phe Ser Ile Gln Val Ile
65                  70                  75                  80

Ser Asn Ala Leu Lys Val Trp Gly Leu Glu Leu Ile Leu Phe Asn Ser
                85                  90                  95

Pro Glu Tyr Gln Arg Leu Arg Ile Asp Pro Ile Asn Glu Arg Ser Phe
            100                 105                 110

Ile Cys Asn Tyr Lys Glu His Trp Phe Thr Val Arg Lys Leu Gly Lys
        115                 120                 125

Gln Trp Phe Asn Leu Asn Ser Leu Leu Thr Gly Pro Glu Leu Ile Ser
    130                 135                 140

Asp Thr Tyr Leu Ala Leu Phe Leu Ala Gln Leu Gln Gln Glu Gly Tyr
145                 150                 155                 160

Ser Ile Phe Val Val Lys Gly Asp Leu Pro Asp Cys Glu Ala Asp Gln
                165                 170                 175

Leu Leu Gln Met Ile Arg Val Gln Gln Met His Arg Pro Lys Leu Ile
            180                 185                 190

Gly Glu Glu Leu Ala Gln Leu Lys Glu Gln Arg Val His Lys Thr Asp
        195                 200                 205

Leu Glu Arg Met Leu Glu Ala Asn Asp Gly Ser Gly Met Leu Asp Glu
    210                 215                 220

Asp Glu Glu Asp Leu Gln Arg Ala Leu Ala Leu Ser Arg Gln Glu Ile

225                 230                 235                 240

Asp Met Glu Asp Glu Glu Ala Asp Leu Arg Arg Ala Ile Gln Leu Ser
                245                 250                 255

Met Gln Gly Ser Ser Arg Asn Ile Ser Gln Asp Met Thr Gln Thr Ser
            260                 265                 270

Gly Thr Asn Leu Thr Ser Glu Glu Leu Arg Lys Arg Arg Glu Ala Tyr
        275                 280                 285

Phe Glu Lys Gln Gln Gln Lys Gln Gln Gln Gln Gln Gln Gln Gln Gln
    290                 295                 300

Gln Gln Gln Gln Gln Gln Gln Gln Gln Gln Gln Gln Gln Gln Gln Gln
305                 310                 315                 320

Gln Gln Gln Gln Gln Gln Gln Gln Gln Gln Gln Gln Gln Gln Gln Gln
                325                 330                 335

Gln Gln Gln Gln Gln Gln Gln Gln Gln Gln Gln Gln Gln Gln Gln Gln
            340                 345                 350

Gln Gln Gln Gln Gln Gln Gln Gln Gln Gln Gln Gln Gln Gln Gln Gln
        355                 360                 365

Gln Arg Asp Leu Ser Gly Gln Ser Ser His Pro Cys Glu Arg Pro Ala
    370                 375                 380

Thr Ser Ser Gly Ala Leu Gly Ser Asp Leu Gly Lys Ala Cys Ser Pro
385                 390                 395                 400

Phe Ile Met Phe Ala Thr Phe Thr Leu Tyr Leu Thr
                405                 410

<210>  3
<211>  895
<212>  PRT
<213>  Homo sapiens

<220>
<223>  ATXN1-Q92

<400>  3

Met Lys Ser Asn Gln Glu Arg Ser Asn Glu Cys Leu Pro Pro Lys Lys
1               5                   10                  15

Arg Glu Ile Pro Ala Thr Ser Arg Ser Ser Glu Glu Lys Ala Pro Thr
            20                  25                  30

Leu Pro Ser Asp Asn His Arg Val Glu Gly Thr Ala Trp Leu Pro Gly
        35                  40                  45

Asn Pro Gly Gly Arg Gly His Gly Gly Gly Arg His Gly Pro Ala Gly
    50                  55                  60

Thr Ser Val Glu Leu Gly Leu Gln Gln Gly Ile Gly Leu His Lys Ala
65                  70                  75                  80

Leu Ser Thr Gly Leu Asp Tyr Ser Pro Pro Ser Ala Pro Arg Ser Val
                85                  90                  95

Pro Val Ala Thr Thr Leu Pro Ala Ala Tyr Ala Thr Pro Gln Pro Gly
            100                 105                 110

Thr Pro Val Ser Pro Val Gln Tyr Ala His Leu Pro His Thr Phe Gln
        115                 120                 125

Phe Ile Gly Ser Ser Gln Tyr Ser Gly Thr Tyr Ala Ser Phe Ile Pro
    130                 135                 140

Ser Gln Leu Ile Pro Pro Thr Ala Asn Pro Val Thr Ser Ala Val Ala
145                 150                 155                 160

Ser Ala Ala Gly Ala Thr Thr Pro Ser Gln Arg Ser Gln Leu Glu Ala
                165                 170                 175

Tyr Ser Thr Leu Leu Ala Asn Met Gly Ser Leu Ser Gln Thr Pro Gly
            180                 185                 190

His Lys Ala Glu Gln Gln Gln Gln Gln Gln Gln Gln Gln Gln Gln Gln
        195                 200                 205

Gln Gln Gln Gln Gln Gln Gln Gln Gln Gln Gln Gln Gln Gln Gln Gln
    210                 215                 220

Gln Gln Gln Gln Gln Gln Gln Gln Gln Gln Gln Gln Gln Gln Gln Gln
225                 230                 235                 240

Gln Gln Gln Gln Gln Gln Gln Gln Gln Gln Gln Gln Gln Gln Gln Gln
                245                 250                 255

Gln Gln Gln Gln Gln Gln Gln Gln Gln Gln Gln Gln Gln Gln Gln Gln
            260                 265                 270

Gln Gln Gln Gln Gln Gln Gln Gln Gln Gln Gln Gln Gln Gln Gln Gln

        275                 280                 285

His Gln His Gln Gln Gln Gln Gln Gln Gln Gln Gln Gln Gln Gln Gln
    290                 295                 300

Gln His Leu Ser Arg Ala Pro Gly Leu Ile Thr Pro Gly Ser Pro Pro
305                 310                 315                 320

Pro Ala Gln Gln Asn Gln Tyr Val His Ile Ser Ser Ser Pro Gln Asn
                325                 330                 335

Thr Gly Arg Thr Ala Ser Pro Pro Ala Ile Pro Val His Leu His Pro
            340                 345                 350

His Gln Thr Met Ile Pro His Thr Leu Thr Leu Gly Pro Pro Ser Gln
        355                 360                 365

Val Val Met Gln Tyr Ala Asp Ser Gly Ser His Phe Val Pro Arg Glu
    370                 375                 380

Ala Thr Lys Lys Ala Glu Ser Ser Arg Leu Gln Gln Ala Ile Gln Ala
385                 390                 395                 400

Lys Glu Val Leu Asn Gly Glu Met Glu Lys Ser Arg Arg Tyr Gly Ala
                405                 410                 415

Pro Ser Ser Ala Asp Leu Gly Leu Gly Lys Ala Gly Gly Lys Ser Val
            420                 425                 430

Pro His Pro Tyr Glu Ser Arg His Val Val Val His Pro Ser Pro Ser
        435                 440                 445

Asp Tyr Ser Ser Arg Asp Pro Ser Gly Val Arg Ala Ser Val Met Val
    450                 455                 460

Leu Pro Asn Ser Asn Thr Pro Ala Ala Asp Leu Glu Val Gln Gln Ala
465                 470                 475                 480

Thr His Arg Glu Ala Ser Pro Ser Thr Leu Asn Asp Lys Ser Gly Leu
                485                 490                 495

His Leu Gly Lys Pro Gly His Arg Ser Tyr Ala Leu Ser Pro His Thr
            500                 505                 510

Val Ile Gln Thr Thr His Ser Ala Ser Glu Pro Leu Pro Val Gly Leu
        515                 520                 525

Pro Ala Thr Ala Phe Tyr Ala Gly Thr Gln Pro Pro Val Ile Gly Tyr
    530                 535                 540

Leu Ser Gly Gln Gln Gln Ala Ile Thr Tyr Ala Gly Ser Leu Pro Gln
545                 550                 555                 560

His Leu Val Ile Pro Gly Thr Gln Pro Leu Leu Ile Pro Val Gly Ser
                565                 570                 575

Thr Asp Met Glu Ala Ser Gly Ala Ala Pro Ala Ile Val Thr Ser Ser
            580                 585                 590

Pro Gln Phe Ala Ala Val Pro His Thr Phe Val Thr Thr Ala Leu Pro
        595                 600                 605

Lys Ser Glu Asn Phe Asn Pro Glu Ala Leu Val Thr Gln Ala Ala Tyr
    610                 615                 620

Pro Ala Met Val Gln Ala Gln Ile His Leu Pro Val Val Gln Ser Val
625                 630                 635                 640

Ala Ser Pro Ala Ala Ala Pro Pro Thr Leu Pro Pro Tyr Phe Met Lys
                645                 650                 655

Gly Ser Ile Ile Gln Leu Ala Asn Gly Glu Leu Lys Lys Val Glu Asp
            660                 665                 670

Leu Lys Thr Glu Asp Phe Ile Gln Ser Ala Glu Ile Ser Asn Asp Leu
        675                 680                 685

Lys Ile Asp Ser Ser Thr Val Glu Arg Ile Glu Asp Ser His Ser Pro
    690                 695                 700

Gly Val Ala Val Ile Gln Phe Ala Val Gly Glu His Arg Ala Gln Val
705                 710                 715                 720

Ser Val Glu Val Leu Val Glu Tyr Pro Phe Phe Val Phe Gly Gln Gly
                725                 730                 735

Trp Ser Ser Cys Cys Pro Glu Arg Thr Ser Gln Leu Phe Asp Leu Pro
            740                 745                 750

Cys Ser Lys Leu Ser Val Gly Asp Val Cys Ile Ser Leu Thr Leu Lys
        755                 760                 765

Asn Leu Lys Asn Gly Ser Val Lys Lys Gly Gln Pro Val Asp Pro Ala
    770                 775                 780

Ser Val Leu Leu Lys His Ser Lys Ala Asp Gly Leu Ala Gly Ser Arg
785                 790                 795                 800

His Arg Tyr Ala Glu Gln Glu Asn Gly Ile Asn Gln Gly Ser Ala Gln
                805                 810                 815

Met Leu Ser Glu Asn Gly Glu Leu Lys Phe Pro Glu Lys Met Gly Leu
            820                 825                 830

Pro Ala Ala Pro Phe Leu Thr Lys Ile Glu Pro Ser Lys Pro Ala Ala
        835                 840                 845

Thr Arg Lys Arg Arg Trp Ser Ala Pro Glu Ser Arg Lys Leu Glu Lys
    850                 855                 860

Ser Glu Asp Glu Pro Pro Leu Thr Leu Pro Lys Pro Ser Leu Ile Pro
865                 870                 875                 880

Gln Glu Val Lys Ile Cys Ile Glu Gly Arg Ser Asn Val Gly Lys
                885                 890                 895

<210>  4
<211>  3144
<212>  PRT
<213>  Homo sapiens

<220>
<223>  HTT-Q23

<400>  4

Met Ala Thr Leu Glu Lys Leu Met Lys Ala Phe Glu Ser Leu Lys Ser
1               5                   10                  15

Phe Gln Gln Gln Gln Gln Gln Gln Gln Gln Gln Gln Gln Gln Gln Gln
            20                  25                  30

Gln Gln Gln Gln Gln Gln Gln Gln Pro Pro Pro Pro Pro Pro Pro Pro
        35                  40                  45

Pro Pro Pro Gln Leu Pro Gln Pro Pro Pro Gln Ala Gln Pro Leu Leu
    50                  55                  60

Pro Gln Pro Gln Pro Pro Pro Pro Pro Pro Pro Pro Pro Pro Gly Pro
65                  70                  75                  80

Ala Val Ala Glu Glu Pro Leu His Arg Pro Lys Lys Glu Leu Ser Ala
                85                  90                  95

Thr Lys Lys Asp Arg Val Asn His Cys Leu Thr Ile Cys Glu Asn Ile
        100                 105                 110

Val Ala Gln Ser Val Arg Asn Ser Pro Glu Phe Gln Lys Leu Leu Gly
    115                 120                 125

Ile Ala Met Glu Leu Phe Leu Leu Cys Ser Asp Asp Ala Glu Ser Asp
    130                 135                 140

Val Arg Met Val Ala Asp Glu Cys Leu Asn Lys Val Ile Lys Ala Leu
145                 150                 155                 160

Met Asp Ser Asn Leu Pro Arg Leu Gln Leu Glu Leu Tyr Lys Glu Ile
                165                 170                 175

Lys Lys Asn Gly Ala Pro Arg Ser Leu Arg Ala Ala Leu Trp Arg Phe
        180                 185                 190

Ala Glu Leu Ala His Leu Val Arg Pro Gln Lys Cys Arg Pro Tyr Leu
    195                 200                 205

Val Asn Leu Leu Pro Cys Leu Thr Arg Thr Ser Lys Arg Pro Glu Glu
    210                 215                 220

Ser Val Gln Glu Thr Leu Ala Ala Ala Val Pro Lys Ile Met Ala Ser
225                 230                 235                 240

Phe Gly Asn Phe Ala Asn Asp Asn Glu Ile Lys Val Leu Leu Lys Ala
                245                 250                 255

Phe Ile Ala Asn Leu Lys Ser Ser Ser Pro Thr Ile Arg Arg Thr Ala
        260                 265                 270

Ala Gly Ser Ala Val Ser Ile Cys Gln His Ser Arg Arg Thr Gln Tyr
    275                 280                 285

Phe Tyr Ser Trp Leu Leu Asn Val Leu Leu Gly Leu Leu Val Pro Val
    290                 295                 300

Glu Asp Glu His Ser Thr Leu Leu Ile Leu Gly Val Leu Leu Thr Leu
305                 310                 315                 320

Arg Tyr Leu Val Pro Leu Leu Gln Gln Gln Val Lys Asp Thr Ser Leu
                325                 330                 335

Lys Gly Ser Phe Gly Val Thr Arg Lys Glu Met Glu Val Ser Pro Ser
        340                 345                 350

Ala Glu Gln Leu Val Gln Val Tyr Glu Leu Thr Leu His His Thr Gln
        355                 360                 365

His Gln Asp His Asn Val Val Thr Gly Ala Leu Glu Leu Leu Gln Gln
    370                 375                 380

Leu Phe Arg Thr Pro Pro Pro Glu Leu Leu Gln Thr Leu Thr Ala Val
385                 390                 395                 400

Gly Gly Ile Gly Gln Leu Thr Ala Ala Lys Glu Glu Ser Gly Gly Arg
                405                 410                 415

Ser Arg Ser Gly Ser Ile Val Glu Leu Ile Ala Gly Gly Gly Ser Ser
            420                 425                 430

Cys Ser Pro Val Leu Ser Arg Lys Gln Lys Gly Lys Val Leu Leu Gly
        435                 440                 445

Glu Glu Glu Ala Leu Glu Asp Asp Ser Glu Ser Arg Ser Asp Val Ser
    450                 455                 460

Ser Ser Ala Leu Thr Ala Ser Val Lys Asp Glu Ile Ser Gly Glu Leu
465                 470                 475                 480

Ala Ala Ser Ser Gly Val Ser Thr Pro Gly Ser Ala Gly His Asp Ile
                485                 490                 495

Ile Thr Glu Gln Pro Arg Ser Gln His Thr Leu Gln Ala Asp Ser Val
            500                 505                 510

Asp Leu Ala Ser Cys Asp Leu Thr Ser Ser Ala Thr Asp Gly Asp Glu
        515                 520                 525

Glu Asp Ile Leu Ser His Ser Ser Ser Gln Val Ser Ala Val Pro Ser
    530                 535                 540

Asp Pro Ala Met Asp Leu Asn Asp Gly Thr Gln Ala Ser Ser Pro Ile
545                 550                 555                 560

Ser Asp Ser Ser Gln Thr Thr Thr Glu Gly Pro Asp Ser Ala Val Thr
                565                 570                 575

Pro Ser Asp Ser Ser Glu Ile Val Leu Asp Gly Thr Asp Asn Gln Tyr
            580                 585                 590

Leu Gly Leu Gln Ile Gly Gln Pro Gln Asp Glu Asp Glu Glu Ala Thr
        595                 600                 605

Gly Ile Leu Pro Asp Glu Ala Ser Glu Ala Phe Arg Asn Ser Ser Met
    610                 615                 620

Ala Leu Gln Gln Ala His Leu Leu Lys Asn Met Ser His Cys Arg Gln
625                 630                 635                 640

Pro Ser Asp Ser Ser Val Asp Lys Phe Val Leu Arg Asp Glu Ala Thr
                645                 650                 655

Glu Pro Gly Asp Gln Glu Asn Lys Pro Cys Arg Ile Lys Gly Asp Ile
            660                 665                 670

Gly Gln Ser Thr Asp Asp Asp Ser Ala Pro Leu Val His Cys Val Arg
        675                 680                 685

Leu Leu Ser Ala Ser Phe Leu Leu Thr Gly Gly Lys Asn Val Leu Val
    690                 695                 700

Pro Asp Arg Asp Val Arg Val Ser Val Lys Ala Leu Ala Leu Ser Cys
705                 710                 715                 720

Val Gly Ala Ala Val Ala Leu His Pro Glu Ser Phe Phe Ser Lys Leu
                725                 730                 735

Tyr Lys Val Pro Leu Asp Thr Thr Glu Tyr Pro Glu Glu Gln Tyr Val
            740                 745                 750

Ser Asp Ile Leu Asn Tyr Ile Asp His Gly Asp Pro Gln Val Arg Gly
        755                 760                 765

Ala Thr Ala Ile Leu Cys Gly Thr Leu Ile Cys Ser Ile Leu Ser Arg
    770                 775                 780

Ser Arg Phe His Val Gly Asp Trp Met Gly Thr Ile Arg Thr Leu Thr
785                 790                 795                 800

Gly Asn Thr Phe Ser Leu Ala Asp Cys Ile Pro Leu Leu Arg Lys Thr
                805                 810                 815

Leu Lys Asp Glu Ser Ser Val Thr Cys Lys Leu Ala Cys Thr Ala Val
            820                 825                 830

Arg Asn Cys Val Met Ser Leu Cys Ser Ser Ser Tyr Ser Glu Leu Gly
        835                 840                 845

Leu Gln Leu Ile Ile Asp Val Leu Thr Leu Arg Asn Ser Ser Tyr Trp

    850                 855                 860

Leu Val Arg Thr Glu Leu Leu Glu Thr Leu Ala Glu Ile Asp Phe Arg
865                 870                 875                 880

Leu Val Ser Phe Leu Glu Ala Lys Ala Glu Asn Leu His Arg Gly Ala
                885                 890                 895

His His Tyr Thr Gly Leu Leu Lys Leu Gln Glu Arg Val Leu Asn Asn
            900                 905                 910

Val Val Ile His Leu Leu Gly Asp Glu Asp Pro Arg Val Arg His Val
        915                 920                 925

Ala Ala Ala Ser Leu Ile Arg Leu Val Pro Lys Leu Phe Tyr Lys Cys
    930                 935                 940

Asp Gln Gly Gln Ala Asp Pro Val Val Ala Val Ala Arg Asp Gln Ser
945                 950                 955                 960

Ser Val Tyr Leu Lys Leu Leu Met His Glu Thr Gln Pro Pro Ser His
                965                 970                 975

Phe Ser Val Ser Thr Ile Thr Arg Ile Tyr Arg Gly Tyr Asn Leu Leu
            980                 985                 990

Pro Ser Ile Thr Asp Val Thr Met Glu Asn Asn Leu Ser Arg Val Ile
        995                 1000                1005

Ala Ala Val Ser His Glu Leu Ile Thr Ser Thr Thr Arg Ala Leu
    1010                1015                1020

Thr Phe Gly Cys Cys Glu Ala Leu Cys Leu Leu Ser Thr Ala Phe
    1025                1030                1035

Pro Val Cys Ile Trp Ser Leu Gly Trp His Cys Gly Val Pro Pro
    1040                1045                1050

Leu Ser Ala Ser Asp Glu Ser Arg Lys Ser Cys Thr Val Gly Met
    1055                1060                1065

Ala Thr Met Ile Leu Thr Leu Leu Ser Ser Ala Trp Phe Pro Leu
    1070                1075                1080

Asp Leu Ser Ala His Gln Asp Ala Leu Ile Leu Ala Gly Asn Leu
    1085                1090                1095

Leu Ala Ala Ser Ala Pro Lys Ser Leu Arg Ser Ser Trp Ala Ser
    1100                1105                1110

Glu Glu Glu Ala Asn Pro Ala Ala Thr Lys Gln Glu Glu Val Trp
    1115                1120                1125

Pro Ala Leu Gly Asp Arg Ala Leu Val Pro Met Val Glu Gln Leu
    1130                1135                1140

Phe Ser His Leu Leu Lys Val Ile Asn Ile Cys Ala His Val Leu
    1145                1150                1155

Asp Asp Val Ala Pro Gly Pro Ala Ile Lys Ala Ala Leu Pro Ser
    1160                1165                1170

Leu Thr Asn Pro Pro Ser Leu Ser Pro Ile Arg Arg Lys Gly Lys
    1175                1180                1185

Glu Lys Glu Pro Gly Glu Gln Ala Ser Val Pro Leu Ser Pro Lys
    1190                1195                1200

Lys Gly Ser Glu Ala Ser Ala Ala Ser Arg Gln Ser Asp Thr Ser
    1205                1210                1215

Gly Pro Val Thr Thr Ser Lys Ser Ser Ser Leu Gly Ser Phe Tyr
    1220                1225                1230

His Leu Pro Ser Tyr Leu Lys Leu His Asp Val Leu Lys Ala Thr
    1235                1240                1245

His Ala Asn Tyr Lys Val Thr Leu Asp Leu Gln Asn Ser Thr Glu
    1250                1255                1260

Lys Phe Gly Gly Phe Leu Arg Ser Ala Leu Asp Val Leu Ser Gln
    1265                1270                1275

Ile Leu Glu Leu Ala Thr Leu Gln Asp Ile Gly Lys Cys Val Glu
    1280                1285                1290

Glu Ile Leu Gly Tyr Leu Lys Ser Cys Phe Ser Arg Glu Pro Met
    1295                1300                1305

Met Ala Thr Val Cys Val Gln Gln Leu Leu Lys Thr Leu Phe Gly
    1310                1315                1320

Thr Asn Leu Ala Ser Gln Phe Asp Gly Leu Ser Ser Asn Pro Ser
    1325                1330                1335

Lys Ser Gln Gly Arg Ala Gln Arg Leu Gly Ser Ser Ser Val Arg
    1340                1345                1350

Pro Gly Leu Tyr His Tyr Cys Phe Met Ala Pro Tyr Thr His Phe
    1355                1360                1365

Thr Gln Ala Leu Ala Asp Ala Ser Leu Arg Asn Met Val Gln Ala
    1370                1375                1380

Glu Gln Glu Asn Asp Thr Ser Gly Trp Phe Asp Val Leu Gln Lys
    1385                1390                1395

Val Ser Thr Gln Leu Lys Thr Asn Leu Thr Ser Val Thr Lys Asn
    1400                1405                1410

Arg Ala Asp Lys Asn Ala Ile His Asn His Ile Arg Leu Phe Glu
    1415                1420                1425

Pro Leu Val Ile Lys Ala Leu Lys Gln Tyr Thr Thr Thr Thr Cys
    1430                1435                1440

Val Gln Leu Gln Lys Gln Val Leu Asp Leu Leu Ala Gln Leu Val
    1445                1450                1455

Gln Leu Arg Val Asn Tyr Cys Leu Leu Asp Ser Asp Gln Val Phe
    1460                1465                1470

Ile Gly Phe Val Leu Lys Gln Phe Glu Tyr Ile Glu Val Gly Gln
    1475                1480                1485

Phe Arg Glu Ser Glu Ala Ile Ile Pro Asn Ile Phe Phe Phe Leu
    1490                1495                1500

Val Leu Leu Ser Tyr Glu Arg Tyr His Ser Lys Gln Ile Ile Gly
    1505                1510                1515

Ile Pro Lys Ile Ile Gln Leu Cys Asp Gly Ile Met Ala Ser Gly
    1520                1525                1530

Arg Lys Ala Val Thr His Ala Ile Pro Ala Leu Gln Pro Ile Val
    1535                1540                1545

His Asp Leu Phe Val Leu Arg Gly Thr Asn Lys Ala Asp Ala Gly
    1550                1555                1560

Lys Glu Leu Glu Thr Gln Lys Glu Val Val Val Ser Met Leu Leu
    1565                1570                1575

Arg Leu Ile Gln Tyr His Gln Val Leu Glu Met Phe Ile Leu Val
    1580                1585                1590

Leu Gln Gln Cys His Lys Glu Asn Glu Asp Lys Trp Lys Arg Leu
    1595                1600                1605

Ser Arg Gln Ile Ala Asp Ile Ile Leu Pro Met Leu Ala Lys Gln
    1610                1615                1620

Gln Met His Ile Asp Ser His Glu Ala Leu Gly Val Leu Asn Thr
    1625                1630                1635

Leu Phe Glu Ile Leu Ala Pro Ser Ser Leu Arg Pro Val Asp Met
    1640                1645                1650

Leu Leu Arg Ser Met Phe Val Thr Pro Asn Thr Met Ala Ser Val
    1655                1660                1665

Ser Thr Val Gln Leu Trp Ile Ser Gly Ile Leu Ala Ile Leu Arg
    1670                1675                1680

Val Leu Ile Ser Gln Ser Thr Glu Asp Ile Val Leu Ser Arg Ile
    1685                1690                1695

Gln Glu Leu Ser Phe Ser Pro Tyr Leu Ile Ser Cys Thr Val Ile
    1700                1705                1710

Asn Arg Leu Arg Asp Gly Asp Ser Thr Ser Thr Leu Glu Glu His
    1715                1720                1725

Ser Glu Gly Lys Gln Ile Lys Asn Leu Pro Glu Glu Thr Phe Ser
    1730                1735                1740

Arg Phe Leu Leu Gln Leu Val Gly Ile Leu Leu Glu Asp Ile Val
    1745                1750                1755

Thr Lys Gln Leu Lys Val Glu Met Ser Glu Gln Gln His Thr Phe
    1760                1765                1770

Tyr Cys Gln Glu Leu Gly Thr Leu Leu Met Cys Leu Ile His Ile
    1775                1780                1785

Phe Lys Ser Gly Met Phe Arg Arg Ile Thr Ala Ala Ala Thr Arg
    1790                1795                1800

Leu Phe Arg Ser Asp Gly Cys Gly Gly Ser Phe Tyr Thr Leu Asp

1805                1810                1815

Ser Leu Asn Leu Arg Ala Arg Ser Met Ile Thr Thr His Pro Ala
    1820                1825                1830

Leu Val Leu Leu Trp Cys Gln Ile Leu Leu Leu Val Asn His Thr
    1835                1840                1845

Asp Tyr Arg Trp Trp Ala Glu Val Gln Gln Thr Pro Lys Arg His
    1850                1855                1860

Ser Leu Ser Ser Thr Lys Leu Leu Ser Pro Gln Met Ser Gly Glu
    1865                1870                1875

Glu Glu Asp Ser Asp Leu Ala Ala Lys Leu Gly Met Cys Asn Arg
    1880                1885                1890

Glu Ile Val Arg Arg Gly Ala Leu Ile Leu Phe Cys Asp Tyr Val
    1895                1900                1905

Cys Gln Asn Leu His Asp Ser Glu His Leu Thr Trp Leu Ile Val
    1910                1915                1920

Asn His Ile Gln Asp Leu Ile Ser Leu Ser His Glu Pro Pro Val
    1925                1930                1935

Gln Asp Phe Ile Ser Ala Val His Arg Asn Ser Ala Ala Ser Gly
    1940                1945                1950

Leu Phe Ile Gln Ala Ile Gln Ser Arg Cys Glu Asn Leu Ser Thr
    1955                1960                1965

Pro Thr Met Leu Lys Lys Thr Leu Gln Cys Leu Glu Gly Ile His
    1970                1975                1980

Leu Ser Gln Ser Gly Ala Val Leu Thr Leu Tyr Val Asp Arg Leu
    1985                1990                1995

Leu Cys Thr Pro Phe Arg Val Leu Ala Arg Met Val Asp Ile Leu
    2000                2005                2010

Ala Cys Arg Arg Val Glu Met Leu Leu Ala Ala Asn Leu Gln Ser
    2015                2020                2025

Ser Met Ala Gln Leu Pro Met Glu Glu Leu Asn Arg Ile Gln Glu
    2030                2035                2040

Tyr Leu Gln Ser Ser Gly Leu Ala Gln Arg His Gln Arg Leu Tyr
    2045                2050                2055

Ser Leu Leu Asp Arg Phe Arg Leu Ser Thr Met Gln Asp Ser Leu
    2060                2065                2070

Ser Pro Ser Pro Pro Val Ser Ser His Pro Leu Asp Gly Asp Gly
    2075                2080                2085

His Val Ser Leu Glu Thr Val Ser Pro Asp Lys Asp Trp Tyr Val
    2090                2095                2100

His Leu Val Lys Ser Gln Cys Trp Thr Arg Ser Asp Ser Ala Leu
    2105                2110                2115

Leu Glu Gly Ala Glu Leu Val Asn Arg Ile Pro Ala Glu Asp Met
    2120                2125                2130

Asn Ala Phe Met Met Asn Ser Glu Phe Asn Leu Ser Leu Leu Ala
    2135                2140                2145

Pro Cys Leu Ser Leu Gly Met Ser Glu Ile Ser Gly Gly Gln Lys
    2150                2155                2160

Ser Ala Leu Phe Glu Ala Ala Arg Glu Val Thr Leu Ala Arg Val
    2165                2170                2175

Ser Gly Thr Val Gln Gln Leu Pro Ala Val His His Val Phe Gln
    2180                2185                2190

Pro Glu Leu Pro Ala Glu Pro Ala Ala Tyr Trp Ser Lys Leu Asn
    2195                2200                2205

Asp Leu Phe Gly Asp Ala Ala Leu Tyr Gln Ser Leu Pro Thr Leu
    2210                2215                2220

Ala Arg Ala Leu Ala Gln Tyr Leu Val Val Val Ser Lys Leu Pro
    2225                2230                2235

Ser His Leu His Leu Pro Pro Glu Lys Glu Lys Asp Ile Val Lys
    2240                2245                2250

Phe Val Val Ala Thr Leu Glu Ala Leu Ser Trp His Leu Ile His
    2255                2260                2265

Glu Gln Ile Pro Leu Ser Leu Asp Leu Gln Ala Gly Leu Asp Cys
    2270                2275                2280

Cys Cys Leu Ala Leu Gln Leu Pro Gly Leu Trp Ser Val Val Ser
    2285                2290                2295

Ser Thr Glu Phe Val Thr His Ala Cys Ser Leu Ile Tyr Cys Val
    2300                2305                2310

His Phe Ile Leu Glu Ala Val Ala Val Gln Pro Gly Glu Gln Leu
    2315                2320                2325

Leu Ser Pro Glu Arg Arg Thr Asn Thr Pro Lys Ala Ile Ser Glu
    2330                2335                2340

Glu Glu Glu Glu Val Asp Pro Asn Thr Gln Asn Pro Lys Tyr Ile
    2345                2350                2355

Thr Ala Ala Cys Glu Met Val Ala Glu Met Val Glu Ser Leu Gln
    2360                2365                2370

Ser Val Leu Ala Leu Gly His Lys Arg Asn Ser Gly Val Pro Ala
    2375                2380                2385

Phe Leu Thr Pro Leu Leu Arg Asn Ile Ile Ile Ser Leu Ala Arg
    2390                2395                2400

Leu Pro Leu Val Asn Ser Tyr Thr Arg Val Pro Pro Leu Val Trp
    2405                2410                2415

Lys Leu Gly Trp Ser Pro Lys Pro Gly Gly Asp Phe Gly Thr Ala
    2420                2425                2430

Phe Pro Glu Ile Pro Val Glu Phe Leu Gln Glu Lys Glu Val Phe
    2435                2440                2445

Lys Glu Phe Ile Tyr Arg Ile Asn Thr Leu Gly Trp Thr Ser Arg
    2450                2455                2460

Thr Gln Phe Glu Glu Thr Trp Ala Thr Leu Leu Gly Val Leu Val
    2465                2470                2475

Thr Gln Pro Leu Val Met Glu Gln Glu Glu Ser Pro Pro Glu Glu
    2480                2485                2490

Asp Thr Glu Arg Thr Gln Ile Asn Val Leu Ala Val Gln Ala Ile
    2495                2500                2505

Thr Ser Leu Val Leu Ser Ala Met Thr Val Pro Val Ala Gly Asn
    2510                2515                2520

Pro Ala Val Ser Cys Leu Glu Gln Gln Pro Arg Asn Lys Pro Leu
    2525                2530                2535

Lys Ala Leu Asp Thr Arg Phe Gly Arg Lys Leu Ser Ile Ile Arg
    2540                2545                2550

Gly Ile Val Glu Gln Glu Ile Gln Ala Met Val Ser Lys Arg Glu
    2555                2560                2565

Asn Ile Ala Thr His His Leu Tyr Gln Ala Trp Asp Pro Val Pro
    2570                2575                2580

Ser Leu Ser Pro Ala Thr Thr Gly Ala Leu Ile Ser His Glu Lys
    2585                2590                2595

Leu Leu Leu Gln Ile Asn Pro Glu Arg Glu Leu Gly Ser Met Ser
    2600                2605                2610

Tyr Lys Leu Gly Gln Val Ser Ile His Ser Val Trp Leu Gly Asn
    2615                2620                2625

Ser Ile Thr Pro Leu Arg Glu Glu Glu Trp Asp Glu Glu Glu Glu
    2630                2635                2640

Glu Glu Ala Asp Ala Pro Ala Pro Ser Ser Pro Pro Thr Ser Pro
    2645                2650                2655

Val Asn Ser Arg Lys His Arg Ala Gly Val Asp Ile His Ser Cys
    2660                2665                2670

Ser Gln Phe Leu Leu Glu Leu Tyr Ser Arg Trp Ile Leu Pro Ser
    2675                2680                2685

Ser Ser Ala Arg Arg Thr Pro Ala Ile Leu Ile Ser Glu Val Val
    2690                2695                2700

Arg Ser Leu Leu Val Val Ser Asp Leu Phe Thr Glu Arg Asn Gln
    2705                2710                2715

Phe Glu Leu Met Tyr Val Thr Leu Thr Glu Leu Arg Arg Val His
    2720                2725                2730

Pro Ser Glu Asp Glu Ile Leu Ala Gln Tyr Leu Val Pro Ala Thr
    2735                2740                2745

Cys Lys Ala Ala Ala Val Leu Gly Met Asp Lys Ala Val Ala Glu

2750                2755                2760

Pro Val Ser Arg Leu Leu Glu Ser Thr Leu Arg Ser Ser His Leu
    2765                2770                2775

Pro Ser Arg Val Gly Ala Leu His Gly Val Leu Tyr Val Leu Glu
    2780                2785                2790

Cys Asp Leu Leu Asp Asp Thr Ala Lys Gln Leu Ile Pro Val Ile
    2795                2800                2805

Ser Asp Tyr Leu Leu Ser Asn Leu Lys Gly Ile Ala His Cys Val
    2810                2815                2820

Asn Ile His Ser Gln Gln His Val Leu Val Met Cys Ala Thr Ala
    2825                2830                2835

Phe Tyr Leu Ile Glu Asn Tyr Pro Leu Asp Val Gly Pro Glu Phe
    2840                2845                2850

Ser Ala Ser Ile Ile Gln Met Cys Gly Val Met Leu Ser Gly Ser
    2855                2860                2865

Glu Glu Ser Thr Pro Ser Ile Ile Tyr His Cys Ala Leu Arg Gly
    2870                2875                2880

Leu Glu Arg Leu Leu Leu Ser Glu Gln Leu Ser Arg Leu Asp Ala
    2885                2890                2895

Glu Ser Leu Val Lys Leu Ser Val Asp Arg Val Asn Val His Ser
    2900                2905                2910

Pro His Arg Ala Met Ala Ala Leu Gly Leu Met Leu Thr Cys Met
    2915                2920                2925

Tyr Thr Gly Lys Glu Lys Val Ser Pro Gly Arg Thr Ser Asp Pro
    2930                2935                2940

Asn Pro Ala Ala Pro Asp Ser Glu Ser Val Ile Val Ala Met Glu
    2945                2950                2955

Arg Val Ser Val Leu Phe Asp Arg Ile Arg Lys Gly Phe Pro Cys
    2960                2965                2970

Glu Ala Arg Val Val Ala Arg Ile Leu Pro Gln Phe Leu Asp Asp
    2975                2980                2985

Phe Phe Pro Pro Gln Asp Ile Met Asn Lys Val Ile Gly Glu Phe
    2990                2995                3000

Leu Ser Asn Gln Gln Pro Tyr Pro Gln Phe Met Ala Thr Val Val
    3005                3010                3015

Tyr Lys Val Phe Gln Thr Leu His Ser Thr Gly Gln Ser Ser Met
    3020                3025                3030

Val Arg Asp Trp Val Met Leu Ser Leu Ser Asn Phe Thr Gln Arg
    3035                3040                3045

Ala Pro Val Ala Met Ala Thr Trp Ser Leu Ser Cys Phe Phe Val
    3050                3055                3060

Ser Ala Ser Thr Ser Pro Trp Val Ala Ala Ile Leu Pro His Val
    3065                3070                3075

Ile Ser Arg Met Gly Lys Leu Glu Gln Val Asp Val Asn Leu Phe
    3080                3085                3090

Cys Leu Val Ala Thr Asp Phe Tyr Arg His Gln Ile Glu Glu Glu
    3095                3100                3105

Leu Asp Arg Arg Ala Phe Gln Ser Val Leu Glu Val Val Ala Ala
    3110                3115                3120

Pro Gly Ser Pro Tyr His Arg Leu Leu Thr Cys Leu Arg Asn Val
    3125                3130                3135

His Lys Val Thr Thr Cys
    3140

<210>  5
<211>  91
<212>  PRT
<213>  Homo sapiens

<220>
<223>  HTTexon1-Q25

<400>  5

Met Ala Thr Leu Glu Lys Leu Met Lys Ala Phe Glu Ser Leu Lys Ser
1               5                   10                  15

Phe Gln Gln Gln Gln Gln Gln Gln Gln Gln Gln Gln Gln Gln Gln Gln
            20                  25                  30

Gln Gln Gln Gln Gln Gln Gln Gln Gln Gln Pro Pro Pro Pro Pro Pro

35                  40                  45

Pro Pro Pro Pro Pro Gln Leu Pro Gln Pro Pro Pro Gln Ala Gln Pro
    50                  55                  60

Leu Leu Pro Gln Pro Gln Pro Pro Pro Pro Pro Pro Pro Pro Pro Pro
65                  70                  75                  80

Gly Pro Ala Val Ala Glu Glu Pro Leu His Arg
                85                  90

<210>  6
<211>  261
<212>  PRT
<213>  Homo sapiens

<220>
<223>  Q25-GFP

<400>  6

Met Gln Gln Gln Gln Gln Gln Gln Gln Gln Gln Gln Gln Gln Gln Gln
1               5                   10                  15

Gln Gln Gln Gln Gln Gln Gln Gln Gln Gln Met Ser Lys Gly Glu Glu
            20                  25                  30

Leu Phe Thr Gly Val Val Pro Ile Leu Val Glu Leu Asp Gly Asp Val
        35                  40                  45

Asn Gly His Lys Phe Ser Val Arg Gly Glu Gly Glu Gly Asp Ala Thr
    50                  55                  60

Asn Gly Lys Leu Thr Leu Lys Phe Ile Cys Thr Thr Gly Lys Leu Pro
65                  70                  75                  80

Val Pro Trp Pro Thr Leu Val Thr Thr Leu Thr Tyr Gly Val Gln Cys
                85                  90                  95

Phe Ser Arg Tyr Pro Asp His Met Lys Arg His Asp Phe Phe Lys Ser
            100                 105                 110

Ala Met Pro Glu Gly Tyr Val Gln Glu Arg Thr Ile Ser Phe Lys Asp
        115                 120                 125

Asp Gly Thr Tyr Lys Thr Arg Ala Glu Val Lys Phe Glu Gly Asp Thr
    130                 135                 140

Leu Val Asn Arg Ile Glu Leu Lys Gly Ile Asp Phe Lys Glu Asp Gly
145                 150                 155                 160

Asn Ile Leu Gly His Lys Leu Glu Tyr Asn Phe Asn Ser His Asn Val
                165                 170                 175

Tyr Ile Thr Ala Asp Lys Gln Lys Asn Gly Ile Lys Ala Asn Phe Lys
            180                 185                 190

Ile Arg His Asn Val Glu Asp Gly Ser Val Gln Leu Ala Asp His Tyr
        195                 200                 205

Gln Gln Asn Thr Pro Ile Gly Asp Gly Pro Val Leu Leu Pro Asp Asn
    210                 215                 220

His Tyr Leu Ser Thr Gln Ser Val Leu Ser Lys Asp Pro Asn Glu Lys
225                 230                 235                 240

Arg Asp His Met Val Leu Leu Glu Phe Val Thr Ala Ala Gly Ile Thr
                245                 250                 255

His Gly Gly Ser Gly
            260

<210>  7
<211>  365
<212>  PRT
<213>  Homo sapiens

<220>
<223>  ATXN3-Q27

<400>  7

Met Glu Ser Ile Phe His Glu Lys Gln Glu Gly Ser Leu Cys Ala Gln
1               5                   10                  15

His Cys Leu Asn Asn Leu Leu Gln Gly Glu Tyr Phe Ser Pro Val Glu
            20                  25                  30

Leu Ser Ser Ile Ala His Gln Leu Asp Glu Glu Glu Arg Met Arg Met
        35                  40                  45

Ala Glu Gly Gly Val Thr Ser Glu Asp Tyr Arg Thr Phe Leu Gln Gln
    50                  55                  60

Pro Ser Gly Asn Met Asp Asp Ser Gly Phe Phe Ser Ile Gln Val Ile
65                  70                  75                  80

Ser Asn Ala Leu Lys Val Trp Gly Leu Glu Leu Ile Leu Phe Asn Ser
                85                  90                  95

Pro Glu Tyr Gln Arg Leu Arg Ile Asp Pro Ile Asn Glu Arg Ser Phe
        100                 105                 110

Ile Cys Asn Tyr Lys Glu His Trp Phe Thr Val Arg Lys Leu Gly Lys
    115                 120                 125

Gln Trp Phe Asn Leu Asn Ser Leu Leu Thr Gly Pro Glu Leu Ile Ser
    130                 135                 140

Asp Thr Tyr Leu Ala Leu Phe Leu Ala Gln Leu Gln Gln Glu Gly Tyr
145                 150                 155                 160

Ser Ile Phe Val Val Lys Gly Asp Leu Pro Asp Cys Glu Ala Asp Gln
                165                 170                 175

Leu Leu Gln Met Ile Arg Val Gln Gln Met His Arg Pro Lys Leu Ile
        180                 185                 190

Gly Glu Glu Leu Ala Gln Leu Lys Glu Gln Arg Val His Lys Thr Asp
    195                 200                 205

Leu Glu Arg Met Leu Glu Ala Asn Asp Gly Ser Gly Met Leu Asp Glu
    210                 215                 220

Asp Glu Glu Asp Leu Gln Arg Ala Leu Ala Leu Ser Arg Gln Glu Ile
225                 230                 235                 240

Asp Met Glu Asp Glu Glu Ala Asp Leu Arg Arg Ala Ile Gln Leu Ser
                245                 250                 255

Met Gln Gly Ser Ser Arg Asn Ile Ser Gln Asp Met Thr Gln Thr Ser
        260                 265                 270

Gly Thr Asn Leu Thr Ser Glu Glu Leu Arg Lys Arg Arg Glu Ala Tyr
    275                 280                 285

Phe Glu Lys Gln Gln Gln Lys Gln Gln Gln Gln Gln Gln Gln Gln Gln
    290                 295                 300

Gln Gln Gln Gln Gln Gln Gln Gln Gln Gln Gln Gln Gln Gln Gln Gln
305                 310                 315                 320

Gln Gln Arg Asp Leu Ser Gly Gln Ser Ser His Pro Cys Glu Arg Pro
                325                 330                 335

Ala Thr Ser Ser Gly Ala Leu Gly Ser Asp Leu Gly Lys Ala Cys Ser
        340                 345                 350

Pro Phe Ile Met Phe Ala Thr Phe Thr Leu Tyr Leu Thr
        355                 360                 365

<210>  8
<211>  125
<212>  PRT
<213>  Homo sapiens

<220>
<223>  LC3B

<400>  8

Met Pro Ser Glu Lys Thr Phe Lys Gln Arg Arg Thr Phe Glu Gln Arg
1               5                   10                  15

Val Glu Asp Val Arg Leu Ile Arg Glu Gln His Pro Thr Lys Ile Pro
            20                  25                  30

Val Ile Ile Glu Arg Tyr Lys Gly Glu Lys Gln Leu Pro Val Leu Asp
        35                  40                  45

Lys Thr Lys Phe Leu Val Pro Asp His Val Asn Met Ser Glu Leu Ile
    50                  55                  60

Lys Ile Ile Arg Arg Arg Leu Gln Leu Asn Ala Asn Gln Ala Phe Phe
65                  70                  75                  80

Leu Leu Val Asn Gly His Ser Met Val Ser Val Ser Thr Pro Ile Ser
                85                  90                  95

Glu Val Tyr Glu Ser Glu Lys Asp Glu Asp Gly Phe Leu Tyr Met Val
            100                 105                 110

Tyr Ala Ser Gln Glu Thr Phe Gly Met Lys Leu Ser Val
        115                 120                 125